# EUROPEAN PATENT SPECIFICATION

(11) **EP 2 283 141 B1**
(45) Date of publication and mention of the grant of the patent: **23.04.2014**
(21) Application number: 09759429.5
(22) Date of filing: 04.06.2009
(51) Int. Cl.: C12P 7/16

(54) **A method for producing butanol using extraction and gas stripping**
Verfahren zur Herstellung von Butanol durch Extraktion und Gas-Strippen
Procédé de production de butanol à l'aide d'une extraction et d'une stripage au gaz

(30) Priority: 04.06.2008 US 58567 P
(43) Date of publication of application: 16.02.2011
(60) Divisional application: 13168584.4
(73) Proprietor: Butamax (TM) Advanced Biofuels LLC, Wilmington, DE 19880 (US)
(72) Inventor: GRADY, Michael, Charles, Oaklyn New Jersey 08107 (US); JAHIC, Mehmedalija, Wilmington Delaware 19810 (US); PATNAIK, Ranjan, Newark Delaware 19702 (US)
(74) Representative: Owen, Deborah Jane
(86) International application number: PCT/US2009/046278
(87) International publication number: WO 2009/149270

(56) References cited:
- WO-A-2008/143704
- US-A1- 2007 092 957
- EVANS ET AL: "Enhancement of butanol formation by Clostridium acetobutylicum in the presence of decanol-oleyl alcohol mixed extractants" APPLIED AND ENVIRONMENTAL MICROBIOLOGY, vol. 54, 1988, pages 1662-1667, XP008113796 cited in the application
- DAVISON ET AL: "Continuous direct solvent extraction of butanol in a fermenting fluidized-bed bioreactor with immobilized Clostridium acetobutylicum" APPLIED BIOCHEMISTRY AND BIOTECHNOLOGY, vol. 39/40, 1993, pages 415-426, XP002569128
- EZEJI ET AL: "Bioproduction of butanol from biomass: from genes to bioreactors" CURRENT OPINION IN BIOTECHNOLOGY, vol. 18, 2007, pages 220-227, XP022110184
- OFFEMAN ET AL: "Extraction of ethanol with higher alcohol solvents and their toxicity to yeast" SEPARATION AND PURIFICATION TECHNOLOGY, vol. 63, October 2008 (2008-10), XP025474769
- EGAN ET AL: "Solvent extraction and recovery of ethanol from aqueous solutions", INDUSTRIAL AND ENGINEERING CHEMISTRY RESEARCH, vol. 27, 1988, pages 1330-1332, XP007920417,

### SEQUENCE LISTING

<110> E.I. du Pont de Nemours and company
<120> A Method for Producing Butanol using Two-Phase Extractive Fermentation
<130> CL4197
<160> 86
<170> PatentIn version 3.4
<210> 1
   <211> 1680
   <212> DNA
   <213> Klebsiella pneumoniae
<400> 1
   atggacaaac agtatccggt acgccagtgg gcgcacggcg ccgatctcgt cgtcagtcag     60
   ctggaagctc agggagtacg ccaggtgttc ggcatccccg gcgccaaaat cgacaaggtc    120
   tttgattcac tgctggattc ctccattcgc attattccgg tacgccacga agccaacgcc    180
   gcatttatgg ccgccgccgt cggacgcatt accggcaaag cgggcgtggc gctggtcacc    240
   tccggtccgg gctgttccaa cctgatcacc ggcatggcca ccgcgaacag cgaaggcgac    300
   ccggtggtgg ccctgggcgg cgcggtaaaa cgcgccgata aagcgaagca ggtccaccag    360
   agtatggata cggtggcgat gttcagcccg gtcaccaaat acgccatcga ggtgacggcg    420
   ccggatgcgc tggcggaagt ggtctccaac gccttccgcg ccgccgagca gggccggccg    480
   ggcagcgcgt tcgttagcct gccgcaggat gtggtcgatg gcccggtcag cggcaaagtg    540
   ctgccggcca gcggggcccc gcagatgggc gccgcgccgg atgatgccat cgaccaggtg    600
   gcgaagctta tcgcccaggc gaagaacccg atcttcctgc tcggcctgat ggccagccag    660
   ccggaaaaca gcaaggcgct gcgccgtttg ctggagacca gccatattcc agtcaccagc    720
   acctatcagg ccgccggagc ggtgaatcag gataacttct ctcgcttcgc cggccgggtt    780
   gggctgttta acaaccaggc cggggaccgt ctgctgcagc tcgccgacct ggtgatctgc    840
   atcggctaca gcccggtgga atacgaaccg gcgatgtgga acagcggcaa cgcgacgctg    900
   gtgcacatcg acgtgctgcc cgcctatgaa gagcgcaact acaccccgga tgtcgagctg    960
   gtgggcgata tcgccggcac tctcaacaag ctggcgcaaa atatcgatca tcggctggtg   1020
   ctctccccgc aggcggcgga gatcctccgc gaccgccagc accagcgcga gctgctggac   1080
   cgccgcggcg cgcagctcaa ccagtttgcc ctgcatcccc tgcgcatcgt tcgcgccatg   1140
   caggatatcg tcaacagcga cgtcacgttg accgtggaca tgggcagctt ccatatctgg   1200
   attgcccgct acctgtacac gttccgcgcc cgtcaggtga tgatctccaa cggccagcag   1260
   accatgggcg tcgccctgcc ctgggctatc ggcgcctggc tggtcaatcc tgagcgcaaa   1320
   gtggtctccg tctccggcga cggcggcttc ctgcagtcga gcatggagct ggagaccgcc   1380
   gtccgcctga aagccaacgt gctgcatctt atctgggtcg ataacggcta caacatggtc   1440
   gctatccagg aagagaaaaa atatcagcgc ctgtccggcg tcgagtttgg gccgatggat 1500
   tttaaagcct atgccgaatc cttcggcgcg aaagggtttg ccgtggaaag cgccgaggcg 1560
   ctggagccga ccctgcgcgc ggcgatggac gtcgacggcc cggcggtagt ggccatcccg 1620
   gtggattatc gcgataaccc gctgctgatg ggccagctgc atctgagtca gattctgtaa 1680
<210> 2
   <211> 559
   <212> PRT
   <213> Klebsiella pneumoniae
<400> 2
   Met Asp Lys Gln Tyr Pro Val Arg Gln Trp Ala His Gly Ala Asp Leu
   1 5 10 15
   Val Val Ser Gln Leu Glu Ala Gln Gly Val Arg Gln Val Phe Gly Ile
   20 25 30
   Pro Gly Ala Lys Ile Asp Lys Val Phe Asp Ser Leu Leu Asp Ser Ser
   35 40 45
   Ile Arg Ile Ile Pro Val Arg His Glu Ala Asn Ala Ala Phe Met Ala
   50 55 60
   Ala Ala Val Gly Arg Ile Thr Gly Lys Ala Gly Val Ala Leu Val Thr
   65 70 75 80
   Ser Gly Pro Gly Cys Ser Asn Leu Ile Thr Gly Met Ala Thr Ala Asn
   85 90 95
   Ser Glu Gly Asp Pro Val Val Ala Leu Gly Gly Ala Val Lys Arg Ala
   100 105 110
   Asp Lys Ala Lys Gln Val His Gln Ser Met Asp Thr Val Ala Met Phe
   115 120 125
   Ser Pro Val Thr Lys Tyr Ala Ile Glu Val Thr Ala Pro Asp Ala Leu
   130 135 140
   Ala Glu Val Val Ser Asn Ala Phe Arg Ala Ala Glu Gln Gly Arg Pro
   145 150 155 160
   Gly Ser Ala Phe Val Ser Leu Pro Gln Asp Val Val Asp Gly Pro Val
   165 170 175
   Ser Gly Lys Val Leu Pro Ala Ser Gly Ala Pro Gln Met Gly Ala Ala
   180 185 190
   Pro Asp Asp Ala Ile Asp Gln Val Ala Lys Leu Ile Ala Gln Ala Lys
   195 200 205
   Asn Pro Ile Phe Leu Leu Gly Leu Met Ala Ser Gln Pro Glu Asn Ser
       210                 215                 220
   Lys Ala Leu Arg Arg Leu Leu Glu Thr Ser His Ile Pro Val Thr Ser
   225                 230                 235                 240
   Thr Tyr Gln Ala Ala Gly Ala Val Asn Gln Asp Asn Phe Ser Arg Phe
                   245                 250                 255
   Ala Gly Arg Val Gly Leu Phe Asn Asn Gln Ala Gly Asp Arg Leu Leu
               260                 265                 270
   Gln Leu Ala Asp Leu Val Ile Cys Ile Gly Tyr Ser Pro Val Glu Tyr
           275                 280                 285
   Glu Pro Ala Met Trp Asn Ser Gly Asn Ala Thr Leu Val His Ile Asp
       290                 295                 300
   Val Leu Pro Ala Tyr Glu Glu Arg Asn Tyr Thr Pro Asp Val Glu Leu
   305                 310                 315                 320
   Val Gly Asp Ile Ala Gly Thr Leu Asn Lys Leu Ala Gln Asn Ile Asp
                   325                 330                 335
   His Arg Leu Val Leu Ser Pro Gln Ala Ala Glu Ile Leu Arg Asp Arg
               340                 345                 350
   Gln His Gln Arg Glu Leu Leu Asp Arg Arg Gly Ala Gln Leu Asn Gln
           355                 360                 365
   Phe Ala Leu His Pro Leu Arg Ile Val Arg Ala Met Gln Asp Ile Val
       370                 375                 380
   Asn Ser Asp Val Thr Leu Thr Val Asp Met Gly Ser Phe His Ile Trp
   385                 390                 395                 400
   Ile Ala Arg Tyr Leu Tyr Thr Phe Arg Ala Arg Gln Val Met Ile Ser
                   405                 410                 415
   Asn Gly Gln Gln Thr Met Gly Val Ala Leu Pro Trp Ala Ile Gly Ala
               420                 425                 430
   Trp Leu Val Asn Pro Glu Arg Lys Val Val Ser Val Ser Gly Asp Gly
           435                 440                 445
   Gly Phe Leu Gln Ser Ser Met Glu Leu Glu Thr Ala Val Arg Leu Lys
       450                 455                 460
   Ala Asn Val Leu His Leu Ile Trp Val Asp Asn Gly Tyr Asn Met Val
   465                 470                 475                 480
   Ala Ile Gln Glu Glu Lys Lys Tyr Gln Arg Leu Ser Gly Val Glu Phe
                   485                 490                 495
   Gly Pro Met Asp Phe Lys Ala Tyr Ala Glu Ser Phe Gly Ala Lys Gly
               500                 505                 510
   Phe Ala Val Glu Ser Ala Glu Ala Leu Glu Pro Thr Leu Arg Ala Ala
           515                 520                 525
   Met Asp Val Asp Gly Pro Ala Val Val Ala Ile Pro Val Asp Tyr Arg
       530                 535                 540
   Asp Asn Pro Leu Leu Met Gly Gln Leu His Leu Ser Gln Ile Leu
   545                 550                 555
<210> 3
   <211> 1476
   <212> DNA
   <213> Escherichia coli
<400> 3
   atggctaact acttcaatac actgaatctg cgccagcagc tggcacagct gggcaaatgt     60
   cgctttatgg gccgcgatga attcgccgat ggcgcgagct accttcaggg taaaaaagta    120
   gtcatcgtcg gctgtggcgc acagggtctg aaccagggcc tgaacatgcg tgattctggt    180
   ctcgatatct cctacgctct gcgtaaagaa gcgattgccg agaagcgcgc gtcctggcgt    240
   aaagcgaccg aaaatggttt taaagtgggt acttacgaag aactgatccc acaggcggat    300
   ctggtgatta acctgacgcc ggacaagcag cactctgatg tagtgcgcac cgtacagcca    360
   ctgatgaaag acggcgcggc gctgggctac tcgcacggtt tcaacatcgt cgaagtgggc    420
   gagcagatcc gtaaagatat caccgtagtg atggttgcgc cgaaatgccc aggcaccgaa    480
   gtgcgtgaag agtacaaacg tgggttcggc gtaccgacgc tgattgccgt tcacccggaa    540
   aacgatccga aaggcgaagg catggcgatt gccaaagcct gggcggctgc aaccggtggt    600
   caccgtgcgg gtgtgctgga atcgtccttc gttgcggaag tgaaatctga cctgatgggc    660
   gagcaaacca tcctgtgcgg tatgttgcag gctggctctc tgctgtgctt cgacaagctg    720
   gtggaagaag gtaccgatcc agcatacgca gaaaaactga ttcagttcgg ttgggaaacc    780
   atcaccgaag cactgaaaca gggcggcatc accctgatga tggaccgtct ctctaacccg    840
   gcgaaactgc gtgcttatgc gctttctgaa cagctgaaag agatcatggc acccctgttc    900
   cagaaacata tggacgacat catctccggc gaattctctt ccggtatgat ggcggactgg    960
   gccaacgatg ataagaaact gctgacctgg cgtgaagaga ccggcaaaac cgcgtttgaa   1020
   accgcgccgc agtatgaagg caaaatcggc gagcaggagt acttcgataa aggcgtactg   1080
   atgattgcga tggtgaaagc gggcgttgaa ctggcgttcg aaaccatggt cgattccggc   1140
   atcattgaag agtctgcata ttatgaatca ctgcacgagc tgccgctgat tgccaacacc   1200
   atcgcccgta agcgtctgta cgaaatgaac gtggttatct ctgataccgc tgagtacggt   1260
   aactatctgt tctcttacgc ttgtgtgccg ttgctgaaac cgtttatggc agagctgcaa   1320
   ccgggcgacc tgggtaaagc tattccggaa ggcgcggtag ataacgggca actgcgtgat   1380
   gtgaacgaag cgattcgcag ccatgcgatt gagcaggtag gtaagaaact gcgcggctat   1440
   atgacagata tgaaacgtat tgctgttgcg ggttaa                             1476
<210> 4
   <211> 491
   <212> PRT
   <213> Escherichia coli
<400> 4
   Met Ala Asn Tyr Phe Asn Thr Leu Asn Leu Arg Gln Gln Leu Ala Gln
   1               5                   10                  15
   Leu Gly Lys Cys Arg Phe Met Gly Arg Asp Glu Phe Ala Asp Gly Ala
               20                  25                  30
   Ser Tyr Leu Gln Gly Lys Lys Val Val Ile Val Gly Cys Gly Ala Gln
           35                  40                  45
   Gly Leu Asn Gln Gly Leu Asn Met Arg Asp Ser Gly Leu Asp Ile Ser
       50                  55                  60
   Tyr Ala Leu Arg Lys Glu Ala Ile Ala Glu Lys Arg Ala Ser Trp Arg
   65                  70                  75                  80
   Lys Ala Thr Glu Asn Gly Phe Lys Val Gly Thr Tyr Glu Glu Leu Ile
                   85                  90                  95
   Pro Gln Ala Asp Leu Val Ile Asn Leu Thr Pro Asp Lys Gln His Ser
               100                 105                 110
   Asp Val Val Arg Thr Val Gln Pro Leu Met Lys Asp Gly Ala Ala Leu
           115                 120                 125
   Gly Tyr Ser His Gly Phe Asn Ile Val Glu Val Gly Glu Gln Ile Arg
       130                 135                 140
   Lys Asp Ile Thr Val Val Met Val Ala Pro Lys Cys Pro Gly Thr Glu
   145                 150                 155                 160
   Val Arg Glu Glu Tyr Lys Arg Gly Phe Gly Val Pro Thr Leu Ile Ala
                   165                 170                 175
   Val His Pro Glu Asn Asp Pro Lys Gly Glu Gly Met Ala Ile Ala Lys
               180                 185                 190
   Ala Trp Ala Ala Ala Thr Gly Gly His Arg Ala Gly Val Leu Glu Ser
           195                 200                 205
   Ser Phe Val Ala Glu Val Lys Ser Asp Leu Met Gly Glu Gln Thr Ile
       210                 215                 220
   Leu Cys Gly Met Leu Gln Ala Gly Ser Leu Leu Cys Phe Asp Lys Leu
   225                 230                 235                 240
   Val Glu Glu Gly Thr Asp Pro Ala Tyr Ala Glu Lys Leu Ile Gln Phe
                   245                 250                 255
   Gly Trp Glu Thr Ile Thr Glu Ala Leu Lys Gln Gly Gly Ile Thr Leu
               260                 265                 270
   Met Met Asp Arg Leu Ser Asn Pro Ala Lys Leu Arg Ala Tyr Ala Leu
           275                 280                 285
   Ser Glu Gln Leu Lys Glu Ile Met Ala Pro Leu Phe Gln Lys His Met
       290                 295                 300
   Asp Asp Ile Ile Ser Gly Glu Phe Ser Ser Gly Met Met Ala Asp Trp
   305                 310                 315                 320
   Ala Asn Asp Asp Lys Lys Leu Leu Thr Trp Arg Glu Glu Thr Gly Lys
                   325                 330                 335
   Thr Ala Phe Glu Thr Ala Pro Gln Tyr Glu Gly Lys Ile Gly Glu Gln
               340                 345                 350
   Glu Tyr Phe Asp Lys Gly Val Leu Met Ile Ala Met Val Lys Ala Gly
           355                 360                 365
   Val Glu Leu Ala Phe Glu Thr Met Val Asp Ser Gly Ile Ile Glu Glu
       370                 375                 380
   Ser Ala Tyr Tyr Glu Ser Leu His Glu Leu Pro Leu Ile Ala Asn Thr
   385                 390                 395                 400
   Ile Ala Arg Lys Arg Leu Tyr Glu Met Asn Val Val Ile Ser Asp Thr
                   405                 410                 415
   Ala Glu Tyr Gly Asn Tyr Leu Phe Ser Tyr Ala Cys Val Pro Leu Leu
               420                 425                 430
   Lys Pro Phe Met Ala Glu Leu Gln Pro Gly Asp Leu Gly Lys Ala Ile
           435                 440                 445
   Pro Glu Gly Ala Val Asp Asn Gly Gln Leu Arg Asp Val Asn Glu Ala
       450                 455                 460
   Ile Arg Ser His Ala Ile Glu Gln Val Gly Lys Lys Leu Arg Gly Tyr
   465                 470                 475                 480
   Met Thr Asp Met Lys Arg Ile Ala Val Ala Gly
   485 490
<210> 5
   <211> 1851
   <212> DNA
   <213> Escherichia coli
<400> 5
   atgcctaagt accgttccgc caccaccact catggtcgta atatggcggg tgctcgtgcg 60
   ctgtggcgcg ccaccggaat gaccgacgcc gatttcggta agccgattat cgcggttgtg 120
   aactcgttca cccaatttgt accgggtcac gtccatctgc gcgatctcgg taaactggtc 180
   gccgaacaaa ttgaagcggc tggcggcgtt gccaaagagt tcaacaccat tgcggtggat 240
   gatgggattg ccatgggcca cggggggatg ctttattcac tgccatctcg cgaactgatc 300
   gctgattccg ttgagtatat ggtcaacgcc cactgcgccg acgccatggt ctgcatctct 360
   aactgcgaca aaatcacccc ggggatgctg atggcttccc tgcgcctgaa tattccggtg 420
   atctttgttt ccggcggccc gatggaggcc gggaaaacca aactttccga tcagatcatc 480
   aagctcgatc tggttgatgc gatgatccag ggcgcagacc cgaaagtatc tgactcccag 540
   agcgatcagg ttgaacgttc cgcgtgtccg acctgcggtt cctgctccgg gatgtttacc 600
   gctaactcaa tgaactgcct gaccgaagcg ctgggcctgt cgcagccggg caacggctcg 660
   ctgctggcaa cccacgccga ccgtaagcag ctgttcctta atgctggtaa acgcattgtt 720
   gaattgacca aacgttatta cgagcaaaac gacgaaagtg cactgccgcg taatatcgcc 780
   agtaaggcgg cgtttgaaaa cgccatgacg ctggatatcg cgatgggtgg atcgactaac 840
   accgtacttc acctgctggc ggcggcgcag gaagcggaaa tcgacttcac catgagtgat 900
   atcgataagc tttcccgcaa ggttccacag ctgtgtaaag ttgcgccgag cacccagaaa 960
   taccatatgg aagatgttca ccgtgctggt ggtgttatcg gtattctcgg cgaactggat 1020
   cgcgcggggt tactgaaccg tgatgtgaaa aacgtacttg gcctgacgtt gccgcaaacg 1080
   ctggaacaat acgacgttat gctgacccag gatgacgcgg taaaaaatat gttccgcgca 1140
   ggtcctgcag gcattcgtac cacacaggca ttctcgcaag attgccgttg ggatacgctg 1200
   gacgacgatc gcgccaatgg ctgtatccgc tcgctggaac acgcctacag caaagacggc 1260
   ggcctggcgg tgctctacgg taactttgcg gaaaacggct gcatcgtgaa aacggcaggc 1320
   gtcgatgaca gcatcctcaa attcaccggc ccggcgaaag tgtacgaaag ccaggacgat 1380
   gcggtagaag cgattctcgg cggtaaagtt gtcgccggag atgtggtagt aattcgctat 1440
   gaaggcccga aaggcggtcc ggggatgcag gaaatgctct acccaaccag cttcctgaaa 1500
   tcaatgggtc tcggcaaagc ctgtgcgctg atcaccgacg gtcgtttctc tggtggcacc 1560
   tctggtcttt ccatcggcca cgtctcaccg gaagcggcaa gcggcggcag cattggcctg 1620
   attgaagatg gtgacctgat cgctatcgac atcccgaacc gtggcattca gttacaggta 1680
   agcgatgccg aactggcggc gcgtcgtgaa gcgcaggacg ctcgaggtga caaagcctgg 1740
   acgccgaaaa atcgtgaacg tcaggtctcc tttgccctgc gtgcttatgc cagcctggca 1800
   accagcgccg acaaaggcgc ggtgcgcgat aaatcgaaac tgggggttta a 1851
<210> 6
   <211> 616
   <212> PRT
   <213> Escherichia coli
<400> 6
   Met Pro Lys Tyr Arg Ser Ala Thr Thr Thr His Gly Arg Asn Met Ala
   1               5                   10                  15
   Gly Ala Arg Ala Leu Trp Arg Ala Thr Gly Met Thr Asp Ala Asp Phe
               20                  25                  30
   Gly Lys Pro Ile Ile Ala Val Val Asn Ser Phe Thr Gln Phe Val Pro
           35                  40                  45
   Gly His Val His Leu Arg Asp Leu Gly Lys Leu Val Ala Glu Gln Ile
       50                  55                  60
   Glu Ala Ala Gly Gly Val Ala Lys Glu Phe Asn Thr Ile Ala Val Asp
   65                  70                  75                  80
   Asp Gly Ile Ala Met Gly His Gly Gly Met Leu Tyr Ser Leu Pro Ser
                   85                  90                  95
   Arg Glu Leu Ile Ala Asp Ser Val Glu Tyr Met Val Asn Ala His Cys
               100                 105                 110
   Ala Asp Ala Met Val Cys Ile Ser Asn Cys Asp Lys Ile Thr Pro Gly
           115                 120                 125
   Met Leu Met Ala Ser Leu Arg Leu Asn Ile Pro Val Ile Phe Val Ser
       130                 135                 140
   Gly Gly Pro Met Glu Ala Gly Lys Thr Lys Leu Ser Asp Gln Ile Ile
   145                 150                 155                 160
   Lys Leu Asp Leu Val Asp Ala Met Ile Gln Gly Ala Asp Pro Lys Val
                   165                 170                 175
   Ser Asp Ser Gln Ser Asp Gln Val Glu Arg Ser Ala Cys Pro Thr Cys
               180                 185                 190
   Gly Ser Cys Ser Gly Met Phe Thr Ala Asn Ser Met Asn Cys Leu Thr
           195                 200                 205
   Glu Ala Leu Gly Leu Ser Gln Pro Gly Asn Gly Ser Leu Leu Ala Thr
       210                 215                 220
   His Ala Asp Arg Lys Gln Leu Phe Leu Asn Ala Gly Lys Arg Ile Val
   225 230 235 240
   Glu Leu Thr Lys Arg Tyr Tyr Glu Gln Asn Asp Glu Ser Ala Leu Pro
   245 250 255
   Arg Asn Ile Ala Ser Lys Ala Ala Phe Glu Asn Ala Met Thr Leu Asp
   260 265 270
   Ile Ala Met Gly Gly Ser Thr Asn Thr Val Leu His Leu Leu Ala Ala
   275 280 285
   Ala Gln Glu Ala Glu Ile Asp Phe Thr Met Ser Asp Ile Asp Lys Leu
   290 295 300
   Ser Arg Lys Val Pro Gln Leu Cys Lys Val Ala Pro Ser Thr Gln Lys
   305 310 315 320
   Tyr His Met Glu Asp Val His Arg Ala Gly Gly Val Ile Gly Ile Leu
   325 330 335
   Gly Glu Leu Asp Arg Ala Gly Leu Leu Asn Arg Asp Val Lys Asn Val
   340 345 350
   Leu Gly Leu Thr Leu Pro Gln Thr Leu Glu Gln Tyr Asp Val Met Leu
   355 360 365
   Thr Gln Asp Asp Ala Val Lys Asn Met Phe Arg Ala Gly Pro Ala Gly
   370 375 380
   Ile Arg Thr Thr Gln Ala Phe Ser Gln Asp Cys Arg Trp Asp Thr Leu
   385 390 395 400
   Asp Asp Asp Arg Ala Asn Gly Cys Ile Arg Ser Leu Glu His Ala Tyr
   405 410 415
   Ser Lys Asp Gly Gly Leu Ala Val Leu Tyr Gly Asn Phe Ala Glu Asn
   420 425 430
   Gly Cys Ile Val Lys Thr Ala Gly Val Asp Asp Ser Ile Leu Lys Phe
   435 440 445
   Thr Gly Pro Ala Lys Val Tyr Glu Ser Gln Asp Asp Ala Val Glu Ala
   450 455 460
   Ile Leu Gly Gly Lys Val Val Ala Gly Asp Val Val Val Ile Arg Tyr
   465 470 475 480
   Glu Gly Pro Lys Gly Gly Pro Gly Met Gln Glu Met Leu Tyr Pro Thr
   485 490 495
   Ser Phe Leu Lys Ser Met Gly Leu Gly Lys Ala Cys Ala Leu Ile Thr
   500 505 510
   Asp Gly Arg Phe Ser Gly Gly Thr Ser Gly Leu Ser Ile Gly His Val
   515 520 525
   Ser Pro Glu Ala Ala Ser Gly Gly Ser Ile Gly Leu Ile Glu Asp Gly
   530 535 540
   Asp Leu Ile Ala Ile Asp Ile Pro Asn Arg Gly Ile Gln Leu Gln Val
   545 550 555 560
   Ser Asp Ala Glu Leu Ala Ala Arg Arg Glu Ala Gln Asp Ala Arg Gly
   565 570 575
   Asp Lys Ala Trp Thr Pro Lys Asn Arg Glu Arg Gln Val Ser Phe Ala
   580 585 590
   Leu Arg Ala Tyr Ala Ser Leu Ala Thr Ser Ala Asp Lys Gly Ala Val
   595 600 605
   Arg Asp Lys Ser Lys Leu Gly Gly
   610 615
<210> 7
   <211> 1662
   <212> DNA
   <213> Artificial Sequence
<220>
   <223> Codon optimized kivD gene from Lactococcus lactis
<400> 7
   tctagacata tgtatactgt gggggattac ctgctggatc gcctgcacga actggggatt 60
   gaagaaattt tcggtgtgcc aggcgattat aacctgcagt tcctggacca gattatctcg 120
   cacaaagata tgaagtgggt cggtaacgcc aacgaactga acgcgagcta tatggcagat 180
   ggttatgccc gtaccaaaaa agctgctgcg tttctgacga cctttggcgt tggcgaactg 240
   agcgccgtca acggactggc aggaagctac gccgagaacc tgccagttgt cgaaattgtt 300
   gggtcgccta cttctaaggt tcagaatgaa ggcaaatttg tgcaccatac tctggctgat 360
   ggggatttta aacattttat gaaaatgcat gaaccggtta ctgcggcccg cacgctgctg 420
   acagcagaga atgctacggt tgagatcgac cgcgtcctgt ctgcgctgct gaaagagcgc 480
   aagccggtat atatcaatct gcctgtcgat gttgccgcag cgaaagccga aaagccgtcg 540
   ctgccactga aaaaagaaaa cagcacctcc aatacatcgg accaggaaat tctgaataaa 600
   atccaggaat cactgaagaa tgcgaagaaa ccgatcgtca tcaccggaca tgagatcatc 660
   tcttttggcc tggaaaaaac ggtcacgcag ttcatttcta agaccaaact gcctatcacc 720
   accctgaact tcggcaaatc tagcgtcgat gaagcgctgc cgagttttct gggtatctat 780
   aatggtaccc tgtccgaacc gaacctgaaa gaattcgtcg aaagcgcgga ctttatcctg 840
   atgctgggcg tgaaactgac ggatagctcc acaggcgcat ttacccacca tctgaacgag 900
   aataaaatga tttccctgaa tatcgacgaa ggcaaaatct ttaacgagcg catccagaac 960
   ttcgattttg aatctctgat tagttcgctg ctggatctgt ccgaaattga gtataaaggt 1020
   aaatatattg ataaaaaaca ggaggatttt gtgccgtcta atgcgctgct gagtcaggat 1080
   cgtctgtggc aagccgtaga aaacctgaca cagtctaatg aaacgattgt tgcggaacag 1140
   ggaacttcat ttttcggcgc ctcatccatt tttctgaaat ccaaaagcca tttcattggc 1200
   caaccgctgt gggggagtat tggttatacc tttccggcgg cgctgggttc acagattgca 1260
   gataaggaat cacgccatct gctgtttatt ggtgacggca gcctgcagct gactgtccag 1320
   gaactggggc tggcgatccg tgaaaaaatc aatccgattt gctttatcat caataacgac 1380
   ggctacaccg tcgaacgcga aattcatgga ccgaatcaaa gttacaatga catcccgatg 1440
   tggaactata gcaaactgcc ggaatccttt ggcgcgacag aggatcgcgt ggtgagtaaa 1500
   attgtgcgta cggaaaacga atttgtgtcg gttatgaaag aagcgcaggc tgacccgaat 1560
   cgcatgtatt ggattgaact gatcctggca aaagaaggcg caccgaaagt tctgaaaaag 1620
   atggggaaac tgtttgcgga gcaaaataaa agctaaggat cc 1662
<210> 8
   <211> 548
   <212> PRT
   <213> Lactococcus lactis
<400> 8
   Met Tyr Thr Val Gly Asp Tyr Leu Leu Asp Arg Leu His Glu Leu Gly
   1               5                   10                  15
   Ile Glu Glu Ile Phe Gly Val Pro Gly Asp Tyr Asn Leu Gln Phe Leu
               20                  25                  30
   Asp Gln Ile Ile Ser His Lys Asp Met Lys Trp Val Gly Asn Ala Asn
           35                  40                  45
   Glu Leu Asn Ala Ser Tyr Met Ala Asp Gly Tyr Ala Arg Thr Lys Lys
       50                  55                  60
   Ala Ala Ala Phe Leu Thr Thr Phe Gly Val Gly Glu Leu Ser Ala Val
   65                  70                  75                  80
   Asn Gly Leu Ala Gly Ser Tyr Ala Glu Asn Leu Pro Val Val Glu Ile
                   85                  90                  95
   Val Gly Ser Pro Thr Ser Lys Val Gln Asn Glu Gly Lys Phe Val His
               100                 105                 110
   His Thr Leu Ala Asp Gly Asp Phe Lys His Phe Met Lys Met His Glu
           115                 120                 125
   Pro Val Thr Ala Ala Arg Thr Leu Leu Thr Ala Glu Asn Ala Thr Val
       130                 135                 140
   Glu Ile Asp Arg Val Leu Ser Ala Leu Leu Lys Glu Arg Lys Pro Val
   145                 150                 155                 160
   Tyr Ile Asn Leu Pro Val Asp Val Ala Ala Ala Lys Ala Glu Lys Pro
                   165                 170                 175
   Ser Leu Pro Leu Lys Lys Glu Asn Ser Thr Ser Asn Thr Ser Asp Gln
               180                 185                 190
   Glu Ile Leu Asn Lys Ile Gln Glu Ser Leu Lys Asn Ala Lys Lys Pro
           195                 200                 205
   Ile Val Ile Thr Gly His Glu Ile Ile Ser Phe Gly Leu Glu Lys Thr
       210                 215                 220
   Val Thr Gln Phe Ile Ser Lys Thr Lys Leu Pro Ile Thr Thr Leu Asn
   225                 230                 235                 240
   Phe Gly Lys Ser Ser Val Asp Glu Ala Leu Pro Ser Phe Leu Gly Ile
                   245                 250                 255
   Tyr Asn Gly Thr Leu Ser Glu Pro Asn Leu Lys Glu Phe Val Glu Ser
               260                 265                 270
   Ala Asp Phe Ile Leu Met Leu Gly Val Lys Leu Thr Asp Ser Ser Thr
           275                 280                 285
   Gly Ala Phe Thr His His Leu Asn Glu Asn Lys Met Ile Ser Leu Asn
       290                 295                 300
   Ile Asp Glu Gly Lys Ile Phe Asn Glu Arg Ile Gln Asn Phe Asp Phe
   305                 310                 315                 320
   Glu Ser Leu Ile Ser Ser Leu Leu Asp Leu Ser Glu Ile Glu Tyr Lys
                   325                 330                 335
   Gly Lys Tyr Ile Asp Lys Lys Gln Glu Asp Phe Val Pro Ser Asn Ala
               340                 345                 350
   Leu Leu Ser Gln Asp Arg Leu Trp Gln Ala Val Glu Asn Leu Thr Gln
           355                 360                 365
   Ser Asn Glu Thr Ile Val Ala Glu Gln Gly Thr Ser Phe Phe Gly Ala
       370                 375                 380
   Ser Ser Ile Phe Leu Lys Ser Lys Ser His Phe Ile Gly Gln Pro Leu
   385                 390                 395                 400
   Trp Gly Ser Ile Gly Tyr Thr Phe Pro Ala Ala Leu Gly Ser Gln Ile
   405 410 415
   Ala Asp Lys Glu Ser Arg His Leu Leu Phe Ile Gly Asp Gly Ser Leu
   420 425 430
   Gln Leu Thr Val Gln Glu Leu Gly Leu Ala Ile Arg Glu Lys Ile Asn
   435 440 445
   Pro Ile Cys Phe Ile Ile Asn Asn Asp Gly Tyr Thr Val Glu Arg Glu
   450 455 460
   Ile His Gly Pro Asn Gln Ser Tyr Asn Asp Ile Pro Met Trp Asn Tyr
   465 470 475 480
   Ser Lys Leu Pro Glu Ser Phe Gly Ala Thr Glu Asp Arg Val Val Ser
   485 490 495
   Lys Ile Val Arg Thr Glu Asn Glu Phe Val Ser Val Met Lys Glu Ala
   500 505 510
   Gln Ala Asp Pro Asn Arg Met Tyr Trp Ile Glu Leu Ile Leu Ala Lys
   515 520 525
   Glu Gly Ala Pro Lys Val Leu Lys Lys Met Gly Lys Leu Phe Ala Glu
   530 535 540
   Gln Asn Lys Ser
   545
<210> 9
   <211> 1047
   <212> DNA
   <213> Achromobacter xylosoxidans
<400> 9
   atgaaagctc tggtttatca cggtgaccac aagatctcgc ttgaagacaa gcccaagccc 60
   acccttcaaa agcccacgga tgtagtagta cgggttttga agaccacgat ctgcggcacg 120
   gatctcggca tctacaaagg caagaatcca gaggtcgccg acgggcgcat cctgggccat 180
   gaaggggtag gcgtcatcga ggaagtgggc gagagtgtca cgcagttcaa gaaaggcgac 240
   aaggtcctga tttcctgcgt cacttcttgc ggctcgtgcg actactgcaa gaagcagctt 300
   tactcccatt gccgcgacgg cgggtggatc ctgggttaca tgatcgatgg cgtgcaggcc 360
   gaatacgtcc gcatcccgca tgccgacaac agcctctaca agatccccca gacaattgac 420
   gacgaaatcg ccgtcctgct gagcgacatc ctgcccaccg gccacgaaat cggcgtccag 480
   tatgggaatg tccagccggg cgatgcggtg gctattgtcg gcgcgggccc cgtcggcatg 540
   tccgtactgt tgaccgccca gttctactcc ccctcgacca tcatcgtgat cgacatggac 600
   gagaatcgcc tccagctcgc caaggagctc ggggcaacgc acaccatcaa ctccggcacg 660
   gagaacgttg tcgaagccgt gcataggatt gcggcagagg gagtcgatgt tgcgatcgag 720
   gcggtgggca taccggcgac ttgggacatc tgccaggaga tcgtcaagcc cggcgcgcac 780
   atcgccaacg tcggcgtgca tggcgtcaag gttgacttcg agattcagaa gctctggatc 840
   aagaacctga cgatcaccac gggactggtg aacacgaaca cgacgcccat gctgatgaag 900
   gtcgcctcga ccgacaagct tccgttgaag aagatgatta cccatcgctt cgagctggcc 960
   gagatcgagc acgcctatca ggtattcctc aatggcgcca aggagaaggc gatgaagatc 1020
   atcctctcga acgcaggcgc tgcctga 1047
<210> 10
   <211> 348
   <212> PRT
   <213> Achromobacter xylosoxidans
<400> 10
   Met Lys Ala Leu Val Tyr His Gly Asp His Lys Ile Ser Leu Glu Asp
   1               5                   10                  15
   Lys Pro Lys Pro Thr Leu Gln Lys Pro Thr Asp Val Val Val Arg Val
               20                  25                  30
   Leu Lys Thr Thr Ile Cys Gly Thr Asp Leu Gly Ile Tyr Lys Gly Lys
           35                  40                  45
   Asn Pro Glu Val Ala Asp Gly Arg Ile Leu Gly His Glu Gly Val Gly
       50                  55                  60
   Val Ile Glu Glu Val Gly Glu Ser Val Thr Gln Phe Lys Lys Gly Asp
   65                  70                  75                  80
   Lys Val Leu Ile Ser Cys Val Thr Ser Cys Gly Ser Cys Asp Tyr Cys
                   85                  90                  95
   Lys Lys Gln Leu Tyr Ser His Cys Arg Asp Gly Gly Trp Ile Leu Gly
               100                 105                 110
   Tyr Met Ile Asp Gly Val Gln Ala Glu Tyr Val Arg Ile Pro His Ala
           115                 120                 125
   Asp Asn Ser Leu Tyr Lys Ile Pro Gln Thr Ile Asp Asp Glu Ile Ala
       130                 135                 140
   Val Leu Leu Ser Asp Ile Leu Pro Thr Gly His Glu Ile Gly Val Gln
   145                 150                 155                 160
   Tyr Gly Asn Val Gln Pro Gly Asp Ala Val Ala Ile Val Gly Ala Gly
                   165                 170                 175
   Pro Val Gly Met Ser Val Leu Leu Thr Ala Gln Phe Tyr Ser Pro Ser
   180 185 190
   Thr Ile Ile Val Ile Asp Met Asp Glu Asn Arg Leu Gln Leu Ala Lys
   195 200 205
   Glu Leu Gly Ala Thr His Thr Ile Asn Ser Gly Thr Glu Asn Val Val
   210 215 220
   Glu Ala Val His Arg Ile Ala Ala Glu Gly Val Asp Val Ala Ile Glu
   225 230 235 240
   Ala Val Gly Ile Pro Ala Thr Trp Asp Ile Cys Gln Glu Ile Val Lys
   245 250 255
   Pro Gly Ala His Ile Ala Asn Val Gly Val His Gly Val Lys Val Asp
   260 265 270
   Phe Glu Ile Gln Lys Leu Trp Ile Lys Asn Leu Thr Ile Thr Thr Gly
   275 280 285
   Leu Val Asn Thr Asn Thr Thr Pro Met Leu Met Lys Val Ala Ser Thr
   290 295 300
   Asp Lys Leu Pro Leu Lys Lys Met Ile Thr His Arg Phe Glu Leu Ala
   305 310 315 320
   Glu Ile Glu His Ala Tyr Gln Val Phe Leu Asn Gly Ala Lys Glu Lys
   325 330 335
   Ala Met Lys Ile Ile Leu Ser Asn Ala Gly Ala Ala
   340 345
<210> 11
   <211> 24
   <212> DNA
   <213> artificial sequence
<220>
   <223> primer
<400> 11
   tcatcactga taacctgatt ccgg 24
<210> 12
   <211> 26
   <212> DNA
   <213> artificial sequence
<220>
   <223> primer
<400> 12
   cgagtctgtt ttggcagtca ccttaa 26
<210> 13
   <211> 23
   <212> DNA
   <213> artificial sequence
<220>
   <223> primer
<400> 13
   gagcgtgacg acgtcaactt cct 23
<210> 14
   <211> 23
   <212> DNA
   <213> artificial sequence
<220>
   <223> primer
<400> 14
   cagttcaatg ctgaaccaca cag 23
<210> 15
   <211> 23
   <212> DNA
   <213> artificial sequence
<220>
   <223> primer
<400> 15
   gaaggttgcg cctacactaa gca 23
<210> 16
   <211> 23
   <212> DNA
   <213> artificial sequence
<220>
   <223> primer
<400> 16
   gggagcggca agattaaacc agt 23
<210> 17
   <211> 23
   <212> DNA
   <213> artificial sequence
<220>
   <223> primer
<400> 17
   tggatcacgt aatcagtacc cag 23
<210> 18
   <211> 23
   <212> DNA
   <213> artificial sequence
<220>
   <223> primer
<400> 18
   atccttaact gatcggcatt gcc 23
<210> 19
   <211> 30
   <212> DNA
   <213> artificial sequence
<220>
   <223> primer
<400> 19
   ggaattcaca catgaaagct ctggtttatc 30
<210> 20
   <211> 28
   <212> DNA
   <213> artificial sequence
<220>
   <223> primer
<400> 20
   gcgtccaggg cgtcaaagat caggcagc 28
<210> 21
   <211> 30
   <212> DNA
   <213> artificial sequence
<220>
   <223> primer
<400> 21
   gacctaggag gtcacacatg aaagctctgg 30
<210> 22
   <211> 25
   <212> DNA
   <213> artificial sequence
<220>
   <223> primer
<400> 22
   cgactctaga ggatccccgg gtacc 25
<210> 23
   <211> 2283
   <212> DNA
   <213> Escherichia coli
<400> 23
   atgtccgagc ttaatgaaaa gttagccaca gcctgggaag gttttaccaa aggtgactgg     60
   cagaatgaag taaacgtccg tgacttcatt cagaaaaact acactccgta cgagggtgac    120
   gagtccttcc tggctggcgc tactgaagcg accaccaccc tgtgggacaa agtaatggaa    180
   ggcgttaaac tggaaaaccg cactcacgcg ccagttgact ttgacaccgc tgttgcttcc    240
   accatcacct ctcacgacgc tggctacatc aacaagcagc ttgagaaaat cgttggtctg    300
   cagactgaag ctccgctgaa acgtgctctt atcccgttcg gtggtatcaa aatgatcgaa    360
   ggttcctgca aagcgtacaa ccgcgaactg gatccgatga tcaaaaaaat cttcactgaa     420
   taccgtaaaa ctcacaacca gggcgtgttc gacgtttaca ctccggacat cctgcgttgc     480
   cgtaaatctg gtgttctgac cggtctgcca gatgcatatg gccgtggccg tatcatcggt     540
   gactaccgtc gcgttgcgct gtacggtatc gactacctga tgaaagacaa actggcacag     600
   ttcacttctc tgcaggctga tctggaaaac ggcgtaaacc tggaacagac tatccgtctg     660
   cgcgaagaaa tcgctgaaca gcaccgcgct ctgggtcaga tgaaagaaat ggctgcgaaa     720
   tacggctacg acatctctgg tccggctacc aacgctcagg aagctatcca gtggacttac     780
   ttcggctacc tggctgctgt taagtctcag aacggtgctg caatgtcctt cggtcgtacc     840
   tccaccttcc tggatgtgta catcgaacgt gacctgaaag ctggcaagat caccgaacaa     900
   gaagcgcagg aaatggttga ccacctggtc atgaaactgc gtatggttcg cttcctgcgt     960
   actccggaat acgatgaact gttctctggc gacccgatct gggcaaccga atctatcggt    1020
   ggtatgggcc tcgacggtcg taccctggtt accaaaaaca gcttccgttt cctgaacacc    1080
   ctgtacacca tgggtccgtc tccggaaccg aacatgacca ttctgtggtc tgaaaaactg    1140
   ccgctgaact tcaagaaatt cgccgctaaa gtgtccatcg acacctcttc tctgcagtat    1200
   gagaacgatg acctgatgcg tccggacttc aacaacgatg actacgctat tgcttgctgc    1260
   gtaagcccga tgatcgttgg taaacaaatg cagttcttcg gtgcgcgtgc aaacctggcg    1320
   aaaaccatgc tgtacgcaat caacggcggc gttgacgaaa aactgaaaat gcaggttggt    1380
   ccgaagtctg aaccgatcaa aggcgatgtc ctgaactatg atgaagtgat ggagcgcatg    1440
   gatcacttca tggactggct ggctaaacag tacatcactg cactgaacat catccactac    1500
   atgcacgaca agtacagcta cgaagcctct ctgatggcgc tgcacgaccg tgacgttatc    1560
   cgcaccatgg cgtgtggtat cgctggtctg tccgttgctg ctgactccct gtctgcaatc    1620
   aaatatgcga aagttaaacc gattcgtgac gaagacggtc tggctatcga cttcgaaatc    1680
   gaaggcgaat acccgcagtt tggtaacaat gatccgcgtg tagatgacct ggctgttgac    1740
   ctggtagaac gtttcatgaa gaaaattcag aaactgcaca cctaccgtga cgctatcccg    1800
   actcagtctg ttctgaccat cacttctaac gttgtgtatg gtaagaaaac gggtaacacc    1860
   ccagacggtc gtcgtgctgg cgcgccgttc ggaccgggtg ctaacccgat gcacggtcgt    1920
   gaccagaaag gtgcagtagc ctctctgact tccgttgcta aactgccgtt tgcttacgct    1980
   aaagatggta tctcctacac cttctctatc gttccgaacg cactgggtaa agacgacgaa    2040
   gttcgtaaga ccaacctggc tggtctgatg gatggttact tccaccacga agcatccatc    2100
   gaaggtggtc agcacctgaa cgttaacgtg atgaaccgtg aaatgctgct cgacgcgatg    2160
   gaaaacccgg aaaaatatcc gcagctgacc atccgtgtat ctggctacgc agtacgtttc    2220
   aactcgctga ctaaagaaca gcagcaggac gttattactc gtaccttcac tcaatctatg    2280
   taa                                                                  2283
<210> 24
   <211> 990
   <212> DNA
   <213> Escherichia coli
<400> 24
   atgaaactcg ccgtttatag cacaaaacag tacgacaaga agtacctgca acaggtgaac     60
   gagtcctttg gctttgagct ggaatttttt gactttctgc tgacggaaaa aaccgctaaa    120
   actgccaatg gctgcgaagc ggtatgtatt ttcgtaaacg atgacggcag ccgcccggtg    180
   ctggaagagc tgaaaaagca cggcgttaaa tatatcgccc tgcgctgtgc cggtttcaat    240
   aacgtcgacc ttgacgcggc aaaagaactg gggctgaaag tagtccgtgt tccagcctat    300
   gatccagagg ccgttgctga acacgccatc ggtatgatga tgacgctgaa ccgccgtatt    360
   caccgcgcgt atcagcgtac ccgtgatgct aacttctctc tggaaggtct gaccggcttt    420
   actatgtatg gcaaaacggc aggcgttatc ggtaccggta aaatcggtgt ggcgatgctg    480
   cgcattctga aaggttttgg tatgcgtctg ctggcgttcg atccgtatcc aagtgcagcg    540
   gcgctggaac tcggtgtgga gtatgtcgat ctgccaaccc tgttctctga atcagacgtt    600
   atctctctgc actgcccgct gacaccggaa aactatcatc tgttgaacga agccgccttc    660
   gaacagatga aaaatggcgt gatgatcgtc aataccagtc gcggtgcatt gattgattct    720
   caggcagcaa ttgaagcgct gaaaaatcag aaaattggtt cgttgggtat ggacgtgtat    780
   gagaacgaac gcgatctatt ctttgaagat aaatccaacg acgtgatcca ggatgacgta    840
   ttccgtcgcc tgtctgcctg ccacaacgtg ctgtttaccg ggcaccaggc attcctgaca    900
   gcagaagctc tgaccagtat ttctcagact acgctgcaaa acttaagcaa tctggaaaaa    960
   ggcgaaacct gcccgaacga actggtttaa                                     990
<210> 25
   <211> 2676
   <212> DNA
   <213> Escherichia coli
<400> 25
   atggctgtta ctaatgtcgc tgaacttaac gcactcgtag agcgtgtaaa aaaagcccag    60
   cgtgaatatg ccagtttcac tcaagagcaa gtagacaaaa tcttccgcgc cgccgctctg   120
   gctgctgcag atgctcgaat cccactcgcg aaaatggccg ttgccgaatc cggcatgggt   180
   atcgtcgaag ataaagtgat caaaaaccac tttgcttctg aatatatcta caacgcctat   240
   aaagatgaaa aaacctgtgg tgttctgtct gaagacgaca cttttggtac catcactatc   300
   gctgaaccaa tcggtattat ttgcggtatc gttccgacca ctaacccgac ttcaactgct   360
   atcttcaaat cgctgatcag tctgaagacc cgtaacgcca ttatcttctc cccgcacccg   420
   cgtgcaaaag atgccaccaa caaagcggct gatatcgttc tgcaggctgc tatcgctgcc   480
   ggtgctccga aagatctgat cggctggatc gatcaacctt ctgttgaact gtctaacgca   540
   ctgatgcacc acccagacat caacctgatc ctcgcgactg gtggtccggg catggttaaa   600
   gccgcataca gctccggtaa accagctatc ggtgtaggcg cgggcaacac tccagttgtt   660
   atcgatgaaa ctgctgatat caaacgtgca gttgcatctg tactgatgtc caaaaccttc 720
   gacaacggcg taatctgtgc ttctgaacag tctgttgttg ttgttgactc tgtttatgac 780
   gctgtacgtg aacgttttgc aacccacggc ggctatctgt tgcagggtaa agagctgaaa 840
   gctgttcagg atgttatcct gaaaaacggt gcgctgaacg cggctatcgt tggtcagcca 900
   gcctataaaa ttgctgaact ggcaggcttc tctgtaccag aaaacaccaa gattctgatc 960
   ggtgaagtga ccgttgttga tgaaagcgaa ccgttcgcac atgaaaaact gtccccgact 1020
   ctggcaatgt accgcgctaa agatttcgaa gacgcggtag aaaaagcaga gaaactggtt 1080
   gctatgggcg gtatcggtca tacctcttgc ctgtacactg accaggataa ccaaccggct 1140
   cgcgtttctt acttcggtca gaaaatgaaa acggcgcgta tcctgattaa caccccagcg 1200
   tctcagggtg gtatcggtga cctgtataac ttcaaactcg caccttccct gactctgggt 1260
   tgtggttctt ggggtggtaa ctccatctct gaaaacgttg gtccgaaaca cctgatcaac 1320
   aagaaaaccg ttgctaagcg agctgaaaac atgttgtggc acaaacttcc gaaatctatc 1380
   tacttccgcc gtggctccct gccaatcgcg ctggatgaag tgattactga tggccacaaa 1440
   cgtgcgctca tcgtgactga ccgcttcctg ttcaacaatg gttatgctga tcagatcact 1500
   tccgtactga aagcagcagg cgttgaaact gaagtcttct tcgaagtaga agcggacccg 1560
   accctgagca tcgttcgtaa aggtgcagaa ctggcaaact ccttcaaacc agacgtgatt 1620
   atcgcgctgg gtggtggttc cccgatggac gccgcgaaga tcatgtgggt tatgtacgaa 1680
   catccggaaa ctcacttcga agagctggcg ctgcgcttta tggatatccg taaacgtatc 1740
   tacaagttcc cgaaaatggg cgtgaaagcg aaaatgatcg ctgtcaccac cacttctggt 1800
   acaggttctg aagtcactcc gtttgcggtt gtaactgacg acgctactgg tcagaaatat 1860
   ccgctggcag actatgcgct gactccggat atggcgattg tcgacgccaa cctggttatg 1920
   gacatgccga agtccctgtg tgctttcggt ggtctggacg cagtaactca cgccatggaa 1980
   gcttatgttt ctgtactggc atctgagttc tctgatggtc aggctctgca ggcactgaaa 2040
   ctgctgaaag aatatctgcc agcgtcctac cacgaagggt ctaaaaatcc ggtagcgcgt 2100
   gaacgtgttc acagtgcagc gactatcgcg ggtatcgcgt ttgcgaacgc cttcctgggt 2160
   gtatgtcact caatggcgca caaactgggt tcccagttcc atattccgca cggtctggca 2220
   aacgccctgc tgatttgtaa cgttattcgc tacaatgcga acgacaaccc gaccaagcag 2280
   actgcattca gccagtatga ccgtccgcag gctcgccgtc gttatgctga aattgccgac 2340
   cacttgggtc tgagcgcacc gggcgaccgt actgctgcta agatcgagaa actgctggca 2400
   tggctggaaa cgctgaaagc tgaactgggt attccgaaat ctatccgtga agctggcgtt 2460
   caggaagcag acttcctggc gaacgtggat aaactgtctg aagatgcatt cgatgaccag 2520
   tgcaccggcg ctaacccgcg ttacccgctg atctccgagc tgaaacagat tctgctggat 2580
   acctactacg gtcgtgatta tgtagaaggt gaaactgcag cgaagaaaga agctgctccg 2640
   gctaaagctg agaaaaaagc gaaaaaatcc gcttaa 2676
<210> 26
   <211> 1809
   <212> DNA
   <213> Escherichia coli
<400> 26
   gtgcaaacct ttcaagccga tcttgccatt gtaggcgccg gtggcgcggg attacgtgct     60
   gcaattgctg ccgcgcaggc aaatccgaat gcaaaaatcg cactaatctc aaaagtatac    120
   ccgatgcgta gccataccgt tgctgcagaa gggggctccg ccgctgtcgc gcaggatcat    180
   gacagcttcg aatatcactt tcacgataca gtagcgggtg gcgactggtt gtgtgagcag    240
   gatgtcgtgg attatttcgt ccaccactgc ccaaccgaaa tgacccaact ggaactgtgg    300
   ggatgcccat ggagccgtcg cccggatggt agcgtcaacg tacgtcgctt cggcggcatg    360
   aaaatcgagc gcacctggtt cgccgccgat aagaccggct tccatatgct gcacacgctg    420
   ttccagacct ctctgcaatt cccgcagatc cagcgttttg acgaacattt cgtgctggat    480
   attctggttg atgatggtca tgttcgcggc ctggtagcaa tgaacatgat ggaaggcacg    540
   ctggtgcaga tccgtgctaa cgcggtcgtt atggctactg gcggtgcggg tcgcgtttat    600
   cgttacaaca ccaacggcgg catcgttacc ggtgacggta tgggtatggc gctaagccac    660
   ggcgttccgc tgcgtgacat ggaattcgtt cagtatcacc caaccggtct gccaggttcc    720
   ggtatcctga tgaccgaagg ttgccgcggt gaaggcggta ttctggtcaa caaaaatggc    780
   taccgttatc tgcaagatta cggcatgggc ccggaaactc cgctgggcga gccgaaaaac    840
   aaatatatgg aactgggtcc acgcgacaaa gtctctcagg ccttctggca cgaatggcgt    900
   aaaggcaaca ccatctccac gccgcgtggc gatgtggttt atctcgactt gcgtcacctc    960
   ggcgagaaaa aactgcatga acgtctgccg ttcatctgcg aactggcgaa agcgtacgtt   1020
   ggcgtcgatc cggttaaaga accgattccg gtacgtccga ccgcacacta caccatgggc   1080
   ggtatcgaaa ccgatcagaa ctgtgaaacc cgcattaaag gtctgttcgc cgtgggtgaa   1140
   tgttcctctg ttggtctgca cggtgcaaac cgtctgggtt ctaactccct ggcggaactg   1200
   gtggtcttcg gccgtctggc cggtgaacaa gcgacagagc gtgcagcaac tgccggtaat   1260
   ggcaacgaag cggcaattga agcgcaggca gctggcgttg aacaacgtct gaaagatctg   1320
   gttaaccagg atggcggcga aaactgggcg aagatccgcg acgaaatggg cctggctatg   1380
   gaagaaggct gcggtatcta ccgtacgccg gaactgatgc agaaaaccat cgacaagctg   1440
   gcagagctgc aggaacgctt caagcgcgtg cgcatcaccg acacttccag cgtgttcaac   1500
   accgacctgc tctacaccat tgaactgggc cacggtctga acgttgctga atgtatggcg   1560
   cactccgcaa tggcacgtaa agagtcccgc ggcgcgcacc agcgtctgga cgaaggttgc   1620
   accgagcgtg acgacgtcaa cttcctcaaa cacaccctcg ccttccgcga tgctgatggc   1680
   acgactcgcc tggagtacag cgacgtgaag attactacgc tgccgccagc taaacgcgtt   1740
   tacggtggcg aagcggatgc agccgataag gcggaagcag ccaataagaa ggagaaggcg   1800
   aatggctga 1809
<210> 27
   <211> 735
   <212> DNA
   <213> Escherichia coli
<400> 27
   atggctgaga tgaaaaacct gaaaattgag gtggtgcgct ataacccgga agtcgatacc 60
   gcaccgcata gcgcattcta tgaagtgcct tatgacgcaa ctacctcatt actggatgcg 120
   ctgggctaca tcaaagacaa cctggcaccg gacctgagct accgctggtc ctgccgtatg 180
   gcgatttgtg gttcctgcgg catgatggtt aacaacgtgc caaaactggc atgtaaaacc 240
   ttcctgcgtg attacaccga cggtatgaag gttgaagcgt tagctaactt cccgattgaa 300
   cgcgatctgg tggtcgatat gacccacttc atcgaaagtc tggaagcgat caaaccgtac 360
   atcatcggca actcccgcac cgcggatcag ggtactaaca tccagacccc ggcgcagatg 420
   gcgaagtatc accagttctc cggttgcatc aactgtggtt tgtgctacgc cgcgtgcccg 480
   cagtttggcc tgaacccaga gttcatcggt ccggctgcca ttacgctggc gcatcgttat 540
   aacgaagata gccgcgacca cggtaagaag gagcgtatgg cgcagttgaa cagccagaac 600
   ggcgtatgga gctgtacttt cgtgggctac tgctccgaag tctgcccgaa acacgtcgat 660
   ccggctgcgg ccattcagca gggcaaagta gaaagttcga aagactttct tatcgcgacc 720
   ctgaaaccac gctaa 735
<210> 28
   <211> 396
   <212> DNA
   <213> Escherichia coli
<400> 28
   atgacgacta aacgtaaacc gtatgtacgg ccaatgacgt ccacctggtg gaaaaaattg 60
   ccgttttatc gcttttacat gctgcgcgaa ggcacggcgg ttccggctgt gtggttcagc 120
   attgaactga ttttcgggct gtttgccctg aaaaatggcc cggaagcctg ggcgggattc 180
   gtcgactttt tacaaaaccc ggttatcgtg atcattaacc tgatcactct ggcggcagct 240
   ctgctgcaca ccaaaacctg gtttgaactg gcaccgaaag cggccaatat cattgtaaaa 300
   gacgaaaaaa tgggaccaga gccaattatc aaaagtctct gggcggtaac tgtggttgcc 360
   accatcgtaa tcctgtttgt tgccctgtac tggtaa 396
<210> 29
   <211> 360
   <212> DNA
   <213> Escherichia coli
<400> 29
   atgattaatc caaatccaaa gcgttctgac gaaccggtat tctggggcct cttcggggcc 60
   ggtggtatgt ggagcgccat cattgcgccg gtgatgatcc tgctggtggg tattctgctg 120
   ccactggggt tgtttccggg tgatgcgctg agctacgagc gcgttctggc gttcgcgcag 180
   agcttcattg gtcgcgtatt cctgttcctg atgatcgttc tgccgctgtg gtgtggttta 240
   caccgtatgc accacgcgat gcacgatctg aaaatccacg tacctgcggg caaatgggtt 300
   ttctacggtc tggctgctat cctgacagtt gtcacgctga ttggtgtcgt tacaatctaa 360
<210> 30
   <211> 16387
   <212> DNA
   <213> artificial sequence
<220>
   <223> plasmid construct
<400> 30
   tcccattacc gacatttggg cgctatacgt gcatatgttc atgtatgtat ctgtatttaa 60
   aacacttttg tattattttt cctcatatat gtgtataggt ttatacggat gatttaatta 120
   ttacttcacc accctttatt tcaggctgat atcttagcct tgttactagt tagaaaaaga 180
   catttttgct gtcagtcact gtcaagagat tcttttgctg gcatttcttc tagaagcaaa 240
   aagagcgatg cgtcttttcc gctgaaccgt tccagcaaaa aagactacca acgcaatatg 300
   gattgtcaga atcatataaa agagaagcaa ataactcctt gtcttgtatc aattgcatta 360
   taatatcttc ttgttagtgc aatatcatat agaagtcatc gaaatagata ttaagaaaaa 420
   caaactgtac aatcaatcaa tcaatcatcg ctgaggatgt tgacaaaagc aacaaaagaa 480
   caaaaatccc ttgtgaaaaa cagaggggcg gagcttgttg ttgattgctt agtggagcaa 540
   ggtgtcacac atgtatttgg cattccaggt gcaaaaattg atgcggtatt tgacgcttta 600
   caagataaag gacctgaaat tatcgttgcc cggcacgaac aaaacgcagc attcatggcc 660
   caagcagtcg gccgtttaac tggaaaaccg ggagtcgtgt tagtcacatc aggaccgggt 720
   gcctctaact tggcaacagg cctgctgaca gcgaacactg aaggagaccc tgtcgttgcg 780
   cttgctggaa acgtgatccg tgcagatcgt ttaaaacgga cacatcaatc tttggataat 840
   gcggcgctat tccagccgat tacaaaatac agtgtagaag ttcaagatgt aaaaaatata 900
   ccggaagctg ttacaaatgc atttaggata gcgtcagcag ggcaggctgg ggccgctttt 960
   gtgagctttc cgcaagatgt tgtgaatgaa gtcacaaata cgaaaaacgt gcgtgctgtt 1020
   gcagcgccaa aactcggtcc tgcagcagat gatgcaatca gtgcggccat agcaaaaatc 1080
   caaacagcaa aacttcctgt cgttttggtc ggcatgaaag gcggaagacc ggaagcaatt 1140
   aaagcggttc gcaagctttt gaaaaaggtt cagcttccat ttgttgaaac atatcaagct 1200
   gccggtaccc tttctagaga tttagaggat caatattttg gccgtatcgg tttgttccgc 1260
   aaccagcctg gcgatttact gctagagcag gcagatgttg ttctgacgat cggctatgac 1320
   ccgattgaat atgatccgaa attctggaat atcaatggag accggacaat tatccattta 1380
   gacgagatta tcgctgacat tgatcatgct taccagcctg atcttgaatt gatcggtgac 1440
   attccgtcca cgatcaatca tatcgaacac gatgctgtga aagtggaatt tgcagagcgt 1500
   gagcagaaaa tcctttctga tttaaaacaa tatatgcatg aaggtgagca ggtgcctgca 1560
   gattggaaat cagacagagc gcaccctctt gaaatcgtta aagagttgcg taatgcagtc 1620
   gatgatcatg ttacagtaac ttgcgatatc ggttcgcacg ccatttggat gtcacgttat 1680
   ttccgcagct acgagccgtt aacattaatg atcagtaacg gtatgcaaac actcggcgtt 1740
   gcgcttcctt gggcaatcgg cgcttcattg gtgaaaccgg gagaaaaagt ggtttctgtc 1800
   tctggtgacg gcggtttctt attctcagca atggaattag agacagcagt tcgactaaaa 1860
   gcaccaattg tacacattgt atggaacgac agcacatatg acatggttgc attccagcaa 1920
   ttgaaaaaat ataaccgtac atctgcggtc gatttcggaa atatcgatat cgtgaaatat 1980
   gcggaaagct tcggagcaac tggcttgcgc gtagaatcac cagaccagct ggcagatgtt 2040
   ctgcgtcaag gcatgaacgc tgaaggtcct gtcatcatcg atgtcccggt tgactacagt 2100
   gataacatta atttagcaag tgacaagctt ccgaaagaat tcggggaact catgaaaacg 2160
   aaagctctct agttaattaa tcatgtaatt agttatgtca cgcttacatt cacgccctcc 2220
   ccccacatcc gctctaaccg aaaaggaagg agttagacaa cctgaagtct aggtccctat 2280
   ttattttttt atagttatgt tagtattaag aacgttattt atatttcaaa tttttctttt 2340
   ttttctgtac agacgcgtgt acgcatgtaa cattatactg aaaaccttgc ttgagaaggt 2400
   tttgggacgc tcgaaggctt taatttgcgg gcggccgctc tagaactagt accacaggtg 2460
   ttgtcctctg aggacataaa atacacaccg agattcatca actcattgct ggagttagca 2520
   tatctacaat tgggtgaaat ggggagcgat ttgcaggcat ttgctcggca tgccggtaga 2580
   ggtgtggtca ataagagcga cctcatgcta tacctgagaa agcaacctga cctacaggaa 2640
   agagttactc aagaataaga attttcgttt taaaacctaa gagtcacttt aaaatttgta 2700
   tacacttatt ttttttataa cttatttaat aataaaaatc ataaatcata agaaattcgc 2760
   ttactcttaa ttaatcaagc atctaaaaca caaccgttgg aagcgttgga aaccaactta 2820
   gcatacttgg atagagtacc tcttgtgtaa cgaggtggag gtgcaaccca actttgttta 2880
   cgttgagcca tttccttatc agagactaat aggtcaatct tgttattatc agcatcaatg 2940
   ataatctcat cgccgtctct gaccaacccg ataggaccac cttcagcggc ttcgggaaca 3000
   atgtggccga ttaagaaccc gtgagaacca ccagagaatc taccatcagt caacaatgca 3060
   acatctttac ccaaaccgta acccatcaga gcagaggaag gctttagcat ttcaggcata 3120
   cctggtgcac ctcttggacc ttcatatctg ataacaacaa cggttttttc acccttcttg 3180
   atttcacctc tttccaaggc ttcaataaag gcaccttcct cttcgaacac acgtgctcta 3240
   cccttgaagt aagtaccttc cttaccggta attttaccca cagctccacc tggtgccaat 3300
   gaaccgtaca gaatttgcaa gtgaccgttg gccttgattg ggtgggagag tggcttaata 3360
   atctcttgtc cttcaggtag gcttggtgct ttctttgcac gttctgccaa agtgtcaccg 3420
   gtaacagtca ttgtgttacc gtgcaacatg ttgttttcat atagatactt aatcacagat 3480
   tgggtaccac caacgttaat caaatcggcc atgacgtatt taccagaagg tttgaagtca 3540
   ccgatcaatg gtgtagtatc actgattctt tggaaatcat ctggtgacaa cttgacaccc 3600
   gcagagtgag caacagccac caaatgcaaa acagcattag tggacccacc ggttgcaacg 3660
   acataagtaa tggcgttttc aaaagcctct tttgtgagga tatcacgagg taaaataccc 3720
   aattccattg tcttcttgat gtattcacca atgttgtcac actcagctaa cttctccttg 3780
   gaaacggctg ggaaggaaga ggagtttgga atggtcaaac ctagcacttc agcggcagaa 3840
   gccattgtgt tggcagtata cataccacca caagaaccag gacctgggca tgcatgttcc 3900
   acaacatctt ctctttcttc ttcagtgaat tgcttggaaa tatattcacc gtaggattgg 3960
   aacgcagaga cgatatcgat gtttttagag atcctgttaa aacctctagt ggagtagtag 4020
   atgtaatcaa tgaagcggaa gccaaaagac cagagtagag gcctatagaa gaaactgcga 4080
   taccttttgt gatggctaaa caaacagaca tctttttata tgtttttact tctgtatatc 4140
   gtgaagtagt aagtgataag cgaatttggc taagaacgtt gtaagtgaac aagggacctc 4200
   ttttgccttt caaaaaagga ttaaatggag ttaatcattg agatttagtt ttcgttagat 4260
   tctgtatccc taaataactc ccttacccga cgggaaggca caaaagactt gaataatagc 4320
   aaacggccag tagccaagac caaataatac tagagttaac tgatggtctt aaacaggcat 4380
   tacgtggtga actccaagac caatatacaa aatatcgata agttattctt gcccaccaat 4440
   ttaaggagcc tacatcagga cagtagtacc attcctcaga gaagaggtat acataacaag 4500
   aaaatcgcgt gaacacctta tataacttag cccgttattg agctaaaaaa ccttgcaaaa 4560
   tttcctatga ataagaatac ttcagacgtg ataaaaattt actttctaac tcttctcacg 4620
   ctgcccctat ctgttcttcc gctctaccgt gagaaataaa gcatcgagta cggcagttcg 4680
   ctgtcactga actaaaacaa taaggctagt tcgaatgatg aacttgcttg ctgtcaaact 4740
   tctgagttgc cgctgatgtg acactgtgac aataaattca aaccggttat agcggtctcc 4800
   tccggtaccg gttctgccac ctccaataga gctcagtagg agtcagaacc tctgcggtgg 4860
   ctgtcagtga ctcatccgcg tttcgtaagt tgtgcgcgtg cacatttcgc ccgttcccgc 4920
   tcatcttgca gcaggcggaa attttcatca cgctgtagga cgcaaaaaaa aaataattaa 4980
   tcgtacaaga atcttggaaa aaaaattgaa aaattttgta taaaagggat gacctaactt 5040
   gactcaatgg cttttacacc cagtattttc cctttccttg tttgttacaa ttatagaagc 5100
   aagacaaaaa catatagaca acctattcct aggagttata tttttttacc ctaccagcaa 5160
   tataagtaaa aaactagtat gaaggtgttt tacgataaag actgcgatct gagcatcatc 5220
   cagggaaaga aggttgctat tataggatat ggttcccaag gacacgcaca agccttgaac 5280
   ttgaaagatt ctggggtcga cgtgacagta ggtctgtata aaggtgctgc tgatgcagca 5340
   aaggctgaag cacatggctt taaagtcaca gatgttgcag cggctgttgc tggcgctgat 5400
   ttagtcatga ttttaattcc agatgaattt caatcgcaat tgtacaaaaa tgaaatagaa 5460
   ccaaacatta agaagggcgc taccttggcc ttcagtcatg gatttgccat tcattacaat 5520
   caagtagtcc ccagggcaga tttggacgtt attatgattg cacctaaggc tccggggcat 5580
   actgttagga gcgaatttgt taagggtggt ggtattccag atttgatcgc tatataccaa 5640
   gacgttagcg gaaacgctaa gaatgtagct ttaagctacg cagcaggagt tggtggcggg 5700
   agaacgggta taatagaaac cacttttaaa gacgagactg agacagattt atttggagaa 5760
   caagcggttc tgtgcggagg aactgttgaa ttggttaaag caggctttga gacgcttgtc 5820
   gaagcagggt acgctcccga aatggcatac ttcgaatgtc tacatgaatt gaagttgata 5880
   gtagacttaa tgtatgaagg tggtatagct aatatgaact attccatttc aaataatgca 5940
   gaatatggtg agtatgtcac cggacctgaa gtcattaacg cagaatcaag acaagccatg 6000
   agaaatgcct tgaaacgtat ccaggacggt gaatacgcta agatgttcat aagtgaaggc 6060
   gctacgggtt acccgagtat gactgctaaa agaagaaaca atgcagcaca tggtatcgaa 6120
   attattggtg aacagttaag gtctatgatg ccctggatcg gtgctaataa gatcgtagac 6180
   aaggcgaaaa attaaggccc tgcaggccta tcaagtgctg gaaacttttt ctcttggaat 6240
   ttttgcaaca tcaagtcata gtcaattgaa ttgacccaat ttcacattta agattttttt 6300
   tttttcatcc gacatacatc tgtacactag gaagccctgt ttttctgaag cagcttcaaa 6360
   tatatatatt ttttacatat ttattatgat tcaatgaaca atctaattaa atcgaaaaca 6420
   agaaccgaaa cgcgaataaa taatttattt agatggtgac aagtgtataa gtcctcatcg 6480
   ggacagctac gatttctctt tcggttttgg ctgagctact ggttgctgtg acgcagcggc 6540
   attagcgcgg cgttatgagc taccctcgtg gcctgaaaga tggcgggaat aaagcggaac 6600
   taaaaattac tgactgagcc atattgaggt caatttgtca actcgtcaag tcacgtttgg 6660
   tggacggccc ctttccaacg aatcgtatat actaacatgc gcgcgcttcc tatatacaca 6720
   tatacatata tatatatata tatatgtgtg cgtgtatgtg tacacctgta tttaatttcc 6780
   ttactcgcgg gtttttcttt tttctcaatt cttggcttcc tctttctcga gtatataatt 6840
   tttcaggtaa aatttagtac gatagtaaaa tacttctcga actcgtcaca tatacgtgta 6900
   cataatgtct gaaccagctc aaaagaaaca aaaggttgct aacaactctc tagagcggcc 6960
   gcccgcaaat taaagccttc gagcgtccca aaaccttctc aagcaaggtt ttcagtataa 7020
   tgttacatgc gtacacgcgt ctgtacagaa aaaaaagaaa aatttgaaat ataaataacg 7080
   ttcttaatac taacataact ataaaaaaat aaatagggac ctagacttca ggttgtctaa 7140
   ctccttcctt ttcggttaga gcggatgtgg ggggagggcg tgaatgtaag cgtgacataa 7200
   ctaattacat gattaattaa ttattggttt tctggtctca actttctgac ttccttacca 7260
   accttccaga tttccatgtt tctgatggtg tctaattcct tttctagctt ttctctgtag 7320
   tcaggttgag agttgaattc caaagatctc ttggtttcgg taccgttctt ggtagattcg 7380
   tacaagtctt ggaaaacagg cttcaaagca ttcttgaaga ttgggtacca gtccaaagca 7440
   cctcttctgg cggtggtgga acaagcatcg tacatgtaat ccataccgta cttaccgatc 7500
   aatgggtata gagattgggt agcttcttcg acggtttcgt tgaaagcttc agatggggag 7560
   tgaccgtttt ctctcaagac gtcgtattga gccaagaaca taccgtggat accacccatt 7620
   aaacaacctc tttcaccgta caagtcagag ttgacttctc tttcgaaagt ggtttggtaa 7680
   acgtaaccgg aaccaatggc aacggccaaa gcttgggcct tttcgtgagc cttaccggtg 7740
   acatcgttcc agacggcgta agaagagtta ataccacgac cttccttgaa caaagatctg 7800
   acagttctac cggaaccctt tggagcaacc aagataacat ctaagtcctt tggtggttca 7860
   acgtgagtca agtccttgaa gactggggag aaaccgtggg agaagtacaa agtcttaccc 7920
   ttggtcaaca atggcttgat agcaggccag gtttctgatt gagcggcatc ggacaacaag 7980
   ttcataacgt aactacctct cttgatagca tcttcaacag tgaacaagtt cttgcctgga 8040
   acccaaccgt cttcgatggc agccttccaa gaagcaccat ctttacggac accaatgata 8100
   acgttcaaac cgttgtctct caagttcaaa ccttgaccgt aaccttggga accgtaaccg 8160
   atcaaagcaa aagtgtcgtt cttgaagtag tccaacaact tttctcttgg ccagtcagct 8220
   ctttcgtaga cggtttcaac agtaccaccg aagttgattt gcttcaacat cctcagctct 8280
   agatttgaat atgtattact tggttatggt tatatatgac aaaagaaaaa gaagaacaga 8340
   agaataacgc aaggaagaac aataactgaa attgatagag aagtattatg tctttgtctt 8400
   tttataataa atcaagtgca gaaatccgtt agacaacatg agggataaaa tttaacgtgg 8460
   gcgaagaaga aggaaaaaag tttttgtgag ggcgtaattg aagcgatctg ttgattgtag 8520
   attttttttt tttgaggagt caaagtcaga agagaacaga caaatggtat taaccatcca 8580
   atactttttt ggagcaacgc taagctcatg cttttccatt ggttacgtgc tcagttgtta 8640
   gatatggaaa gagaggatgc tcacggcagc gtgactccaa ttgagcccga aagagaggat 8700
   gccacgtttt cccgacggct gctagaatgg aaaaaggaaa aatagaagaa tcccattcct 8760
   atcattattt acgtaatgac ccacacattt ttgagatttt caactattac gtattacgat 8820
   aatcctgctg tcattatcat tattatctat atcgacgtat gcaacgtatg tgaagccaag 8880
   taggcaatta tttagtactg tcagtattgt tattcatttc agatctatcc gcggtggagc 8940
   tcgaattcac tggccgtcgt tttacaacgt cgtgactggg aaaaccctgg cgttacccaa 9000
   cttaatcgcc ttgcagcaca tccccctttc gccagctggc gtaatagcga agaggcccgc 9060
   accgatcgcc cttcccaaca gttgcgcagc ctgaatggcg aatggcgcct gatgcggtat 9120
   tttctcctta cgcatctgtg cggtatttca caccgcatac gtcaaagcaa ccatagtacg 9180
   cgccctgtag cggcgcatta agcgcggcgg gtgtggtggt tacgcgcagc gtgaccgcta 9240
   cacttgccag cgccttagcg cccgctcctt tcgctttctt cccttccttt ctcgccacgt 9300
   tcgccggctt tccccgtcaa gctctaaatc gggggctccc tttagggttc cgatttagtg 9360
   ctttacggca cctcgacccc aaaaaacttg atttgggtga tggttcacgt agtgggccat 9420
   cgccctgata gacggttttt cgccctttga cgttggagtc cacgttcttt aatagtggac 9480
   tcttgttcca aactggaaca acactcaact ctatctcggg ctattctttt gatttataag 9540
   ggattttgcc gatttcggtc tattggttaa aaaatgagct gatttaacaa aaatttaacg 9600
   cgaattttaa caaaatatta acgtttacaa ttttatggtg cactctcagt acaatctgct 9660
   ctgatgccgc atagttaagc cagccccgac acccgccaac acccgctgac gcgccctgac 9720
   gggcttgtct gctcccggca tccgcttaca gacaagctgt gaccgtctcc gggagctgca 9780
   tgtgtcagag gttttcaccg tcatcaccga aacgcgcgag acgaaagggc ctcgtgatac 9840
   gcctattttt ataggttaat gtcatgataa taatggtttc ttagacgtca ggtggcactt 9900
   ttcggggaaa tgtgcgcgga acccctattt gtttattttt ctaaatacat tcaaatatgt 9960
   atccgctcat gagacaataa ccctgataaa tgcttcaata atattgaaaa aggaagagta 10020
   tgagtattca acatttccgt gtcgccctta ttcccttttt tgcggcattt tgccttcctg 10080
   tttttgctca cccagaaacg ctggtgaaag taaaagatgc tgaagatcag ttgggtgcac 10140
   gagtgggtta catcgaactg gatctcaaca gcggtaagat ccttgagagt tttcgccccg 10200
   aagaacgttt tccaatgatg agcactttta aagttctgct atgtggcgcg gtattatccc 10260
   gtattgacgc cgggcaagag caactcggtc gccgcataca ctattctcag aatgacttgg 10320
   ttgagtactc accagtcaca gaaaagcatc ttacggatgg catgacagta agagaattat 10380
   gcagtgctgc cataaccatg agtgataaca ctgcggccaa cttacttctg acaacgatcg 10440
   gaggaccgaa ggagctaacc gcttttttgc acaacatggg ggatcatgta actcgccttg 10500
   atcgttggga accggagctg aatgaagcca taccaaacga cgagcgtgac accacgatgc 10560
   ctgtagcaat ggcaacaacg ttgcgcaaac tattaactgg cgaactactt actctagctt 10620
   cccggcaaca attaatagac tggatggagg cggataaagt tgcaggacca cttctgcgct 10680
   cggcccttcc ggctggctgg tttattgctg ataaatctgg agccggtgag cgtgggtctc 10740
   gcggtatcat tgcagcactg gggccagatg gtaagccctc ccgtatcgta gttatctaca 10800
   cgacggggag tcaggcaact atggatgaac gaaatagaca gatcgctgag ataggtgcct 10860
   cactgattaa gcattggtaa ctgtcagacc aagtttactc atatatactt tagattgatt 10920
   taaaacttca tttttaattt aaaaggatct aggtgaagat cctttttgat aatctcatga 10980
   ccaaaatccc ttaacgtgag ttttcgttcc actgagcgtc agaccccgta gaaaagatca 11040
   aaggatcttc ttgagatcct ttttttctgc gcgtaatctg ctgcttgcaa acaaaaaaac 11100
   caccgctacc agcggtggtt tgtttgccgg atcaagagct accaactctt tttccgaagg 11160
   taactggctt cagcagagcg cagataccaa atactgttct tctagtgtag ccgtagttag 11220
   gccaccactt caagaactct gtagcaccgc ctacatacct cgctctgcta atcctgttac 11280
   cagtggctgc tgccagtggc gataagtcgt gtcttaccgg gttggactca agacgatagt 11340
   taccggataa ggcgcagcgg tcgggctgaa cggggggttc gtgcacacag cccagcttgg 11400
   agcgaacgac ctacaccgaa ctgagatacc tacagcgtga gctatgagaa agcgccacgc 11460
   ttcccgaagg gagaaaggcg gacaggtatc cggtaagcgg cagggtcgga acaggagagc 11520
   gcacgaggga gcttccaggg ggaaacgcct ggtatcttta tagtcctgtc gggtttcgcc 11580
   acctctgact tgagcgtcga tttttgtgat gctcgtcagg ggggcggagc ctatggaaaa 11640
   acgccagcaa cgcggccttt ttacggttcc tggccttttg ctggcctttt gctcacatgt 11700
   tctttcctgc gttatcccct gattctgtgg ataaccgtat taccgccttt gagtgagctg 11760
   ataccgctcg ccgcagccga acgaccgagc gcagcgagtc agtgagcgag gaagcggaag 11820
   agcgcccaat acgcaaaccg cctctccccg cgcgttggcc gattcattaa tgcagctggc 11880
   acgacaggtt tcccgactgg aaagcgggca gtgagcgcaa cgcaattaat gtgagttagc 11940
   tcactcatta ggcaccccag gctttacact ttatgcttcc ggctcgtatg ttgtgtggaa 12000
   ttgtgagcgg ataacaattt cacacaggaa acagctatga ccatgattac gccaagcttt 12060
   ttctttccaa tttttttttt ttcgtcatta taaaaatcat tacgaccgag attcccgggt 12120
   aataactgat ataattaaat tgaagctcta atttgtgagt ttagtataca tgcatttact 12180
   tataatacag ttttttagtt ttgctggccg catcttctca aatatgcttc ccagcctgct 12240
   tttctgtaac gttcaccctc taccttagca tcccttccct ttgcaaatag tcctcttcca 12300
   acaataataa tgtcagatcc tgtagagacc acatcatcca cggttctata ctgttgaccc 12360
   aatgcgtctc ccttgtcatc taaacccaca ccgggtgtca taatcaacca atcgtaacct 12420
   tcatctcttc cacccatgtc tctttgagca ataaagccga taacaaaatc tttgtcgctc 12480
   ttcgcaatgt caacagtacc cttagtatat tctccagtag atagggagcc cttgcatgac 12540
   aattctgcta acatcaaaag gcctctaggt tcctttgtta cttcttctgc cgcctgcttc 12600
   aaaccgctaa caatacctgg gcccaccaca ccgtgtgcat tcgtaatgtc tgcccattct 12660
   gctattctgt atacacccgc agagtactgc aatttgactg tattaccaat gtcagcaaat 12720
   tttctgtctt cgaagagtaa aaaattgtac ttggcggata atgcctttag cggcttaact 12780
   gtgccctcca tggaaaaatc agtcaagata tccacatgtg tttttagtaa acaaattttg 12840
   ggacctaatg cttcaactaa ctccagtaat tccttggtgg tacgaacatc caatgaagca 12900
   cacaagtttg tttgcttttc gtgcatgata ttaaatagct tggcagcaac aggactagga 12960
   tgagtagcag cacgttcctt atatgtagct ttcgacatga tttatcttcg tttcctgcag 13020
   gtttttgttc tgtgcagttg ggttaagaat actgggcaat ttcatgtttc ttcaacacta 13080
   catatgcgta tatataccaa tctaagtctg tgctccttcc ttcgttcttc cttctgttcg 13140
   gagattaccg aatcaaaaaa atttcaagga aaccgaaatc aaaaaaaaga ataaaaaaaa 13200
   aatgatgaat tgaaaagctt gcatgcctgc aggtcgactc tagtatactc cgtctactgt 13260
   acgatacact tccgctcagg tccttgtcct ttaacgaggc cttaccactc ttttgttact 13320
   ctattgatcc agctcagcaa aggcagtgtg atctaagatt ctatcttcgc gatgtagtaa 13380
   aactagctag accgagaaag agactagaaa tgcaaaaggc acttctacaa tggctgccat 13440
   cattattatc cgatgtgacg ctgcattttt tttttttttt tttttttttt tttttttttt 13500
   tttttttttt tttttttgta caaatatcat aaaaaaagag aatcttttta agcaaggatt 13560
   ttcttaactt cttcggcgac agcatcaccg acttcggtgg tactgttgga accacctaaa 13620
   tcaccagttc tgatacctgc atccaaaacc tttttaactg catcttcaat ggctttacct 13680
   tcttcaggca agttcaatga caatttcaac atcattgcag cagacaagat agtggcgata 13740
   gggttgacct tattctttgg caaatctgga gcggaaccat ggcatggttc gtacaaacca 13800
   aatgcggtgt tcttgtctgg caaagaggcc aaggacgcag atggcaacaa acccaaggag 13860
   cctgggataa cggaggcttc atcggagatg atatcaccaa acatgttgct ggtgattata 13920
   ataccattta ggtgggttgg gttcttaact aggatcatgg cggcagaatc aatcaattga 13980
   tgttgaactt tcaatgtagg gaattcgttc ttgatggttt cctccacagt ttttctccat 14040
   aatcttgaag aggccaaaac attagcttta tccaaggacc aaataggcaa tggtggctca 14100
   tgttgtaggg ccatgaaagc ggccattctt gtgattcttt gcacttctgg aacggtgtat 14160
   tgttcactat cccaagcgac accatcacca tcgtcttcct ttctcttacc aaagtaaata 14220
   cctcccacta attctctaac aacaacgaag tcagtacctt tagcaaattg tggcttgatt 14280
   ggagataagt ctaaaagaga gtcggatgca aagttacatg gtcttaagtt ggcgtacaat 14340
   tgaagttctt tacggatttt tagtaaacct tgttcaggtc taacactacc ggtaccccat 14400
   ttaggaccac ccacagcacc taacaaaacg gcatcagcct tcttggaggc ttccagcgcc 14460
   tcatctggaa gtggaacacc tgtagcatcg atagcagcac caccaattaa atgattttcg 14520
   aaatcgaact tgacattgga acgaacatca gaaatagctt taagaacctt aatggcttcg 14580
   gctgtgattt cttgaccaac gtggtcacct ggcaaaacga cgatcttctt aggggcagac 14640
   attacaatgg tatatccttg aaatatatat aaaaaaaaaa aaaaaaaaaa aaaaaaaaaa 14700
   tgcagcttct caatgatatt cgaatacgct ttgaggagat acagcctaat atccgacaaa 14760
   ctgttttaca gatttacgat cgtacttgtt acccatcatt gaattttgaa catccgaacc 14820
   tgggagtttt ccctgaaaca gatagtatat ttgaacctgt ataataatat atagtctagc 14880
   gctttacgga agacaatgta tgtatttcgg ttcctggaga aactattgca tctattgcat 14940
   aggtaatctt gcacgtcgca tccccggttc attttctgcg tttccatctt gcacttcaat 15000
   agcatatctt tgttaacgaa gcatctgtgc ttcattttgt agaacaaaaa tgcaacgcga 15060
   gagcgctaat tttcaaacaa aagaatctga gctgcatttt tacagaacag aaatgcaacg 15120
   cgaaagcgct attttaccaa cgaagaatct gtgcttcatt tttgtaaaac aaaaatgcaa 15180
   cgcgagagcg ctaatttttc aaacaaagaa tctgagctgc atttttacag aacagaaatg 15240
   caacgcgaga gcgctatttt accaacaaag aatctatact tcttttttgt tctacaaaaa 15300
   tgcatcccga gagcgctatt tttctaacaa agcatcttag attacttttt ttctcctttg 15360
   tgcgctctat aatgcagtct cttgataact ttttgcactg taggtccgtt aaggttagaa 15420
   gaaggctact ttggtgtcta ttttctcttc cataaaaaaa gcctgactcc acttcccgcg 15480
   tttactgatt actagcgaag ctgcgggtgc attttttcaa gataaaggca tccccgatta 15540
   tattctatac cgatgtggat tgcgcatact ttgtgaacag aaagtgatag cgttgatgat 15600
   tcttcattgg tcagaaaatt atgaacggtt tcttctattt tgtctctata tactacgtat 15660
   aggaaatgtt tacattttcg tattgttttc gattcactct atgaatagtt cttactacaa 15720
   tttttttgtc taaagagtaa tactagagat aaacataaaa aatgtagagg tcgagtttag 15780
   atgcaagttc aaggagcgaa aggtggatgg gtaggttata tagggatata gcacagagat 15840
   atatagcaaa gagatacttt tgagcaatgt ttgtggaagc ggtattcgca atattttagt 15900
   agctcgttac agtccggtgc gtttttggtt ttttgaaagt gcgtcttcag agcgcttttg 15960
   gttttcaaaa gcgctctgaa gttcctatac tttctagaga ataggaactt cggaatagga 16020
   acttcaaagc gtttccgaaa acgagcgctt ccgaaaatgc aacgcgagct gcgcacatac 16080
   agctcactgt tcacgtcgca cctatatctg cgtgttgcct gtatatatat atacatgaga 16140
   agaacggcat agtgcgtgtt tatgcttaaa tgcgtactta tatgcgtcta tttatgtagg 16200
   atgaaaggta gtctagtacc tcctgtgata ttatcccatt ccatgcgggg tatcgtatgc 16260
   ttccttcagc actacccttt agctgttcta tatgctgcca ctcctcaatt ggattagtct 16320
   catccttcaa tgctatcatt tcctttgata ttggatcata tgcatagtac cgagaaacta 16380
   gaggatc 16387
<210> 31
   <211> 448
   <212> DNA
   <213> Saccharomyces cerevisiae
<400> 31
   cccattaccg acatttgggc gctatacgtg catatgttca tgtatgtatc tgtatttaaa 60
   acacttttgt attatttttc ctcatatatg tgtataggtt tatacggatg atttaattat 120
   tacttcacca ccctttattt caggctgata tcttagcctt gttactagtt agaaaaagac 180
   atttttgctg tcagtcactg tcaagagatt cttttgctgg catttcttct agaagcaaaa 240
   agagcgatgc gtcttttccg ctgaaccgtt ccagcaaaaa agactaccaa cgcaatatgg 300
   attgtcagaa tcatataaaa gagaagcaaa taactccttg tcttgtatca attgcattat 360
   aatatcttct tgttagtgca atatcatata gaagtcatcg aaatagatat taagaaaaac 420
   aaactgtaca atcaatcaat caatcatc 448
<210> 32
   <211> 1716
   <212> DNA
   <213> Bacillus subtilis
<400> 32
   atgttgacaa aagcaacaaa agaacaaaaa tcccttgtga aaaacagagg ggcggagctt      60
   gttgttgatt gcttagtgga gcaaggtgtc acacatgtat ttggcattcc aggtgcaaaa     120
   attgatgcgg tatttgacgc tttacaagat aaaggacctg aaattatcgt tgcccggcac     180
   gaacaaaacg cagcattcat ggcccaagca gtcggccgtt taactggaaa accgggagtc     240
   gtgttagtca catcaggacc gggtgcctct aacttggcaa caggcctgct gacagcgaac     300
   actgaaggag accctgtcgt tgcgcttgct ggaaacgtga tccgtgcaga tcgtttaaaa     360
   cggacacatc aatctttgga taatgcggcg ctattccagc cgattacaaa atacagtgta     420
   gaagttcaag atgtaaaaaa tataccggaa gctgttacaa atgcatttag gatagcgtca     480
   gcagggcagg ctggggccgc ttttgtgagc tttccgcaag atgttgtgaa tgaagtcaca     540
   aatacgaaaa acgtgcgtgc tgttgcagcg ccaaaactcg gtcctgcagc agatgatgca     600
   atcagtgcgg ccatagcaaa aatccaaaca gcaaaacttc ctgtcgtttt ggtcggcatg     660
   aaaggcggaa gaccggaagc aattaaagcg gttcgcaagc ttttgaaaaa ggttcagctt     720
   ccatttgttg aaacatatca agctgccggt accctttcta gagatttaga ggatcaatat     780
   tttggccgta tcggtttgtt ccgcaaccag cctggcgatt tactgctaga gcaggcagat     840
   gttgttctga cgatcggcta tgacccgatt gaatatgatc cgaaattctg gaatatcaat     900
   ggagaccgga caattatcca tttagacgag attatcgctg acattgatca tgcttaccag     960
   cctgatcttg aattgatcgg tgacattccg tccacgatca atcatatcga acacgatgct    1020
   gtgaaagtgg aatttgcaga gcgtgagcag aaaatccttt ctgatttaaa acaatatatg    1080
   catgaaggtg agcaggtgcc tgcagattgg aaatcagaca gagcgcaccc tcttgaaatc    1140
   gttaaagagt tgcgtaatgc agtcgatgat catgttacag taacttgcga tatcggttcg    1200
   cacgccattt ggatgtcacg ttatttccgc agctacgagc cgttaacatt aatgatcagt    1260
   aacggtatgc aaacactcgg cgttgcgctt ccttgggcaa tcggcgcttc attggtgaaa    1320
   ccgggagaaa aagtggtttc tgtctctggt gacggcggtt tcttattctc agcaatggaa    1380
   ttagagacag cagttcgact aaaagcacca attgtacaca ttgtatggaa cgacagcaca    1440
   tatgacatgg ttgcattcca gcaattgaaa aaatataacc gtacatctgc ggtcgatttc    1500
   ggaaatatcg atatcgtgaa atatgcggaa agcttcggag caactggctt gcgcgtagaa    1560
   tcaccagacc agctggcaga tgttctgcgt caaggcatga acgctgaagg tcctgtcatc    1620
   atcgatgtcc cggttgacta cagtgataac attaatttag caagtgacaa gcttccgaaa    1680
   gaattcgggg aactcatgaa aacgaaagct ctctag                              1716
<210> 33
   <211> 571
   <212> PRT
   <213> Bacillus subtilis
<400> 33
   Met Leu Thr Lys Ala Thr Lys Glu Gln Lys Ser Leu Val Lys Asn Arg
   1               5                   10                  15
   Gly Ala Glu Leu Val Val Asp Cys Leu Val Glu Gln Gly Val Thr His
               20                  25                  30
   Val Phe Gly Ile Pro Gly Ala Lys Ile Asp Ala Val Phe Asp Ala Leu
           35                  40                  45
   Gln Asp Lys Gly Pro Glu Ile Ile Val Ala Arg His Glu Gln Asn Ala
       50                  55                  60
   Ala Phe Met Ala Gln Ala Val Gly Arg Leu Thr Gly Lys Pro Gly Val
   65                  70                  75                  80
   Val Leu Val Thr Ser Gly Pro Gly Ala Ser Asn Leu Ala Thr Gly Leu
                   85                  90                  95
   Leu Thr Ala Asn Thr Glu Gly Asp Pro Val Val Ala Leu Ala Gly Asn
               100                 105                 110
   Val Ile Arg Ala Asp Arg Leu Lys Arg Thr His Gln Ser Leu Asp Asn
           115                 120                 125
   Ala Ala Leu Phe Gln Pro Ile Thr Lys Tyr Ser Val Glu Val Gln Asp
       130                 135                 140
   Val Lys Asn Ile Pro Glu Ala Val Thr Asn Ala Phe Arg Ile Ala Ser
   145                 150                 155                 160
   Ala Gly Gln Ala Gly Ala Ala Phe Val Ser Phe Pro Gln Asp Val Val
                   165                 170                 175
   Asn Glu Val Thr Asn Thr Lys Asn Val Arg Ala Val Ala Ala Pro Lys
               180                 185                 190
   Leu Gly Pro Ala Ala Asp Asp Ala Ile Ser Ala Ala Ile Ala Lys Ile
           195                 200                 205
   Gln Thr Ala Lys Leu Pro Val Val Leu Val Gly Met Lys Gly Gly Arg
       210                 215                 220
   Pro Glu Ala Ile Lys Ala Val Arg Lys Leu Leu Lys Lys Val Gln Leu
   225                 230                 235                 240
   Pro Phe Val Glu Thr Tyr Gln Ala Ala Gly Thr Leu Ser Arg Asp Leu
                   245                 250                 255
   Glu Asp Gln Tyr Phe Gly Arg Ile Gly Leu Phe Arg Asn Gln Pro Gly
               260                 265                 270
   Asp Leu Leu Leu Glu Gln Ala Asp Val Val Leu Thr Ile Gly Tyr Asp
           275                 280                 285
   Pro Ile Glu Tyr Asp Pro Lys Phe Trp Asn Ile Asn Gly Asp Arg Thr
       290                 295                 300
   Ile Ile His Leu Asp Glu Ile Ile Ala Asp Ile Asp His Ala Tyr Gln
   305                 310                 315                 320
   Pro Asp Leu Glu Leu Ile Gly Asp Ile Pro Ser Thr Ile Asn His Ile
                   325                 330                 335
   Glu His Asp Ala Val Lys Val Glu Phe Ala Glu Arg Glu Gln Lys Ile
               340                 345                 350
   Leu Ser Asp Leu Lys Gln Tyr Met His Glu Gly Glu Gln Val Pro Ala
           355                 360                 365
   Asp Trp Lys Ser Asp Arg Ala His Pro Leu Glu Ile Val Lys Glu Leu
       370                 375                 380
   Arg Asn Ala Val Asp Asp His Val Thr Val Thr Cys Asp Ile Gly Ser
   385                 390                 395                 400
   His Ala Ile Trp Met Ser Arg Tyr Phe Arg Ser Tyr Glu Pro Leu Thr
                   405                 410                 415
   Leu Met Ile Ser Asn Gly Met Gln Thr Leu Gly Val Ala Leu Pro Trp
               420                 425                 430
   Ala Ile Gly Ala Ser Leu Val Lys Pro Gly Glu Lys Val Val Ser Val
           435                 440                 445
   Ser Gly Asp Gly Gly Phe Leu Phe Ser Ala Met Glu Leu Glu Thr Ala
       450                 455                 460
   Val Arg Leu Lys Ala Pro Ile Val His Ile Val Trp Asn Asp Ser Thr
   465                 470                 475                 480
   Tyr Asp Met Val Ala Phe Gln Gln Leu Lys Lys Tyr Asn Arg Thr Ser
                   485                 490                 495
   Ala Val Asp Phe Gly Asn Ile Asp Ile Val Lys Tyr Ala Glu Ser Phe
               500                 505                 510
   Gly Ala Thr Gly Leu Arg Val Glu Ser Pro Asp Gln Leu Ala Asp Val
           515                 520                 525
   Leu Arg Gln Gly Met Asn Ala Glu Gly Pro Val Ile Ile Asp Val Pro
       530                 535                 540
   Val Asp Tyr Ser Asp Asn Ile Asn Leu Ala Ser Asp Lys Leu Pro Lys
   545                 550                 555                 560
   Glu Phe Gly Glu Leu Met Lys Thr Lys Ala Leu
                   565                 570
<210> 34
   <211> 250
   <212> DNA
   <213> Saccharymyces cerevisiae
<400> 34
   ccgcaaatta aagccttcga gcgtcccaaa accttctcaa gcaaggtttt cagtataatg 60
   ttacatgcgt acacgcgtct gtacagaaaa aaaagaaaaa tttgaaatat aaataacgtt 120
   cttaatacta acataactat aaaaaaataa atagggacct agacttcagg ttgtctaact 180
   ccttcctttt cggttagagc ggatgtgggg ggagggcgtg aatgtaagcg tgacataact 240
   aattacatga 250
<210> 35
   <211> 1181
   <212> DNA
   <213> Saccharomyces cerevisiae
<400> 35
   taaaacctct agtggagtag tagatgtaat caatgaagcg gaagccaaaa gaccagagta 60
   gaggcctata gaagaaactg cgataccttt tgtgatggct aaacaaacag acatcttttt 120
   atatgttttt acttctgtat atcgtgaagt agtaagtgat aagcgaattt ggctaagaac 180
   gttgtaagtg aacaagggac ctcttttgcc tttcaaaaaa ggattaaatg gagttaatca 240
   ttgagattta gttttcgtta gattctgtat ccctaaataa ctcccttacc cgacgggaag 300
   gcacaaaaga cttgaataat agcaaacggc cagtagccaa gaccaaataa tactagagtt 360
   aactgatggt cttaaacagg cattacgtgg tgaactccaa gaccaatata caaaatatcg 420
   ataagttatt cttgcccacc aatttaagga gcctacatca ggacagtagt accattcctc 480
   agagaagagg tatacataac aagaaaatcg cgtgaacacc ttatataact tagcccgtta 540
   ttgagctaaa aaaccttgca aaatttccta tgaataagaa tacttcagac gtgataaaaa 600
   tttactttct aactcttctc acgctgcccc tatctgttct tccgctctac cgtgagaaat 660
   aaagcatcga gtacggcagt tcgctgtcac tgaactaaaa caataaggct agttcgaatg 720
   atgaacttgc ttgctgtcaa acttctgagt tgccgctgat gtgacactgt gacaataaat 780
   tcaaaccggt tatagcggtc tcctccggta ccggttctgc cacctccaat agagctcagt 840
   aggagtcaga acctctgcgg tggctgtcag tgactcatcc gcgtttcgta agttgtgcgc 900
   gtgcacattt cgcccgttcc cgctcatctt gcagcaggcg gaaattttca tcacgctgta 960
   ggacgcaaaa aaaaaataat taatcgtaca agaatcttgg aaaaaaaatt gaaaaatttt 1020
   gtataaaagg gatgacctaa cttgactcaa tggcttttac acccagtatt ttccctttcc 1080
   ttgtttgtta caattataga agcaagacaa aaacatatag acaacctatt cctaggagtt 1140
   atattttttt accctaccag caatataagt aaaaaactag t 1181
<210> 36
   <211> 1014
   <212> DNA
   <213> artificial sequence
<220>
   <223> mutant of ilvC from Pseudomonas fluorescens
<400> 36
   atgaaggtgt tttacgataa agactgcgat ctgagcatca tccagggaaa gaaggttgct      60
   attataggat atggttccca aggacacgca caagccttga acttgaaaga ttctggggtc     120
   gacgtgacag taggtctgta taaaggtgct gctgatgcag caaaggctga agcacatggc     180
   tttaaagtca cagatgttgc agcggctgtt gctggcgctg atttagtcat gattttaatt     240
   ccagatgaat ttcaatcgca attgtacaaa aatgaaatag aaccaaacat taagaagggc     300
   gctaccttgg ccttcagtca tggatttgcc attcattaca atcaagtagt ccccagggca     360
   gatttggacg ttattatgat tgcacctaag gctccggggc atactgttag gagcgaattt     420
   gttaagggtg gtggtattcc agatttgatc gctatatacc aagacgttag cggaaacgct     480
   aagaatgtag ctttaagcta cgcagcagga gttggtggcg ggagaacggg tataatagaa     540
   accactttta aagacgagac tgagacagat ttatttggag aacaagcggt tctgtgcgga     600
   ggaactgttg aattggttaa agcaggcttt gagacgcttg tcgaagcagg gtacgctccc     660
   gaaatggcat acttcgaatg tctacatgaa ttgaagttga tagtagactt aatgtatgaa     720
   ggtggtatag ctaatatgaa ctattccatt tcaaataatg cagaatatgg tgagtatgtc     780
   accggacctg aagtcattaa cgcagaatca agacaagcca tgagaaatgc cttgaaacgt     840
   atccaggacg gtgaatacgc taagatgttc ataagtgaag gcgctacggg ttacccgagt     900
   atgactgcta aaagaagaaa caatgcagca catggtatcg aaattattgg tgaacagtta     960
   aggtctatga tgccctggat cggtgctaat aagatcgtag acaaggcgaa aaat          1014
<210> 37
   <211> 338
   <212> PRT
   <213> Artificial sequence
<220>
   <223> synthetic construct mutant ilcv
<400> 37
   Met Lys Val Phe Tyr Asp Lys Asp Cys Asp Leu Ser Ile Ile Gln Gly
   1               5                   10                  15
   Lys Lys Val Ala Ile Ile Gly Tyr Gly Ser Gln Gly His Ala Gln Ala
               20                  25                  30
   Leu Asn Leu Lys Asp Ser Gly Val Asp Val Thr Val Gly Leu Tyr Lys
           35                  40                  45
   Gly Ala Ala Asp Ala Ala Lys Ala Glu Ala His Gly Phe Lys Val Thr
       50                  55                  60
   Asp Val Ala Ala Ala Val Ala Gly Ala Asp Leu Val Met Ile Leu Ile
   65                  70                  75                  80
   Pro Asp Glu Phe Gln Ser Gln Leu Tyr Lys Asn Glu Ile Glu Pro Asn
                   85                  90                  95
   Ile Lys Lys Gly Ala Thr Leu Ala Phe Ser His Gly Phe Ala Ile His
               100                 105                 110
   Tyr Asn Gln Val Val Pro Arg Ala Asp Leu Asp Val Ile Met Ile Ala
           115                 120                 125
   Pro Lys Ala Pro Gly His Thr Val Arg Ser Glu Phe Val Lys Gly Gly
       130                 135                 140
   Gly Ile Pro Asp Leu Ile Ala Ile Tyr Gln Asp Val Ser Gly Asn Ala
   145                 150                 155                 160
   Lys Asn Val Ala Leu Ser Tyr Ala Ala Ala Val Gly Gly Gly Arg Thr
                   165                 170                 175
   Gly Ile Ile Glu Thr Thr Phe Lys Asp Glu Thr Glu Thr Asp Leu Phe
               180                 185                 190
   Gly Glu Gln Ala Val Leu Cys Gly Gly Thr Val Glu Leu Val Lys Ala
           195                 200                 205
   Gly Phe Glu Thr Leu Val Glu Ala Gly Tyr Ala Pro Glu Met Ala Tyr
       210                 215                 220
   Phe Glu Cys Leu His Glu Leu Lys Leu Ile Val Asp Leu Met Tyr Glu
   225                 230                 235                 240
   Gly Gly Ile Ala Asn Met Asn Tyr Ser Ile Ser Asn Asn Ala Glu Tyr
                   245                 250                 255
   Gly Glu Tyr Val Thr Gly Pro Glu Val Ile Asn Ala Glu Ser Arg Gln
               260                 265                 270
   Ala Met Arg Asn Ala Leu Lys Arg Ile Gln Asp Gly Glu Tyr Ala Lys
           275                 280                 285
   Met Phe Ile Ser Glu Gly Ala Thr Gly Tyr Pro Ser Met Thr Ala Lys
       290                 295                 300
   Arg Arg Asn Asn Ala Ala His Gly Ile Glu Ile Ile Gly Glu Gln Leu
   305                 310                 315                 320
   Arg Ser Met Met Pro Trp Ile Gly Ala Asn Lys Ile Val Asp Lys Ala
                   325                 330                 335
   Lys Asn
<210> 38
   <211> 759
   <212> DNA
   <213> saccharomyces cerevisiae
<400> 38
   ggccctgcag gcctatcaag tgctggaaac tttttctctt ggaatttttg caacatcaag      60
   tcatagtcaa ttgaattgac ccaatttcac atttaagatt tttttttttt catccgacat     120
   acatctgtac actaggaagc cctgtttttc tgaagcagct tcaaatatat atatttttta 180
   catatttatt atgattcaat gaacaatcta attaaatcga aaacaagaac cgaaacgcga 240
   ataaataatt tatttagatg gtgacaagtg tataagtcct catcgggaca gctacgattt 300
   ctctttcggt tttggctgag ctactggttg ctgtgacgca gcggcattag cgcggcgtta 360
   tgagctaccc tcgtggcctg aaagatggcg ggaataaagc ggaactaaaa attactgact 420
   gagccatatt gaggtcaatt tgtcaactcg tcaagtcacg tttggtggac ggcccctttc 480
   caacgaatcg tatatactaa catgcgcgcg cttcctatat acacatatac atatatatat 540
   atatatatat gtgtgcgtgt atgtgtacac ctgtatttaa tttccttact cgcgggtttt 600
   tcttttttct caattcttgg cttcctcttt ctcgagtata taatttttca ggtaaaattt 660
   agtacgatag taaaatactt ctcgaactcg tcacatatac gtgtacataa tgtctgaacc 720
   agctcaaaag aaacaaaagg ttgctaacaa ctctctaga 759
<210> 39
   <211> 643
   <212> DNA
   <213> Saccharomyces cerevisiae
<400> 39
   gaaatgaata acaatactga cagtactaaa taattgccta cttggcttca catacgttgc 60
   atacgtcgat atagataata atgataatga cagcaggatt atcgtaatac gtaatagttg 120
   aaaatctcaa aaatgtgtgg gtcattacgt aaataatgat aggaatggga ttcttctatt 180
   tttccttttt ccattctagc agccgtcggg aaaacgtggc atcctctctt tcgggctcaa 240
   ttggagtcac gctgccgtga gcatcctctc tttccatatc taacaactga gcacgtaacc 300
   aatggaaaag catgagctta gcgttgctcc aaaaaagtat tggatggtta ataccatttg 360
   tctgttctct tctgactttg actcctcaaa aaaaaaaaat ctacaatcaa cagatcgctt 420
   caattacgcc ctcacaaaaa cttttttcct tcttcttcgc ccacgttaaa ttttatccct 480
   catgttgtct aacggatttc tgcacttgat ttattataaa aagacaaaga cataatactt 540
   ctctatcaat ttcagttatt gttcttcctt gcgttattct tctgttcttc tttttctttt 600
   gtcatatata accataacca agtaatacat attcaaatct aga 643
<210> 40
   <211> 1188
   <212> DNA
   <213> Saccharomyces cerevisiae
<400> 40
   atgttgagaa ctcaagccgc cagattgatc tgcaactccc gtgtcatcac tgctaagaga     60
   acctttgctt tggccacccg tgctgctgct tacagcagac cagctgcccg tttcgttaag    120
   ccaatgatca ctacccgtgg tttgaagcaa atcaacttcg gtggtactgt tgaaaccgtc    180
   tacgaaagag ctgactggcc aagagaaaag ttgttggact acttcaagaa cgacactttt    240
   gctttgatcg gttacggttc ccaaggttac ggtcaaggtt tgaacttgag agacaacggt    300
   ttgaacgtta tcattggtgt ccgtaaagat ggtgcttctt ggaaggctgc catcgaagac    360
   ggttgggttc caggcaagaa cttgttcact gttgaagatg ctatcaagag aggtagttac    420
   gttatgaact tgttgtccga tgccgctcaa tcagaaacct ggcctgctat caagccattg    480
   ttgaccaagg gtaagacttt gtacttctcc cacggtttct ccccagtctt caaggacttg    540
   actcacgttg aaccaccaaa ggacttagat gttatcttgg ttgctccaaa gggttccggt    600
   agaactgtca gatctttgtt caaggaaggt cgtggtatta actcttctta cgccgtctgg    660
   aacgatgtca ccggtaaggc tcacgaaaag gcccaagctt tggccgttgc cattggttcc    720
   ggttacgttt accaaaccac tttcgaaaga gaagtcaact ctgacttgta cggtgaaaga    780
   ggttgtttaa tgggtggtat ccacggtatg ttcttggctc aatacgacgt cttgagagaa    840
   aacggtcact ccccatctga agctttcaac gaaaccgtcg aagaagctac ccaatctcta    900
   tacccattga tcggtaagta cggtatggat tacatgtacg atgcttgttc caccaccgcc    960
   agaagaggtg ctttggactg gtacccaatc ttcaagaatg ctttgaagcc tgttttccaa   1020
   gacttgtacg aatctaccaa gaacggtacc gaaaccaaga gatctttgga attcaactct   1080
   caacctgact acagagaaaa gctagaaaag gaattagaca ccatcagaaa catggaaatc   1140
   tggaaggttg gtaaggaagt cagaaagttg agaccagaaa accaataa                1188
<210> 41
   <211> 395
   <212> PRT
   <213> Saccharomyces cerevisiae
<400> 41
   Met Leu Arg Thr Gln Ala Ala Arg Leu Ile Cys Asn Ser Arg Val Ile
   1               5                   10                  15
   Thr Ala Lys Arg Thr Phe Ala Leu Ala Thr Arg Ala Ala Ala Tyr Ser
               20                  25                  30
   Arg Pro Ala Ala Arg Phe Val Lys Pro Met Ile Thr Thr Arg Gly Leu
           35                  40                  45
   Lys Gln Ile Asn Phe Gly Gly Thr Val Glu Thr Val Tyr Glu Arg Ala
       50                  55                  60
   Asp Trp Pro Arg Glu Lys Leu Leu Asp Tyr Phe Lys Asn Asp Thr Phe
   65                  70                  75                  80
   Ala Leu Ile Gly Tyr Gly Ser Gln Gly Tyr Gly Gln Gly Leu Asn Leu
                   85                  90                  95
   Arg Asp Asn Gly Leu Asn Val Ile Ile Gly Val Arg Lys Asp Gly Ala
               100                 105                 110
   Ser Trp Lys Ala Ala Ile Glu Asp Gly Trp Val Pro Gly Lys Asn Leu
   115 120 125
   Phe Thr Val Glu Asp Ala Ile Lys Arg Gly Ser Tyr Val Met Asn Leu
   130 135 140
   Leu Ser Asp Ala Ala Gln Ser Glu Thr Trp Pro Ala Ile Lys Pro Leu
   145 150 155 160
   Leu Thr Lys Gly Lys Thr Leu Tyr Phe Ser His Gly Phe Ser Pro Val
   165 170 175
   Phe Lys Asp Leu Thr His Val Glu Pro Pro Lys Asp Leu Asp Val Ile
   180 185 190
   Leu Val Ala Pro Lys Gly Ser Gly Arg Thr Val Arg Ser Leu Phe Lys
   195 200 205
   Glu Gly Arg Gly Ile Asn Ser Ser Tyr Ala Val Trp Asn Asp Val Thr
   210 215 220
   Gly Lys Ala His Glu Lys Ala Gln Ala Leu Ala Val Ala Ile Gly Ser
   225 230 235 240
   Gly Tyr Val Tyr Gln Thr Thr Phe Glu Arg Glu Val Asn Ser Asp Leu
   245 250 255
   Tyr Gly Glu Arg Gly Cys Leu Met Gly Gly Ile His Gly Met Phe Leu
   260 265 270
   Ala Gln Tyr Asp Val Leu Arg Glu Asn Gly His Ser Pro Ser Glu Ala
   275 280 285
   Phe Asn Glu Thr Val Glu Glu Ala Thr Gln Ser Leu Tyr Pro Leu Ile
   290 295 300
   Gly Lys Tyr Gly Met Asp Tyr Met Tyr Asp Ala Cys Ser Thr Thr Ala
   305 310 315 320
   Arg Arg Gly Ala Leu Asp Trp Tyr Pro Ile Phe Lys Asn Ala Leu Lys
   325 330 335
   Pro Val Phe Gln Asp Leu Tyr Glu Ser Thr Lys Asn Gly Thr Glu Thr
   340 345 350
   Lys Arg Ser Leu Glu Phe Asn Ser Gln Pro Asp Tyr Arg Glu Lys Leu
   355 360 365
   Glu Lys Glu Leu Asp Thr Ile Arg Asn Met Glu Ile Trp Lys Val Gly
   370 375 380
   Lys Glu Val Arg Lys Leu Arg Pro Glu Asn Gln
   385 390 395
<210> 42
   <211> 15539
   <212> DNA
   <213> artificial sequence
<220>
   <223> constructed plasmid
<400> 42
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accataaatt cccgttttaa gagcttggtg agcgctagga gtcactgcca ggtatcgttt     240
   gaacacggca ttagtcaggg aagtcataac acagtccttt cccgcaattt tctttttcta     300
   ttactcttgg cctcctctag tacactctat atttttttat gcctcggtaa tgattttcat     360
   tttttttttt ccacctagcg gatgactctt tttttttctt agcgattggc attatcacat     420
   aatgaattat acattatata aagtaatgtg atttcttcga agaatatact aaaaaatgag     480
   caggcaagat aaacgaaggc aaagatgaca gagcagaaag ccctagtaaa gcgtattaca     540
   aatgaaacca agattcagat tgcgatctct ttaaagggtg gtcccctagc gatagagcac     600
   tcgatcttcc cagaaaaaga ggcagaagca gtagcagaac aggccacaca atcgcaagtg     660
   attaacgtcc acacaggtat agggtttctg gaccatatga tacatgctct ggccaagcat     720
   tccggctggt cgctaatcgt tgagtgcatt ggtgacttac acatagacga ccatcacacc     780
   actgaagact gcgggattgc tctcggtcaa gcttttaaag aggccctagg ggccgtgcgt     840
   ggagtaaaaa ggtttggatc aggatttgcg cctttggatg aggcactttc cagagcggtg     900
   gtagatcttt cgaacaggcc gtacgcagtt gtcgaacttg gtttgcaaag ggagaaagta     960
   ggagatctct cttgcgagat gatcccgcat tttcttgaaa gctttgcaga ggctagcaga    1020
   attaccctcc acgttgattg tctgcgaggc aagaatgatc atcaccgtag tgagagtgcg    1080
   ttcaaggctc ttgcggttgc cataagagaa gccacctcgc ccaatggtac caacgatgtt    1140
   ccctccacca aaggtgttct tatgtagtga caccgattat ttaaagctgc agcatacgat    1200
   atatatacat gtgtatatat gtatacctat gaatgtcagt aagtatgtat acgaacagta    1260
   tgatactgaa gatgacaagg taatgcatca ttctatacgt gtcattctga acgaggcgcg    1320
   ctttcctttt ttctttttgc tttttctttt tttttctctt gaactcgacg gatctatgcg    1380
   gtgtgaaata ccgcacagat gcgtaaggag aaaataccgc atcaggaaat tgtaagcgtt    1440
   aatattttgt taaaattcgc gttaaatttt tgttaaatca gctcattttt taaccaatag    1500
   gccgaaatcg gcaaaatccc ttataaatca aaagaataga ccgagatagg gttgagtgtt    1560
   gttccagttt ggaacaagag tccactatta aagaacgtgg actccaacgt caaagggcga    1620
   aaaaccgtct atcagggcga tggcccacta cgtgaaccat caccctaatc aagttttttg    1680
   gggtcgaggt gccgtaaagc actaaatcgg aaccctaaag ggagcccccg atttagagct 1740
   tgacggggaa agccggcgaa cgtggcgaga aaggaaggga agaaagcgaa aggagcgggc 1800
   gctagggcgc tggcaagtgt agcggtcacg ctgcgcgtaa ccaccacacc cgccgcgctt 1860
   aatgcgccgc tacagggcgc gtccattcgc cattcaggct gcgcaactgt tgggaagggc 1920
   gcggtgcggg cctcttcgct attacgccag ctggcgaaag ggggatgtgc tgcaaggcga 1980
   ttaagttggg taacgccagg gttttcccag tcacgacgtt gtaaaacgac ggccagtgag 2040
   cgcgcgtaat acgactcact atagggcgaa ttgggtaccg ggccccccct cgaggtcgac 2100
   ggcgcgccac tggtagagag cgactttgta tgccccaatt gcgaaacccg cgatatcctt 2160
   ctcgattctt tagtacccga ccaggacaag gaaaaggagg tcgaaacgtt tttgaagaaa 2220
   caagaggaac tacacggaag ctctaaagat ggcaaccagc cagaaactaa gaaaatgaag 2280
   ttgatggatc caactggcac cgctggcttg aacaacaata ccagccttcc aacttctgta 2340
   aataacggcg gtacgccagt gccaccagta ccgttacctt tcggtatacc tcctttcccc 2400
   atgtttccaa tgcccttcat gcctccaacg gctactatca caaatcctca tcaagctgac 2460
   gcaagcccta agaaatgaat aacaatactg acagtactaa ataattgcct acttggcttc 2520
   acatacgttg catacgtcga tatagataat aatgataatg acagcaggat tatcgtaata 2580
   cgtaatagct gaaaatctca aaaatgtgtg ggtcattacg taaataatga taggaatggg 2640
   attcttctat ttttcctttt tccattctag cagccgtcgg gaaaacgtgg catcctctct 2700
   ttcgggctca attggagtca cgctgccgtg agcatcctct ctttccatat ctaacaactg 2760
   agcacgtaac caatggaaaa gcatgagctt agcgttgctc caaaaaagta ttggatggtt 2820
   aataccattt gtctgttctc ttctgacttt gactcctcaa aaaaaaaaat ctacaatcaa 2880
   cagatcgctt caattacgcc ctcacaaaaa cttttttcct tcttcttcgc ccacgttaaa 2940
   ttttatccct catgttgtct aacggatttc tgcacttgat ttattataaa aagacaaaga 3000
   cataatactt ctctatcaat ttcagttatt gttcttcctt gcgttattct tctgttcttc 3060
   tttttctttt gtcatatata accataacca agtaatacat attcaaacta gtatgactga 3120
   caaaaaaact cttaaagact taagaaatcg tagttctgtt tacgattcaa tggttaaatc 3180
   acctaatcgt gctatgttgc gtgcaactgg tatgcaagat gaagactttg aaaaacctat 3240
   cgtcggtgtc atttcaactt gggctgaaaa cacaccttgt aatatccact tacatgactt 3300
   tggtaaacta gccaaagtcg gtgttaagga agctggtgct tggccagttc agttcggaac 3360
   aatcacggtt tctgatggaa tcgccatggg aacccaagga atgcgtttct ccttgacatc 3420
   tcgtgatatt attgcagatt ctattgaagc agccatggga ggtcataatg cggatgcttt 3480
   tgtagccatt ggcggttgtg ataaaaacat gcccggttct gttatcgcta tggctaacat 3540
   ggatatccca gccatttttg cttacggcgg aacaattgca cctggtaatt tagacggcaa 3600
   agatatcgat ttagtctctg tctttgaagg tgtcggccat tggaaccacg gcgatatgac 3660
   caaagaagaa gttaaagctt tggaatgtaa tgcttgtccc ggtcctggag gctgcggtgg 3720
   tatgtatact gctaacacaa tggcgacagc tattgaagtt ttgggactta gccttccggg 3780
   ttcatcttct cacccggctg aatccgcaga aaagaaagca gatattgaag aagctggtcg 3840
   cgctgttgtc aaaatgctcg aaatgggctt aaaaccttct gacattttaa cgcgtgaagc 3900
   ttttgaagat gctattactg taactatggc tctgggaggt tcaaccaact caacccttca 3960
   cctcttagct attgcccatg ctgctaatgt ggaattgaca cttgatgatt tcaatacttt 4020
   ccaagaaaaa gttcctcatt tggctgattt gaaaccttct ggtcaatatg tattccaaga 4080
   cctttacaag gtcggagggg taccagcagt tatgaaatat ctccttaaaa atggcttcct 4140
   tcatggtgac cgtatcactt gtactggcaa aacagtcgct gaaaatttga aggcttttga 4200
   tgatttaaca cctggtcaaa aggttattat gccgcttgaa aatcctaaac gtgaagatgg 4260
   tccgctcatt attctccatg gtaacttggc tccagacggt gccgttgcca aagtttctgg 4320
   tgtaaaagtg cgtcgtcatg tcggtcctgc taaggtcttt aattctgaag aagaagccat 4380
   tgaagctgtc ttgaatgatg atattgttga tggtgatgtt gttgtcgtac gttttgtagg 4440
   accaaagggc ggtcctggta tgcctgaaat gctttccctt tcatcaatga ttgttggtaa 4500
   agggcaaggt gaaaaagttg cccttctgac agatggccgc ttctcaggtg gtacttatgg 4560
   tcttgtcgtg ggtcatatcg ctcctgaagc acaagatggc ggtccaatcg cctacctgca 4620
   aacaggagac atagtcacta ttgaccaaga cactaaggaa ttacactttg atatctccga 4680
   tgaagagtta aaacatcgtc aagagaccat tgaattgcca ccgctctatt cacgcggtat 4740
   ccttggtaaa tatgctcaca tcgtttcgtc tgcttctagg ggagccgtaa cagacttttg 4800
   gaagcctgaa gaaactggca aaaaatgttg tcctggttgc tgtggttaag cggccgcgtt 4860
   aattcaaatt aattgatata gttttttaat gagtattgaa tctgtttaga aataatggaa 4920
   tattattttt atttatttat ttatattatt ggtcggctct tttcttctga aggtcaatga 4980
   caaaatgata tgaaggaaat aatgatttct aaaattttac aacgtaagat atttttacaa 5040
   aagcctagct catcttttgt catgcactat tttactcacg cttgaaatta acggccagtc 5100
   cactgcggag tcatttcaaa gtcatcctaa tcgatctatc gtttttgata gctcattttg 5160
   gagttcgcga ttgtcttctg ttattcacaa ctgttttaat ttttatttca ttctggaact 5220
   cttcgagttc tttgtaaagt ctttcatagt agcttacttt atcctccaac atatttaact 5280
   tcatgtcaat ttcggctctt aaattttcca catcatcaag ttcaacatca tcttttaact 5340
   tgaatttatt ctctagctct tccaaccaag cctcattgct ccttgattta ctggtgaaaa 5400
   gtgatacact ttgcgcgcaa tccaggtcaa aactttcctg caaagaattc accaatttct 5460
   cgacatcata gtacaatttg ttttgttctc ccatcacaat ttaatatacc tgatggattc 5520
   ttatgaagcg ctgggtaatg gacgtgtcac tctacttcgc ctttttccct actcctttta 5580
   gtacggaaga caatgctaat aaataagagg gtaataataa tattattaat cggcaaaaaa 5640
   gattaaacgc caagcgttta attatcagaa agcaaacgtc gtaccaatcc ttgaatgctt 5700
   cccaattgta tattaagagt catcacagca acatattctt gttattaaat taattattat 5760
   tgatttttga tattgtataa aaaaaccaaa tatgtataaa aaaagtgaat aaaaaatacc 5820
   aagtatggag aaatatatta gaagtctata cgttaaacca cccgggcccc ccctcgaggt 5880
   cgacggtatc gataagcttg atatcgaatt cctgcagccc gggggatcca ctagttctag 5940
   agcggccgct ctagaactag taccacaggt gttgtcctct gaggacataa aatacacacc 6000
   gagattcatc aactcattgc tggagttagc atatctacaa ttgggtgaaa tggggagcga 6060
   tttgcaggca tttgctcggc atgccggtag aggtgtggtc aataagagcg acctcatgct 6120
   atacctgaga aagcaacctg acctacagga aagagttact caagaataag aattttcgtt 6180
   ttaaaaccta agagtcactt taaaatttgt atacacttat tttttttata acttatttaa 6240
   taataaaaat cataaatcat aagaaattcg cttactctta attaatcaaa aagttaaaat 6300
   tgtacgaata gattcaccac ttcttaacaa atcaaaccct tcattgattt tctcgaatgg 6360
   caatacatgt gtaattaaag gatcaagagc aaacttcttc gccataaagt cggcaacaag 6420
   ttttggaaca ctatccttgc tcttaaaacc gccaaatata gctcccttcc atgtacgacc 6480
   gcttagcaac agcataggat tcatcgacaa attttgtgaa tcaggaggaa cacctacgat 6540
   cacactgact ccatatgcct cttgacagca ggacaacgca gttaccatag tatcaagacg 6600
   gcctataact tcaaaagaga aatcaactcc accgtttgac atttcagtaa ggacttcttg 6660
   tattggtttc ttataatctt gagggttaac acattcagta gccccgacct ccttagcttt 6720
   tgcaaatttg tccttattga tgtctacacc tataatcctc gctgcgcctg cagctttaca 6780
   ccccataata acgcttagtc ctactcctcc taaaccgaat actgcacaag tcgaaccctg 6840
   tgtaaccttt gcaactttaa ctgcggaacc gtaaccggtg gaaaatccgc accctatcaa 6900
   gcaaactttt tccagtggtg aagctgcatc gattttagcg acagatatct cgtccaccac 6960
   tgtgtattgg gaaaatgtag aagtaccaag gaaatggtgt ataggtttcc ctctgcatgt 7020
   aaatctgctt gtaccatcct gcatagtacc tctaggcata gacaaatcat ttttaaggca 7080
   gaaattaccc tcaggatgtt tgcagactct acacttacca cattgaggag tgaacagtgg 7140
   gatcacttta tcaccaggac gaacagtggt aacaccttca cctatggatt caacgattcc 7200
   ggcagcctcg tgtcccgcga ttactggcaa aggagtaact agagtgccac tcaccacatg 7260
   gtcgtcggat ctacagattc cggtggcaac catcttgatt ctaacctcgt gtgcttttgg 7320
   tggcgctact tctacttctt ctatgctaaa cggctttttc tcttcccaca aaactgccgc 7380
   tttacactta ataactttac cggctgttga catcctcagc tagctattgt aatatgtgtg 7440
   tttgtttgga ttattaagaa gaataattac aaaaaaaatt acaaaggaag gtaattacaa 7500
   cagaattaag aaaggacaag aaggaggaag agaatcagtt cattatttct tctttgttat 7560
   ataacaaacc caagtagcga tttggccata cattaaaagt tgagaaccac cctccctggc 7620
   aacagccaca actcgttacc attgttcatc acgatcatga aactcgctgt cagctgaaat 7680
   ttcacctcag tggatctctc tttttattct tcatcgttcc actaaccttt ttccatcagc 7740
   tggcagggaa cggaaagtgg aatcccattt agcgagcttc ctcttttctt caagaaaaga 7800
   cgaagcttgt gtgtgggtgc gcgcgctagt atctttccac attaagaaat ataccataaa 7860
   ggttacttag acatcactat ggctatatat atatatatat atatatgtaa cttagcacca 7920
   tcgcgcgtgc atcactgcat gtgttaaccg aaaagtttgg cgaacacttc accgacacgg 7980
   tcatttagat ctgtcgtctg cattgcacgt cccttagcct taaatcctag gcgggagcat 8040
   tctcgtgtaa ttgtgcagcc tgcgtagcaa ctcaacatag cgtagtctac ccagtttttc 8100
   aagggtttat cgttagaaga ttctcccttt tcttcctgct cacaaatctt aaagtcatac 8160
   attgcacgac taaatgcaag catgcggatc ccccgggctg caggaattcg atatcaagct 8220
   tatcgatacc gtcgactggc cattaatctt tcccatatta gatttcgcca agccatgaaa 8280
   gttcaagaaa ggtctttaga cgaattaccc ttcatttctc aaactggcgt caagggatcc 8340
   tggtatggtt ttatcgtttt atttctggtt cttatagcat cgttttggac ttctctgttc 8400
   ccattaggcg gttcaggagc cagcgcagaa tcattctttg aaggatactt atcctttcca 8460
   attttgattg tctgttacgt tggacataaa ctgtatacta gaaattggac tttgatggtg 8520
   aaactagaag atatggatct tgataccggc agaaaacaag tagatttgac tcttcgtagg 8580
   gaagaaatga ggattgagcg agaaacatta gcaaaaagat ccttcgtaac aagattttta 8640
   catttctggt gttgaaggga aagatatgag ctatacagcg gaatttccat atcactcaga 8700
   ttttgttatc taattttttc cttcccacgt ccgcgggaat ctgtgtatat tactgcatct 8760
   agatatatgt tatcttatct tggcgcgtac atttaatttt caacgtattc tataagaaat 8820
   tgcgggagtt tttttcatgt agatgatact gactgcacgc aaatataggc atgatttata 8880
   ggcatgattt gatggctgta ccgataggaa cgctaagagt aacttcagaa tcgttatcct 8940
   ggcggaaaaa attcatttgt aaactttaaa aaaaaaagcc aatatcccca aaattattaa 9000
   gagcgcctcc attattaact aaaatttcac tcagcatcca caatgtatca ggtatctact 9060
   acagatatta catgtggcga aaaagacaag aacaatgcaa tagcgcatca agaaaaaaca 9120
   caaagctttc aatcaatgaa tcgaaaatgt cattaaaata gtatataaat tgaaactaag 9180
   tcataaagct ataaaaagaa aatttattta aatgcaagat ttaaagtaaa ttcacggccc 9240
   tgcaggcctc agctcttgtt ttgttctgca aataacttac ccatcttttt caaaacttta 9300
   ggtgcaccct cctttgctag aataagttct atccaataca tcctatttgg atctgcttga 9360
   gcttctttca tcacggatac gaattcattt tctgttctca caattttgga cacaactctg 9420
   tcttccgttg ccccgaaact ttctggcagt tttgagtaat tccacatagg aatgtcatta 9480
   taactctggt tcggaccatg aatttccctc tcaaccgtgt aaccatcgtt attaatgata 9540
   aagcagattg ggtttatctt ctctctaatg gctagtccta attcttggac agtcagttgc 9600
   aatgatccat ctccgataaa caataaatgt ctagattctt tatctgcaat ttggctgcct 9660
   agagctgcgg ggaaagtgta tcctatagat ccccacaagg gttgaccaat aaaatgtgat 9720
   ttcgatttca gaaatataga tgaggcaccg aagaaagaag tgccttgttc agccacgatc 9780
   gtctcattac tttgggtcaa attttcgaca gcttgccaca gtctatcttg tgacaacagc 9840
   gcgttagaag gtacaaaatc ttcttgcttt ttatctatgt acttgccttt atattcaatt 9900
   tcggacaagt caagaagaga tgatatcagg gattcgaagt cgaaattttg gattctttcg 9960
   ttgaaaattt taccttcatc gatattcaag gaaatcattt tattttcatt aagatggtga 10020
   gtaaatgcac ccgtactaga atcggtaagc tttacaccca acataagaat aaaatcagca 10080
   gattccacaa attccttcaa gtttggctct gacagagtac cgttgtaaat ccccaaaaat 10140
   gagggcaatg cttcatcaac agatgattta ccaaagttca aagtagtaat aggtaactta 10200
   gtctttgaaa taaactgagt aacagtcttc tctaggccga acgatataat ttcatggcct 10260
   gtgattacaa ttggtttctt ggcattcttc agactttcct gtattttgtt cagaatctct 10320
   tgatcagatg tattcgacgt ggaattttcc ttcttaagag gcaaggatgg tttttcagcc 10380
   ttagcggcag ctacatctac aggtaaattg atgtaaaccg gctttctttc ctttagtaag 10440
   gcagacaaca ctctatcaat ttcaacagtt gcattctcgg ctgtcaataa agtcctggca 10500
   gcagtaaccg gttcgtgcat cttcataaag tgcttgaaat caccatcagc caacgtatgg 10560
   tgaacaaact taccttcgtt ctgcactttc gaggtaggag atcccacgat ctcaacaaca 10620
   ggcaggttct cagcatagga gcccgctaag ccattaactg cggataattc gccaacacca 10680
   aatgtagtca agaatgccgc agcctttttc gttcttgcgt acccgtcggc catataggag 10740
   gcatttaact cattagcatt tcccacccat ttcatatctt tgtgtgaaat aatttgatct 10800
   agaaattgca aattgtagtc acctggtact ccgaatattt cttctatacc taattcgtgt 10860
   aatctgtcca acagatagtc acctactgta tacattttgt ttactagttt atgtgtgttt 10920
   attcgaaact aagttcttgg tgttttaaaa ctaaaaaaaa gactaactat aaaagtagaa 10980
   tttaagaagt ttaagaaata gatttacaga attacaatca atacctaccg tctttatata 11040
   cttattagtc aagtagggga ataatttcag ggaactggtt tcaacctttt ttttcagctt 11100
   tttccaaatc agagagagca gaaggtaata gaaggtgtaa gaaaatgaga tagatacatg 11160
   cgtgggtcaa ttgccttgtg tcatcattta ctccaggcag gttgcatcac tccattgagg 11220
   ttgtgcccgt tttttgcctg tttgtgcccc tgttctctgt agttgcgcta agagaatgga 11280
   cctatgaact gatggttggt gaagaaaaca atattttggt gctgggattc tttttttttc 11340
   tggatgccag cttaaaaagc gggctccatt atatttagtg gatgccagga ataaactgtt 11400
   cacccagaca cctacgatgt tatatattct gtgtaacccg ccccctattt tgggcatgta 11460
   cgggttacag cagaattaaa aggctaattt tttgactaaa taaagttagg aaaatcacta 11520
   ctattaatta tttacgtatt ctttgaaatg gcagtattga taatgataaa ctcgaactga 11580
   aaaagcgtgt tttttattca aaatgattct aactccctta cgtaatcaag gaatcttttt 11640
   gccttggcct ccgcgtcatt aaacttcttg ttgttgacgc taacattcaa cgctagtata 11700
   tattcgtttt tttcaggtaa gttcttttca acgggtctta ctgatgaggc agtcgcgtct 11760
   gaacctgtta agaggtcaaa tatgtcttct tgaccgtacg tgtcttgcat gttattagct 11820
   ttgggaattt gcatcaagtc ataggaaaat ttaaatcttg gctctcttgg gctcaaggtg 11880
   acaaggtcct cgaaaatagg gcgcgcccca ccgcggtgga gctccagctt ttgttccctt 11940
   tagtgagggt taattgcgcg cttggcgtaa tcatggtcat agctgtttcc tgtgtgaaat 12000
   tgttatccgc tcacaattcc acacaacata cgagccggaa gcataaagtg taaagcctgg 12060
   ggtgcctaat gagtgagcta actcacatta attgcgttgc gctcactgcc cgctttccag 12120
   tcgggaaacc tgtcgtgcca gctgcattaa tgaatcggcc aacgcgcggg gagaggcggt 12180
   ttgcgtattg ggcgctcttc cgcttcctcg ctcactgact cgctgcgctc ggtcgttcgg 12240
   ctgcggcgag cggtatcagc tcactcaaag gcggtaatac ggttatccac agaatcaggg 12300
   gataacgcag gaaagaacat gtgagcaaaa ggccagcaaa aggccaggaa ccgtaaaaag 12360
   gccgcgttgc tggcgttttt ccataggctc cgcccccctg acgagcatca caaaaatcga 12420
   cgctcaagtc agaggtggcg aaacccgaca ggactataaa gataccaggc gtttccccct 12480
   ggaagctccc tcgtgcgctc tcctgttccg accctgccgc ttaccggata cctgtccgcc 12540
   tttctccctt cgggaagcgt ggcgctttct catagctcac gctgtaggta tctcagttcg 12600
   gtgtaggtcg ttcgctccaa gctgggctgt gtgcacgaac cccccgttca gcccgaccgc 12660
   tgcgccttat ccggtaacta tcgtcttgag tccaacccgg taagacacga cttatcgcca 12720
   ctggcagcag ccactggtaa caggattagc agagcgaggt atgtaggcgg tgctacagag 12780
   ttcttgaagt ggtggcctaa ctacggctac actagaagaa cagtatttgg tatctgcgct 12840
   ctgctgaagc cagttacctt cggaaaaaga gttggtagct cttgatccgg caaacaaacc 12900
   accgctggta gcggtggttt ttttgtttgc aagcagcaga ttacgcgcag aaaaaaagga 12960
   tctcaagaag atcctttgat cttttctacg gggtctgacg ctcagtggaa cgaaaactca 13020
   cgttaaggga ttttggtcat gagattatca aaaaggatct tcacctagat ccttttaaat 13080
   taaaaatgaa gttttaaatc aatctaaagt atatatgagt aaacttggtc tgacagttac 13140
   caatgcttaa tcagtgaggc acctatctca gcgatctgtc tatttcgttc atccatagtt 13200
   gcctgactcc ccgtcgtgta gataactacg atacgggagg gcttaccatc tggccccagt 13260
   gctgcaatga taccgcgaga cccacgctca ccggctccag atttatcagc aataaaccag 13320
   ccagccggaa gggccgagcg cagaagtggt cctgcaactt tatccgcctc catccagtct 13380
   attaattgtt gccgggaagc tagagtaagt agttcgccag ttaatagttt gcgcaacgtt 13440
   gttgccattg ctacaggcat cgtggtgtca cgctcgtcgt ttggtatggc ttcattcagc 13500
   tccggttccc aacgatcaag gcgagttaca tgatccccca tgttgtgcaa aaaagcggtt 13560
   agctccttcg gtcctccgat cgttgtcaga agtaagttgg ccgcagtgtt atcactcatg 13620
   gttatggcag cactgcataa ttctcttact gtcatgccat ccgtaagatg cttttctgtg 13680
   actggtgagt actcaaccaa gtcattctga gaatagtgta tgcggcgacc gagttgctct 13740
   tgcccggcgt caatacggga taataccgcg ccacatagca gaactttaaa agtgctcatc 13800
   attggaaaac gttcttcggg gcgaaaactc tcaaggatct taccgctgtt gagatccagt 13860
   tcgatgtaac ccactcgtgc acccaactga tcttcagcat cttttacttt caccagcgtt 13920
   tctgggtgag caaaaacagg aaggcaaaat gccgcaaaaa agggaataag ggcgacacgg 13980
   aaatgttgaa tactcatact cttccttttt caatattatt gaagcattta tcagggttat 14040
   tgtctcatga gcggatacat atttgaatgt atttagaaaa ataaacaaat aggggttccg 14100
   cgcacatttc cccgaaaagt gccacctgaa cgaagcatct gtgcttcatt ttgtagaaca 14160
   aaaatgcaac gcgagagcgc taatttttca aacaaagaat ctgagctgca tttttacaga 14220
   acagaaatgc aacgcgaaag cgctatttta ccaacgaaga atctgtgctt catttttgta 14280
   aaacaaaaat gcaacgcgag agcgctaatt tttcaaacaa agaatctgag ctgcattttt 14340
   acagaacaga aatgcaacgc gagagcgcta ttttaccaac aaagaatcta tacttctttt 14400
   ttgttctaca aaaatgcatc ccgagagcgc tatttttcta acaaagcatc ttagattact 14460
   ttttttctcc tttgtgcgct ctataatgca gtctcttgat aactttttgc actgtaggtc 14520
   cgttaaggtt agaagaaggc tactttggtg tctattttct cttccataaa aaaagcctga 14580
   ctccacttcc cgcgtttact gattactagc gaagctgcgg gtgcattttt tcaagataaa 14640
   ggcatccccg attatattct ataccgatgt ggattgcgca tactttgtga acagaaagtg 14700
   atagcgttga tgattcttca ttggtcagaa aattatgaac ggtttcttct attttgtctc 14760
   tatatactac gtataggaaa tgtttacatt ttcgtattgt tttcgattca ctctatgaat 14820
   agttcttact acaatttttt tgtctaaaga gtaatactag agataaacat aaaaaatgta 14880
   gaggtcgagt ttagatgcaa gttcaaggag cgaaaggtgg atgggtaggt tatataggga 14940
   tatagcacag agatatatag caaagagata cttttgagca atgtttgtgg aagcggtatt 15000
   cgcaatattt tagtagctcg ttacagtccg gtgcgttttt ggttttttga aagtgcgtct 15060
   tcagagcgct tttggttttc aaaagcgctc tgaagttcct atactttcta gagaatagga 15120
   acttcggaat aggaacttca aagcgtttcc gaaaacgagc gcttccgaaa atgcaacgcg 15180
   agctgcgcac atacagctca ctgttcacgt cgcacctata tctgcgtgtt gcctgtatat 15240
   atatatacat gagaagaacg gcatagtgcg tgtttatgct taaatgcgta cttatatgcg 15300
   tctatttatg taggatgaaa ggtagtctag tacctcctgt gatattatcc cattccatgc 15360
   ggggtatcgt atgcttcctt cagcactacc ctttagctgt tctatatgct gccactcctc 15420
   aattggatta gtctcatcct tcaatgctat catttccttt gatattggat catactaaga 15480
   aaccattatt atcatgacat taacctataa aaataggcgt atcacgaggc cctttcgtc 15539
<210> 43
   <211> 1644
   <212> DNA
   <213> artificial sequence
<220>
   <223> B subtilis kivD cocon optimized for S cerevisiae expression
<400> 43
   atgtatacag taggtgacta tctgttggac agattacacg aattaggtat agaagaaata     60
   ttcggagtac caggtgacta caatttgcaa tttctagatc aaattatttc acacaaagat    120
   atgaaatggg tgggaaatgc taatgagtta aatgcctcct atatggccga cgggtacgca    180
   agaacgaaaa aggctgcggc attcttgact acatttggtg ttggcgaatt atccgcagtt    240
   aatggcttag cgggctccta tgctgagaac ctgcctgttg ttgagatcgt gggatctcct    300
   acctcgaaag tgcagaacga aggtaagttt gttcaccata cgttggctga tggtgatttc    360
   aagcacttta tgaagatgca cgaaccggtt actgctgcca ggactttatt gacagccgag    420
   aatgcaactg ttgaaattga tagagtgttg tctgccttac taaaggaaag aaagccggtt    480
   tacatcaatt tacctgtaga tgtagctgcc gctaaggctg aaaaaccatc cttgcctctt    540
   aagaaggaaa attccacgtc gaatacatct gatcaagaga ttctgaacaa aatacaggaa    600
   agtctgaaga atgccaagaa accaattgta atcacaggcc atgaaattat atcgttcggc    660
   ctagagaaga ctgttactca gtttatttca aagactaagt tacctattac tactttgaac    720
   tttggtaaat catctgttga tgaagcattg ccctcatttt tggggattta caacggtact    780
   ctgtcagagc caaacttgaa ggaatttgtg gaatctgctg attttattct tatgttgggt    840
   gtaaagctta ccgattctag tacgggtgca tttactcacc atcttaatga aaataaaatg    900
   atttccttga atatcgatga aggtaaaatt ttcaacgaaa gaatccaaaa tttcgacttc    960
   gaatccctga tatcatctct tcttgacttg tccgaaattg aatataaagg caagtacata   1020
   gataaaaagc aagaagattt tgtaccttct aacgcgctgt tgtcacaaga tagactgtgg   1080
   caagctgtcg aaaatttgac ccaaagtaat gagacgatcg tggctgaaca aggcacttct   1140
   ttcttcggtg cctcatctat atttctgaaa tcgaaatcac attttattgg tcaacccttg   1200
   tggggatcta taggatacac tttccccgca gctctaggca gccaaattgc agataaagaa   1260
   tctagacatt tattgtttat cggagatgga tcattgcaac tgactgtcca agaattagga   1320
   ctagccatta gagagaagat aaacccaatc tgctttatca ttaataacga tggttacacg   1380
   gttgagaggg aaattcatgg tccgaaccag agttataatg acattcctat gtggaattac   1440
   tcaaaactgc cagaaagttt cggggcaacg gaagacagag ttgtgtccaa aattgtgaga   1500
   acagaaaatg aattcgtatc cgtgatgaaa gaagctcaag cagatccaaa taggatgtat   1560
   tggatagaac ttattctagc aaaggagggt gcacctaaag ttttgaaaaa gatgggtaag   1620
   ttatttgcag aacaaaacaa gagc                                          1644
<210> 44
   <211> 548
   <212> PRT
   <213> Bacillus subtilis
<400> 44
   Met Tyr Thr Val Gly Asp Tyr Leu Leu Asp Arg Leu His Glu Leu Gly
   1               5                   10                  15
   Ile Glu Glu Ile Phe Gly Val Pro Gly Asp Tyr Asn Leu Gln Phe Leu
               20                  25                  30
   Asp Gln Ile Ile Ser His Lys Asp Met Lys Trp Val Gly Asn Ala Asn
           35                  40                  45
   Glu Leu Asn Ala Ser Tyr Met Ala Asp Gly Tyr Ala Arg Thr Lys Lys
       50                  55                  60
   Ala Ala Ala Phe Leu Thr Thr Phe Gly Val Gly Glu Leu Ser Ala Val
   65                  70                  75                  80
   Asn Gly Leu Ala Gly Ser Tyr Ala Glu Asn Leu Pro Val Val Glu Ile
                   85                  90                  95
   Val Gly Ser Pro Thr Ser Lys Val Gln Asn Glu Gly Lys Phe Val His
               100                 105                 110
   His Thr Leu Ala Asp Gly Asp Phe Lys His Phe Met Lys Met His Glu
           115                 120                 125
   Pro Val Thr Ala Ala Arg Thr Leu Leu Thr Ala Glu Asn Ala Thr Val
       130                 135                 140
   Glu Ile Asp Arg Val Leu Ser Ala Leu Leu Lys Glu Arg Lys Pro Val
   145                 150                 155                 160
   Tyr Ile Asn Leu Pro Val Asp Val Ala Ala Ala Lys Ala Glu Lys Pro
                   165                 170                 175
   Ser Leu Pro Leu Lys Lys Glu Asn Ser Thr Ser Asn Thr Ser Asp Gln
               180                 185                 190
   Glu Ile Leu Asn Lys Ile Gln Glu Ser Leu Lys Asn Ala Lys Lys Pro
           195                 200                 205
   Ile Val Ile Thr Gly His Glu Ile Ile Ser Phe Gly Leu Glu Lys Thr
       210                 215                 220
   Val Thr Gln Phe Ile Ser Lys Thr Lys Leu Pro Ile Thr Thr Leu Asn
   225                 230                 235                 240
   Phe Gly Lys Ser Ser Val Asp Glu Ala Leu Pro Ser Phe Leu Gly Ile
                   245                 250                 255
   Tyr Asn Gly Thr Leu Ser Glu Pro Asn Leu Lys Glu Phe Val Glu Ser
               260                 265                 270
   Ala Asp Phe Ile Leu Met Leu Gly Val Lys Leu Thr Asp Ser Ser Thr
           275                 280                 285
   Gly Ala Phe Thr His His Leu Asn Glu Asn Lys Met Ile Ser Leu Asn
       290                 295                 300
   Ile Asp Glu Gly Lys Ile Phe Asn Glu Arg Ile Gln Asn Phe Asp Phe
   305 310 315 320
   Glu Ser Leu Ile Ser Ser Leu Leu Asp Leu Ser Glu Ile Glu Tyr Lys
   325 330 335
   Gly Lys Tyr Ile Asp Lys Lys Gln Glu Asp Phe Val Pro Ser Asn Ala
   340 345 350
   Leu Leu Ser Gln Asp Arg Leu Trp Gln Ala Val Glu Asn Leu Thr Gln
   355 360 365
   Ser Asn Glu Thr Ile Val Ala Glu Gln Gly Thr Ser Phe Phe Gly Ala
   370 375 380
   Ser Ser Ile Phe Leu Lys Ser Lys Ser His Phe Ile Gly Gln Pro Leu
   385 390 395 400
   Trp Gly Ser Ile Gly Tyr Thr Phe Pro Ala Ala Leu Gly Ser Gln Ile
   405 410 415
   Ala Asp Lys Glu Ser Arg His Leu Leu Phe Ile Gly Asp Gly Ser Leu
   420 425 430
   Gln Leu Thr Val Gln Glu Leu Gly Leu Ala Ile Arg Glu Lys Ile Asn
   435 440 445
   Pro Ile Cys Phe Ile Ile Asn Asn Asp Gly Tyr Thr Val Glu Arg Glu
   450 455 460
   Ile His Gly Pro Asn Gln Ser Tyr Asn Asp Ile Pro Met Trp Asn Tyr
   465 470 475 480
   Ser Lys Leu Pro Glu Ser Phe Gly Ala Thr Glu Asp Arg Val Val Ser
   485 490 495
   Lys Ile Val Arg Thr Glu Asn Glu Phe Val Ser Val Met Lys Glu Ala
   500 505 510
   Gln Ala Asp Pro Asn Arg Met Tyr Trp Ile Glu Leu Ile Leu Ala Lys
   515 520 525
   Glu Gly Ala Pro Lys Val Leu Lys Lys Met Gly Lys Leu Phe Ala Glu
   530 535 540
   Gln Asn Lys Ser
   545
<210> 45
   <211> 1125
   <212> DNA
   <213> artificial sequence
<220>
   <223> horse ADH coding region codon optimized for S. cerevisiae expression
<400> 45
   atgtcaacag ccggtaaagt tattaagtgt aaagcggcag ttttgtggga agagaaaaag    60
   ccgtttagca tagaagaagt agaagtagcg ccaccaaaag cacacgaggt tagaatcaag   120
   atggttgcca ccggaatctg tagatccgac gaccatgtgg tgagtggcac tctagttact   180
   cctttgccag taatcgcggg acacgaggct gccggaatcg ttgaatccat aggtgaaggt   240
   gttaccactg ttcgtcctgg tgataaagtg atcccactgt tcactcctca atgtggtaag   300
   tgtagagtct gcaaacatcc tgagggtaat ttctgcctta aaaatgattt gtctatgcct   360
   agaggtacta tgcaggatgg tacaagcaga tttacatgca gagggaaacc tatacaccat   420
   ttccttggta cttctacatt ttcccaatac acagtggtgg acgagatatc tgtcgctaaa   480
   atcgatgcag cttcaccact ggaaaaagtt tgcttgatag ggtgcggatt ttccaccggt   540
   tacggttccg cagttaaagt tgcaaaggtt acacagggtt cgacttgtgc agtattcggt   600
   ttaggaggag taggactaag cgttattatg gggtgtaaag ctgcaggcgc agcgaggatt    660
   ataggtgtag acatcaataa ggacaaattt gcaaaagcta aggaggtcgg ggctactgaa   720
   tgtgttaacc ctcaagatta taagaaacca atacaagaag tccttactga aatgtcaaac   780
   ggtggagttg atttctcttt tgaagttata ggccgtcttg atactatggt aactgcgttg   840
   tcctgctgtc aagaggcata tggagtcagt gtgatcgtag gtgttcctcc tgattcacaa   900
   aatttgtcga tgaatcctat gctgttgcta agcggtcgta catggaaggg agctatattt   960
   ggcggtttta agagcaagga tagtgttcca aaacttgttg ccgactttat ggcgaagaag  1020
   tttgctcttg atcctttaat tacacatgta ttgccattcg agaaaatcaa tgaagggttt  1080
   gatttgttaa gaagtggtga atctattcgt acaattttaa ctttt                  1125
<210> 46
   <211> 375
   <212> PRT
   <213> Equus caballus
<400> 46
   Met Ser Thr Ala Gly Lys Val Ile Lys Cys Lys Ala Ala Val Leu Trp
   1               5                   10                  15
   Glu Glu Lys Lys Pro Phe Ser Ile Glu Glu Val Glu Val Ala Pro Pro
               20                  25                  30
   Lys Ala His Glu Val Arg Ile Lys Met Val Ala Thr Gly Ile Cys Arg
           35                  40                  45
   Ser Asp Asp His Val Val Ser Gly Thr Leu Val Thr Pro Leu Pro Val
       50                  55                  60
   Ile Ala Gly His Glu Ala Ala Gly Ile Val Glu Ser Ile Gly Glu Gly
   65 70 75 80
   Val Thr Thr Val Arg Pro Gly Asp Lys Val Ile Pro Leu Phe Thr Pro
   85 90 95
   Gln Cys Gly Lys Cys Arg Val Cys Lys His Pro Glu Gly Asn Phe Cys
   100 105 110
   Leu Lys Asn Asp Leu Ser Met Pro Arg Gly Thr Met Gln Asp Gly Thr
   115 120 125
   Ser Arg Phe Thr Cys Arg Gly Lys Pro Ile His His Phe Leu Gly Thr
   130 135 140
   Ser Thr Phe Ser Gln Tyr Thr Val Val Asp Glu Ile Ser Val Ala Lys
   145 150 155 160
   Ile Asp Ala Ala Ser Pro Leu Glu Lys Val Cys Leu Ile Gly Cys Gly
   165 170 175
   Phe Ser Thr Gly Tyr Gly Ser Ala Val Lys Val Ala Lys Val Thr Gln
   180 185 190
   Gly Ser Thr Cys Ala Val Phe Gly Leu Gly Gly Val Gly Leu Ser Val
   195 200 205
   Ile Met Gly Cys Lys Ala Ala Gly Ala Ala Arg Ile Ile Gly Val Asp
   210 215 220
   Ile Asn Lys Asp Lys Phe Ala Lys Ala Lys Glu Val Gly Ala Thr Glu
   225 230 235 240
   Cys Val Asn Pro Gln Asp Tyr Lys Lys Pro Ile Gln Glu Val Leu Thr
   245 250 255
   Glu Met Ser Asn Gly Gly Val Asp Phe Ser Phe Glu Val Ile Gly Arg
   260 265 270
   Leu Asp Thr Met Val Thr Ala Leu Ser Cys Cys Gln Glu Ala Tyr Gly
   275 280 285
   Val Ser Val Ile Val Gly Val Pro Pro Asp Ser Gln Asn Leu Ser Met
   290 295 300
   Asn Pro Met Leu Leu Leu Ser Gly Arg Thr Trp Lys Gly Ala Ile Phe
   305 310 315 320
   Gly Gly Phe Lys Ser Lys Asp Ser Val Pro Lys Leu Val Ala Asp Phe
   325 330 335
   Met Ala Lys Lys Phe Ala Leu Asp Pro Leu Ile Thr His Val Leu Pro
   340 345 350
   Phe Glu Lys Ile Asn Glu Gly Phe Asp Leu Leu Arg Ser Gly Glu Ser
   355 360 365
   Ile Arg Thr Ile Leu Thr Phe
   370 375
<210> 47
   <211> 9089
   <212> DNA
   <213> artificial sequence
<220>
   <223> constructed plasmid
<400> 47
   tcgcgcgttt cggtgatgac ggtgaaaacc tctgacacat gcagctcccg gagacggtca     60
   cagcttgtct gtaagcggat gccgggagca gacaagcccg tcagggcgcg tcagcgggtg    120
   ttggcgggtg tcggggctgg cttaactatg cggcatcaga gcagattgta ctgagagtgc    180
   accataccac agcttttcaa ttcaattcat catttttttt ttattctttt ttttgatttc    240
   ggtttctttg aaattttttt gattcggtaa tctccgaaca gaaggaagaa cgaaggaagg    300
   agcacagact tagattggta tatatacgca tatgtagtgt tgaagaaaca tgaaattgcc    360
   cagtattctt aacccaactg cacagaacaa aaacctgcag gaaacgaaga taaatcatgt    420
   cgaaagctac atataaggaa cgtgctgcta ctcatcctag tcctgttgct gccaagctat    480
   ttaatatcat gcacgaaaag caaacaaact tgtgtgcttc attggatgtt cgtaccacca    540
   aggaattact ggagttagtt gaagcattag gtcccaaaat ttgtttacta aaaacacatg    600
   tggatatctt gactgatttt tccatggagg gcacagttaa gccgctaaag gcattatccg    660
   ccaagtacaa ttttttactc ttcgaagaca gaaaatttgc tgacattggt aatacagtca    720
   aattgcagta ctctgcgggt gtatacagaa tagcagaatg ggcagacatt acgaatgcac    780
   acggtgtggt gggcccaggt attgttagcg gtttgaagca ggcggcagaa gaagtaacaa    840
   aggaacctag aggccttttg atgttagcag aattgtcatg caagggctcc ctatctactg    900
   gagaatatac taagggtact gttgacattg cgaagagcga caaagatttt gttatcggct    960
   ttattgctca aagagacatg ggtggaagag atgaaggtta cgattggttg attatgacac   1020
   ccggtgtggg tttagatgac aagggagacg cattgggtca acagtataga accgtggatg   1080
   atgtggtctc tacaggatct gacattatta ttgttggaag aggactattt gcaaagggaa   1140
   gggatgctaa ggtagagggt gaacgttaca gaaaagcagg ctgggaagca tatttgagaa   1200
   gatgcggcca gcaaaactaa aaaactgtat tataagtaaa tgcatgtata ctaaactcac   1260
   aaattagagc ttcaatttaa ttatatcagt tattacccta tgcggtgtga aataccgcac   1320
   agatgcgtaa ggagaaaata ccgcatcagg aaattgtaaa cgttaatatt ttgttaaaat   1380
   tcgcgttaaa tttttgttaa atcagctcat tttttaacca ataggccgaa atcggcaaaa   1440
   tcccttataa atcaaaagaa tagaccgaga tagggttgag tgttgttcca gtttggaaca 1500
   agagtccact attaaagaac gtggactcca acgtcaaagg gcgaaaaacc gtctatcagg 1560
   gcgatggccc actacgtgaa ccatcaccct aatcaagttt tttggggtcg aggtgccgta 1620
   aagcactaaa tcggaaccct aaagggagcc cccgatttag agcttgacgg ggaaagccgg 1680
   cgaacgtggc gagaaaggaa gggaagaaag cgaaaggagc gggcgctagg gcgctggcaa 1740
   gtgtagcggt cacgctgcgc gtaaccacca cacccgccgc gcttaatgcg ccgctacagg 1800
   gcgcgtcgcg ccattcgcca ttcaggctgc gcaactgttg ggaagggcga tcggtgcggg 1860
   cctcttcgct attacgccag ctggcgaaag ggggatgtgc tgcaaggcga ttaagttggg 1920
   taacgccagg gttttcccag tcacgacgtt gtaaaacgac ggccagtgag cgcgcgtaat 1980
   acgactcact atagggcgaa ttgggtaccg ggccccccct cgaggtcgac tggccattaa 2040
   tctttcccat attagatttc gccaagccat gaaagttcaa gaaaggtctt tagacgaatt 2100
   acccttcatt tctcaaactg gcgtcaaggg atcctggtat ggttttatcg ttttatttct 2160
   ggttcttata gcatcgtttt ggacttctct gttcccatta ggcggttcag gagccagcgc 2220
   agaatcattc tttgaaggat acttatcctt tccaattttg attgtctgtt acgttggaca 2280
   taaactgtat actagaaatt ggactttgat ggtgaaacta gaagatatgg atcttgatac 2340
   cggcagaaaa caagtagatt tgactcttcg tagggaagaa atgaggattg agcgagaaac 2400
   attagcaaaa agatccttcg taacaagatt tttacatttc tggtgttgaa gggaaagata 2460
   tgagctatac agcggaattt ccatatcact cagattttgt tatctaattt tttccttccc 2520
   acgtccgcgg gaatctgtgt atattactgc atctagatat atgttatctt atcttggcgc 2580
   gtacatttaa ttttcaacgt attctataag aaattgcggg agtttttttc atgtagatga 2640
   tactgactgc acgcaaatat aggcatgatt tataggcatg atttgatggc tgtaccgata 2700
   ggaacgctaa gagtaacttc agaatcgtta tcctggcgga aaaaattcat ttgtaaactt 2760
   taaaaaaaaa agccaatatc cccaaaatta ttaagagcgc ctccattatt aactaaaatt 2820
   tcactcagca tccacaatgt atcaggtatc tactacagat attacatgtg gcgaaaaaga 2880
   caagaacaat gcaatagcgc atcaagaaaa aacacaaagc tttcaatcaa tgaatcgaaa 2940
   atgtcattaa aatagtatat aaattgaaac taagtcataa agctataaaa agaaaattta 3000
   tttaaatgca agatttaaag taaattcacg gccctgcagg ccctaacctg ctaggacaca 3060
   acgtctttgc ctggtaaagt ttctagctga cgtgattcct tcacctgtgg atccggcaat 3120
   tgtaaaggtt gtgaaaccct cagcttcata accgacacct gcaaatgact ttgcattctt 3180
   aacaaagata gttgtatcaa tttcacgttc gaatctatta aggttatcga tgttcttaga 3240
   ataaatgtag gcggaatgtt ttctattctg ctcagctatc ttggcgtatt taatggcttc 3300
   atcaatgtcc ttcactctaa ctataggcaa aattggcatc atcaactccg tcataacgaa 3360
   cggatggttt gcgttgactt cacaaataat acactttaca ttacttggtg actctacatc 3420
   tatttcatcc aaaaacagtt tagcgtcctt accaacccac ttcttattaa tgaaatattc 3480
   ttgagtttca ttgttctttt gaagaacaag gtctatcagc ttggatactt ggtcttcatt 3540
   gataatgacg gcgttgtttt tcaacatgtt agagatcaga tcatctgcaa cgttttcaaa 3600
   cacgaacact tctttttccg cgatacaagg aagattgttg tcaaacgaac aaccttcaat 3660
   aatgcttctg ccggccttct cgatatctgc tgtatcgtct acaataaccg gaggattacc 3720
   cgcgccagct ccgatggcct ttttaccaga attaagaagg gtttttacca tacccgggcc 3780
   acccgtaccg cacaacaatt ttatggatgg atgtttgata atagcgtcta aactttccat 3840
   agttgggttc tttatagtag tgacaaggtt ttcaggtcca ccacagctaa ttatggcttt 3900
   gtttatcatt tctactgcga aagcgacaca ctttttggcg catgggtgac cattaaatac 3960
   aactgcattc cccgcagcta tcatacctat agaattgcag ataacggttt ctgttggatt 4020
   cgtgcttgga gttatagcgc cgataactcc gtatggactc atttcaacca ctgttagtcc 4080
   attatcgccg gaccatgctg ttgttgtcag atcttcagtg cctggggtat acttggccac 4140
   taattcatgt ttcaagattt tatcctcata ccttcccatg tgggtttcct ccaggatcat 4200
   tgtggctaag acctctttat tctgtaatgc ggcttttctt atttcggtga ttattttctc 4260
   tctttgttcc tttgtgtagt gtagggaaag aatcttttgt gcatgtactg cagaagaaat 4320
   ggcattctca acattttcaa atactccaaa acatgaagag ttatctttgt aattctttaa 4380
   gttgatgttt tcaccattag tcttcacttt caagtctttg gtggttggga ttaaggtatc 4440
   tttatccatg gtgtttgttt atgtgtgttt attcgaaact aagttcttgg tgttttaaaa 4500
   ctaaaaaaaa gactaactat aaaagtagaa tttaagaagt ttaagaaata gatttacaga 4560
   attacaatca atacctaccg tctttatata cttattagtc aagtagggga ataatttcag 4620
   ggaactggtt tcaacctttt ttttcagctt tttccaaatc agagagagca gaaggtaata 4680
   gaaggtgtaa gaaaatgaga tagatacatg cgtgggtcaa ttgccttgtg tcatcattta 4740
   ctccaggcag gttgcatcac tccattgagg ttgtgcccgt tttttgcctg tttgtgcccc 4800
   tgttctctgt agttgcgcta agagaatgga cctatgaact gatggttggt gaagaaaaca 4860
   atattttggt gctgggattc tttttttttc tggatgccag cttaaaaagc gggctccatt 4920
   atatttagtg gatgccagga ataaactgtt cacccagaca cctacgatgt tatatattct 4980
   gtgtaacccg ccccctattt tgggcatgta cgggttacag cagaattaaa aggctaattt 5040
   tttgactaaa taaagttagg aaaatcacta ctattaatta tttacgtatt ctttgaaatg 5100
   gcagtattga taatgataaa ctcgaactga aaaagcgtgt tttttattca aaatgattct 5160
   aactccctta cgtaatcaag gaatcttttt gccttggcct ccgcgtcatt aaacttcttg 5220
   ttgttgacgc taacattcaa cgctagtata tattcgtttt tttcaggtaa gttcttttca 5280
   acgggtctta ctgatgaggc agtcgcgtct gaacctgtta agaggtcaaa tatgtcttct 5340
   tgaccgtacg tgtcttgcat gttattagct ttgggaattt gcatcaagtc ataggaaaat 5400
   ttaaatcttg gctctcttgg gctcaaggtg acaaggtcct cgaaaatagg gcgcgcccca 5460
   ccgcggtgga gctccagctt ttgttccctt tagtgagggt taattgcgcg cttggcgtaa 5520
   tcatggtcat agctgtttcc tgtgtgaaat tgttatccgc tcacaattcc acacaacata 5580
   ggagccggaa gcataaagtg taaagcctgg ggtgcctaat gagtgaggta actcacatta 5640
   attgcgttgc gctcactgcc cgctttccag tcgggaaacc tgtcgtgcca gctgcattaa 5700
   tgaatcggcc aacgcgcggg gagaggcggt ttgcgtattg ggcgctcttc cgcttcctcg 5760
   ctcactgact cgctgcgctc ggtcgttcgg ctgcggcgag cggtatcagc tcactcaaag 5820
   gcggtaatac ggttatccac agaatcaggg gataacgcag gaaagaacat gtgagcaaaa 5880
   ggccagcaaa aggccaggaa ccgtaaaaag gccgcgttgc tggcgttttt ccataggctc 5940
   cgcccccctg acgagcatca caaaaatcga cgctcaagtc agaggtggcg aaacccgaca 6000
   ggactataaa gataccaggc gtttccccct ggaagctccc tcgtgcgctc tcctgttccg 6060
   accctgccgc ttaccggata cctgtccgcc tttctccctt cgggaagcgt ggcgctttct 6120
   catagctcac gctgtaggta tctcagttcg gtgtaggtcg ttcgctccaa gctgggctgt 6180
   gtgcacgaac cccccgttca gcccgaccgc tgcgccttat ccggtaacta tcgtcttgag 6240
   tccaacccgg taagacacga cttatcgcca ctggcagcag ccactggtaa caggattagc 6300
   agagcgaggt atgtaggcgg tgctacagag ttcttgaagt ggtggcctaa ctacggctac 6360
   actagaagga cagtatttgg tatctgcgct ctgctgaagc cagttacctt cggaaaaaga 6420
   gttggtagct cttgatccgg caaacaaacc accgctggta gcggtggttt ttttgtttgc 6480
   aagcagcaga ttacgcgcag aaaaaaagga tctcaagaag atcctttgat cttttctacg 6540
   gggtctgacg ctcagtggaa cgaaaactca cgttaaggga ttttggtcat gagattatca 6600
   aaaaggatct tcacctagat ccttttaaat taaaaatgaa gttttaaatc aatctaaagt 6660
   atatatgagt aaacttggtc tgacagttac caatgcttaa tcagtgaggc acctatctca 6720
   gcgatctgtc tatttcgttc atccatagtt gcctgactcc ccgtcgtgta gataactacg 6780
   atacgggagg gcttaccatc tggccccagt gctgcaatga taccgcgaga cccacgctca 6840
   ccggctccag atttatcagc aataaaccag ccagccggaa gggccgagcg cagaagtggt 6900
   cctgcaactt tatccgcctc catccagtct attaattgtt gccgggaagc tagagtaagt 6960
   agttcgccag ttaatagttt gcgcaacgtt gttgccattg ctacaggcat cgtggtgtca 7020
   cgctcgtcgt ttggtatggc ttcattcagc tccggttccc aacgatcaag gcgagttaca 7080
   tgatccccca tgttgtgcaa aaaagcggtt agctccttcg gtcctccgat cgttgtcaga 7140
   agtaagttgg ccgcagtgtt atcactcatg gttatggcag cactgcataa ttctcttact 7200
   gtcatgccat ccgtaagatg cttttctgtg actggtgagt actcaaccaa gtcattctga 7260
   gaatagtgta tgcggcgacc gagttgctct tgcccggcgt caatacggga taataccgcg 7320
   ccacatagca gaactttaaa agtgctcatc attggaaaac gttcttcggg gcgaaaactc 7380
   tcaaggatct taccgctgtt gagatccagt tcgatgtaac ccactcgtgc acccaactga 7440
   tcttcagcat cttttacttt caccagcgtt tctgggtgag caaaaacagg aaggcaaaat 7500
   gccgcaaaaa agggaataag ggcgacacgg aaatgttgaa tactcatact cttccttttt 7560
   caatattatt gaagcattta tcagggttat tgtctcatga gcggatacat atttgaatgt 7620
   atttagaaaa ataaacaaat aggggttccg cgcacatttc cccgaaaagt gccacctgaa 7680
   cgaagcatct gtgcttcatt ttgtagaaca aaaatgcaac gcgagagcgc taatttttca 7740
   aacaaagaat ctgagctgca tttttacaga acagaaatgc aacgcgaaag cgctatttta 7800
   ccaacgaaga atctgtgctt catttttgta aaacaaaaat gcaacgcgag agcgctaatt 7860
   tttcaaacaa agaatctgag ctgcattttt acagaacaga aatgcaacgc gagagcgcta 7920
   ttttaccaac aaagaatcta tacttctttt ttgttctaca aaaatgcatc ccgagagcgc 7980
   tatttttcta acaaagcatc ttagattact ttttttctcc tttgtgcgct ctataatgca 8040
   gtctcttgat aactttttgc actgtaggtc cgttaaggtt agaagaaggc tactttggtg 8100
   tctattttct cttccataaa aaaagcctga ctccacttcc cgcgtttact gattactagc 8160
   gaagctgcgg gtgcattttt tcaagataaa ggcatccccg attatattct ataccgatgt 8220
   ggattgcgca tactttgtga acagaaagtg atagcgttga tgattcttca ttggtcagaa 8280
   aattatgaac ggtttcttct attttgtctc tatatactac gtataggaaa tgtttacatt 8340
   ttcgtattgt tttcgattca ctctatgaat agttcttact acaatttttt tgtctaaaga 8400
   gtaatactag agataaacat aaaaaatgta gaggtcgagt ttagatgcaa gttcaaggag 8460
   cgaaaggtgg atgggtaggt tatataggga tatagcacag agatatatag caaagagata 8520
   cttttgagca atgtttgtgg aagcggtatt cgcaatattt tagtagctcg ttacagtccg 8580
   gtgcgttttt ggttttttga aagtgcgtct tcagagcgct tttggttttc aaaagcgctc 8640
   tgaagttcct atactttcta gagaatagga acttcggaat aggaacttca aagcgtttcc 8700
   gaaaacgagc gcttccgaaa atgcaacgcg agctgcgcac atacagctca ctgttcacgt 8760
   cgcacctata tctgcgtgtt gcctgtatat atatatacat gagaagaacg gcatagtgcg 8820
   tgtttatgct taaatgcgta cttatatgcg tctatttatg taggatgaaa ggtagtctag 8880
   tacctcctgt gatattatcc cattccatgc ggggtatcgt atgcttcctt cagcactacc 8940
   ctttagctgt tctatatgct gccactcctc aattggatta gtctcatcct tcaatgctat 9000
   catttccttt gatattggat catactaaga aaccattatt atcatgacat taacctataa 9060
   aaataggcgt atcacgaggc cctttcgtc 9089
<210> 48
   <211> 1023
   <212> DNA
   <213> Saccharomyces cerevisiae
<400> 48
   caccgcggtg gggcgcgccc tattttcgag gaccttgtca ccttgagccc aagagagcca 60
   agatttaaat tttcctatga cttgatgcaa attcccaaag ctaataacat gcaagacacg 120
   tacggtcaag aagacatatt tgacctctta acaggttcag acgcgactgc ctcatcagta 180
   agacccgttg aaaagaactt acctgaaaaa aacgaatata tactagcgtt gaatgttagc 240
   gtcaacaaca agaagtttaa tgacgcggag gccaaggcaa aaagattcct tgattacgta    300
   agggagttag aatcattttg aataaaaaac acgctttttc agttcgagtt tatcattatc    360
   aatactgcca tttcaaagaa tacgtaaata attaatagta gtgattttcc taactttatt    420
   tagtcaaaaa attagccttt taattctgct gtaacccgta catgcccaaa atagggggcg    480
   ggttacacag aatatataac atcgtaggtg tctgggtgaa cagtttattc ctggcatcca    540
   ctaaatataa tggagcccgc tttttaagct ggcatccaga aaaaaaaaga atcccagcac    600
   caaaatattg ttttcttcac caaccatcag ttcataggtc cattctctta gcgcaactac    660
   agagaacagg ggcacaaaca ggcaaaaaac gggcacaacc tcaatggagt gatgcaacct    720
   gcctggagta aatgatgaca caaggcaatt gacccacgca tgtatctatc tcattttctt    780
   acaccttcta ttaccttctg ctctctctga tttggaaaaa gctgaaaaaa aaggttgaaa    840
   ccagttccct gaaattattc ccctacttga ctaataagta tataaagacg gtaggtattg    900
   attgtaattc tgtaaatcta tttcttaaac ttcttaaatt ctacttttat agttagtctt    960
   ttttttagtt ttaaaacacc aagaacttag tttcgaataa acacacataa actagtaaac    1020
   aaa                                                                 1023
<210> 49
   <211> 21
   <212> DNA
   <213> artificial sequence
<220>
   <223> primer
<400> 49
   caaaagctga gctccaccgc g 21
<210> 50
   <211> 44
   <212> DNA
   <213> artificial sequence
<220>
   <223> primer
<400> 50
   gtttactagt ttatgtgtgt ttattcgaaa ctaagttctt ggtg 44
<210> 51
   <211> 8994
   <212> DNA
   <213> artificial sequence
<220>
   <223> constructed plasmid
<400> 51
   ctagttctag agcggccgcc accgcggtgg agctccagct tttgttccct ttagtgaggg 60
   ttaattgcgc gcttggcgta atcatggtca tagctgtttc ctgtgtgaaa ttgttatccg 120
   ctcacaattc cacacaacat aggagccgga agcataaagt gtaaagcctg gggtgcctaa 180
   tgagtgaggt aactcacatt aattgcgttg cgctcactgc ccgctttcca gtcgggaaac 240
   ctgtcgtgcc agctgcatta atgaatcggc caacgcgcgg ggagaggcgg tttgcgtatt 300
   gggcgctctt ccgcttcctc gctcactgac tcgctgcgct cggtcgttcg gctgcggcga 360
   gcggtatcag ctcactcaaa ggcggtaata cggttatcca cagaatcagg ggataacgca 420
   ggaaagaaca tgtgagcaaa aggccagcaa aaggccagga accgtaaaaa ggccgcgttg 480
   ctggcgtttt tccataggct ccgcccccct gacgagcatc acaaaaatcg acgctcaagt 540
   cagaggtggc gaaacccgac aggactataa agataccagg cgtttccccc tggaagctcc 600
   ctcgtgcgct ctcctgttcc gaccctgccg cttaccggat acctgtccgc ctttctccct 660
   tcgggaagcg tggcgctttc tcatagctca cgctgtaggt atctcagttc ggtgtaggtc 720
   gttcgctcca agctgggctg tgtgcacgaa ccccccgttc agcccgaccg ctgcgcctta 780
   tccggtaact atcgtcttga gtccaacccg gtaagacacg acttatcgcc actggcagca 840
   gccactggta acaggattag cagagcgagg tatgtaggcg gtgctacaga gttcttgaag 900
   tggtggccta actacggcta cactagaagg acagtatttg gtatctgcgc tctgctgaag 960
   ccagttacct tcggaaaaag agttggtagc tcttgatccg gcaaacaaac caccgctggt 1020
   agcggtggtt tttttgtttg caagcagcag attacgcgca gaaaaaaagg atctcaagaa 1080
   gatcctttga tcttttctac ggggtctgac gctcagtgga acgaaaactc acgttaaggg 1140
   attttggtca tgagattatc aaaaaggatc ttcacctaga tccttttaaa ttaaaaatga 1200
   agttttaaat caatctaaag tatatatgag taaacttggt ctgacagtta ccaatgctta 1260
   atcagtgagg cacctatctc agcgatctgt ctatttcgtt catccatagt tgcctgactc 1320
   cccgtcgtgt agataactac gatacgggag ggcttaccat ctggccccag tgctgcaatg 1380
   ataccgcgag acccacgctc accggctcca gatttatcag caataaacca gccagccgga 1440
   agggccgagc gcagaagtgg tcctgcaact ttatccgcct ccatccagtc tattaattgt 1500
   tgccgggaag ctagagtaag tagttcgcca gttaatagtt tgcgcaacgt tgttgccatt 1560
   gctacaggca tcgtggtgtc acgctcgtcg tttggtatgg cttcattcag ctccggttcc 1620
   caacgatcaa ggcgagttac atgatccccc atgttgtgca aaaaagcggt tagctccttc 1680
   ggtcctccga tcgttgtcag aagtaagttg gccgcagtgt tatcactcat ggttatggca 1740
   gcactgcata attctcttac tgtcatgcca tccgtaagat gcttttctgt gactggtgag 1800
   tactcaacca agtcattctg agaatagtgt atgcggcgac cgagttgctc ttgcccggcg 1860
   tcaatacggg ataataccgc gccacatagc agaactttaa aagtgctcat cattggaaaa 1920
   cgttcttcgg ggcgaaaact ctcaaggatc ttaccgctgt tgagatccag ttcgatgtaa 1980
   cccactcgtg cacccaactg atcttcagca tcttttactt tcaccagcgt ttctgggtga 2040
   gcaaaaacag gaaggcaaaa tgccgcaaaa aagggaataa gggcgacacg gaaatgttga 2100
   atactcatac tcttcctttt tcaatattat tgaagcattt atcagggtta ttgtctcatg 2160
   agcggataca tatttgaatg tatttagaaa aataaacaaa taggggttcc gcgcacattt 2220
   ccccgaaaag tgccacctga acgaagcatc tgtgcttcat tttgtagaac aaaaatgcaa 2280
   cgcgagagcg ctaatttttc aaacaaagaa tctgagctgc atttttacag aacagaaatg 2340
   caacgcgaaa gcgctatttt accaacgaag aatctgtgct tcatttttgt aaaacaaaaa 2400
   tgcaacgcga gagcgctaat ttttcaaaca aagaatctga gctgcatttt tacagaacag 2460
   aaatgcaacg cgagagcgct attttaccaa caaagaatct atacttcttt tttgttctac 2520
   aaaaatgcat cccgagagcg ctatttttct aacaaagcat cttagattac tttttttctc 2580
   ctttgtgcgc tctataatgc agtctcttga taactttttg cactgtaggt ccgttaaggt 2640
   tagaagaagg ctactttggt gtctattttc tcttccataa aaaaagcctg actccacttc 2700
   ccgcgtttac tgattactag cgaagctgcg ggtgcatttt ttcaagataa aggcatcccc 2760
   gattatattc tataccgatg tggattgcgc atactttgtg aacagaaagt gatagcgttg 2820
   atgattcttc attggtcaga aaattatgaa cggtttcttc tattttgtct ctatatacta 2880
   cgtataggaa atgtttacat tttcgtattg ttttcgattc actctatgaa tagttcttac 2940
   tacaattttt ttgtctaaag agtaatacta gagataaaca taaaaaatgt agaggtcgag 3000
   tttagatgca agttcaagga gcgaaaggtg gatgggtagg ttatataggg atatagcaca 3060
   gagatatata gcaaagagat acttttgagc aatgtttgtg gaagcggtat tcgcaatatt 3120
   ttagtagctc gttacagtcc ggtgcgtttt tggttttttg aaagtgcgtc ttcagagcgc 3180
   ttttggtttt caaaagcgct ctgaagttcc tatactttct agagaatagg aacttcggaa 3240
   taggaacttc aaagcgtttc cgaaaacgag cgcttccgaa aatgcaacgc gagctgcgca 3300
   catacagctc actgttcacg tcgcacctat atctgcgtgt tgcctgtata tatatataca 3360
   tgagaagaac ggcatagtgc gtgtttatgc ttaaatgcgt acttatatgc gtctatttat 3420
   gtaggatgaa aggtagtcta gtacctcctg tgatattatc ccattccatg cggggtatcg 3480
   tatgcttcct tcagcactac cctttagctg ttctatatgc tgccactcct caattggatt 3540
   agtctcatcc ttcaatgcta tcatttcctt tgatattgga tcatactaag aaaccattat 3600
   tatcatgaca ttaacctata aaaataggcg tatcacgagg ccctttcgtc tcgcgcgttt 3660
   cggtgatgac ggtgaaaacc tctgacacat gcagctcccg gagacggtca cagcttgtct 3720
   gtaagcggat gccgggagca gacaagcccg tcagggcgcg tcagcgggtg ttggcgggtg 3780
   tcggggctgg cttaactatg cggcatcaga gcagattgta ctgagagtgc accatatcga 3840
   ctacgtcgta aggccgtttc tgacagagta aaattcttga gggaactttc accattatgg 3900
   gaaatgcttc aagaaggtat tgacttaaac tccatcaaat ggtcaggtca ttgagtgttt 3960
   tttatttgtt gtattttttt ttttttagag aaaatcctcc aatatcaaat taggaatcgt 4020
   agtttcatga ttttctgtta cacctaactt tttgtgtggt gccctcctcc ttgtcaatat 4080
   taatgttaaa gtgcaattct ttttccttat cacgttgagc cattagtatc aatttgctta 4140
   cctgtattcc tttactatcc tcctttttct ccttcttgat aaatgtatgt agattgcgta 4200
   tatagtttcg tctaccctat gaacatattc cattttgtaa tttcgtgtcg tttctattat 4260
   gaatttcatt tataaagttt atgtacaaat atcataaaaa aagagaatct ttttaagcaa 4320
   ggattttctt aacttcttcg gcgacagcat caccgacttc ggtggtactg ttggaaccac 4380
   ctaaatcacc agttctgata cctgcatcca aaaccttttt aactgcatct tcaatggcct 4440
   taccttcttc aggcaagttc aatgacaatt tcaacatcat tgcagcagac aagatagtgg 4500
   cgatagggtc aaccttattc tttggcaaat ctggagcaga accgtggcat ggttcgtaca 4560
   aaccaaatgc ggtgttcttg tctggcaaag aggccaagga cgcagatggc aacaaaccca 4620
   aggaacctgg gataacggag gcttcatcgg agatgatatc accaaacatg ttgctggtga 4680
   ttataatacc atttaggtgg gttgggttct taactaggat catggcggca gaatcaatca 4740
   attgatgttg aaccttcaat gtagggaatt cgttcttgat ggtttcctcc acagtttttc 4800
   tccataatct tgaagaggcc aaaagattag ctttatccaa ggaccaaata ggcaatggtg 4860
   gctcatgttg tagggccatg aaagcggcca ttcttgtgat tctttgcact tctggaacgg 4920
   tgtattgttc actatcccaa gcgacaccat caccatcgtc ttcctttctc ttaccaaagt 4980
   aaatacctcc cactaattct ctgacaacaa cgaagtcagt acctttagca aattgtggct 5040
   tgattggaga taagtctaaa agagagtcgg atgcaaagtt acatggtctt aagttggcgt 5100
   acaattgaag ttctttacgg atttttagta aaccttgttc aggtctaaca ctaccggtac 5160
   cccatttagg accagccaca gcacctaaca aaacggcatc aaccttcttg gaggcttcca 5220
   gcgcctcatc tggaagtggg acacctgtag catcgatagc agcaccacca attaaatgat 5280
   tttcgaaatc gaacttgaca ttggaacgaa catcagaaat agctttaaga accttaatgg 5340
   cttcggctgt gatttcttga ccaacgtggt cacctggcaa aacgacgatc ttcttagggg 5400
   cagacatagg ggcagacatt agaatggtat atccttgaaa tatatatata tattgctgaa 5460
   atgtaaaagg taagaaaagt tagaaagtaa gacgattgct aaccacctat tggaaaaaac 5520
   aataggtcct taaataatat tgtcaacttc aagtattgtg atgcaagcat ttagtcatga 5580
   acgcttctct attctatatg aaaagccggt tccggcctct cacctttcct ttttctccca 5640
   atttttcagt tgaaaaaggt atatgcgtca ggcgacctct gaaattaaca aaaaatttcc 5700
   agtcatcgaa tttgattctg tgcgatagcg cccctgtgtg ttctcgttat gttgaggaaa 5760
   aaaataatgg ttgctaagag attcgaactc ttgcatctta cgatacctga gtattcccac 5820
   agttaactgc ggtcaagata tttcttgaat caggcgcctt agaccgctcg gccaaacaac 5880
   caattacttg ttgagaaata gagtataatt atcctataaa tataacgttt ttgaacacac 5940
   atgaacaagg aagtacagga caattgattt tgaagagaat gtggattttg atgtaattgt 6000
   tgggattcca tttttaataa ggcaataata ttaggtatgt ggatatacta gaagttctcc 6060
   tcgaccgtcg atatgcggtg tgaaataccg cacagatgcg taaggagaaa ataccgcatc 6120
   aggaaattgt aaacgttaat attttgttaa aattcgcgtt aaatttttgt taaatcagct 6180
   cattttttaa ccaataggcc gaaatcggca aaatccctta taaatcaaaa gaatagaccg 6240
   agatagggtt gagtgttgtt ccagtttgga acaagagtcc actattaaag aacgtggact 6300
   ccaacgtcaa agggcgaaaa accgtctatc agggcgatgg cccactacgt gaaccatcac 6360
   cctaatcaag ttttttgggg tcgaggtgcc gtaaagcact aaatcggaac cctaaaggga 6420
   gcccccgatt tagagcttga cggggaaagc cggcgaacgt ggcgagaaag gaagggaaga 6480
   aagcgaaagg agcgggcgct agggcgctgg caagtgtagc ggtcacgctg cgcgtaacca 6540
   ccacacccgc cgcgcttaat gcgccgctac agggcgcgtc gcgccattcg ccattcaggc 6600
   tgcgcaactg ttgggaaggg cgatcggtgc gggcctcttc gctattacgc cagctggcga 6660
   aagggggatg tgctgcaagg cgattaagtt gggtaacgcc agggttttcc cagtcacgac 6720
   gttgtaaaac gacggccagt gagcgcgcgt aatacgactc actatagggc gaattgggta 6780
   ccgggccccc cctcgaggtc gacggtatcg ataagcttga tatcgaattc ctgcagcccg 6840
   ggggatccgc atgcttgcat ttagtcgtgc aatgtatgac tttaagattt gtgagcagga 6900
   agaaaaggga gaatcttcta acgataaacc cttgaaaaac tgggtagact acgctatgtt 6960
   gagttgctac gcaggctgca caattacacg agaatgctcc cgcctaggat ttaaggctaa 7020
   gggacgtgca atgcagacga cagatctaaa tgaccgtgtc ggtgaagtgt tcgccaaact 7080
   tttcggttaa cacatgcagt gatgcacgcg cgatggtgct aagttacata tatatatata 7140
   tatatatata tagccatagt gatgtctaag taacctttat ggtatatttc ttaatgtgga 7200
   aagatactag cgcgcgcacc cacacacaag cttcgtcttt tcttgaagaa aagaggaagc 7260
   tcgctaaatg ggattccact ttccgttccc tgccagctga tggaaaaagg ttagtggaac 7320
   gatgaagaat aaaaagagag atccactgag gtgaaatttc agctgacagc gagtttcatg 7380
   atcgtgatga acaatggtaa cgagttgtgg ctgttgccag ggagggtggt tctcaacttt 7440
   taatgtatgg ccaaatcgct acttgggttt gttatataac aaagaagaaa taatgaactg 7500
   attctcttcc tccttcttgt cctttcttaa ttctgttgta attaccttcc tttgtaattt 7560
   tttttgtaat tattcttctt aataatccaa acaaacacac atattacaat agctagctga 7620
   ggatgaaggc attagtttat catggggatc acaaaatttc gttagaagac aaaccaaaac 7680
   ccactctgca gaaaccaaca gacgttgtgg ttagggtgtt gaaaacaaca atttgcggta 7740
   ctgacttggg aatatacaaa ggtaagaatc ctgaagtggc agatggcaga atcctgggtc 7800
   atgagggcgt tggcgtcatt gaagaagtgg gcgaatccgt gacacaattc aaaaaggggg 7860
   ataaagtttt aatctcctgc gttactagct gtggatcgtg tgattattgc aagaagcaac 7920
   tgtattcaca ctgtagagac ggtggctgga ttttaggtta catgatcgac ggtgtccaag 7980
   ccgaatacgt cagaatacca catgctgaca attcattgta taagatcccg caaactatcg 8040
   atgatgaaat tgcagtacta ctgtccgata ttttacctac tggacatgaa attggtgttc 8100
   aatatggtaa cgttcaacca ggcgatgctg tagcaattgt aggagcaggt cctgttggaa 8160
   tgtcagtttt gttaactgct caattttact cgcctagtac cattattgtt atcgacatgg 8220
   acgaaaaccg tttacaatta gcgaaggagc ttggggccac acacactatt aactccggta 8280
   ctgaaaatgt tgtcgaagct gtgcatcgta tagcagccga aggagtggat gtagcaatag 8340
   aagctgttgg tatacccgca acctgggaca tctgtcagga aattgtaaaa cccggcgctc 8400
   atattgccaa cgtgggagtt catggtgtta aggtggactt tgaaattcaa aagttgtgga 8460
   ttaagaatct aaccatcacc actggtttgg ttaacactaa tactacccca atgttgatga 8520
   aggtagcctc tactgataaa ttgcctttaa agaaaatgat tactcacagg tttgagttag 8580
   ctgaaatcga acacgcatat caggttttct tgaatggcgc taaagaaaaa gctatgaaga 8640
   ttattctatc taatgcaggt gccgcctaat taattaagag taagcgaatt tcttatgatt 8700
   tatgattttt attattaaat aagttataaa aaaaataagt gtatacaaat tttaaagtga 8760
   ctcttaggtt ttaaaacgaa aattcttatt cttgagtaac tctttcctgt aggtcaggtt 8820
   gctttctcag gtatagcatg aggtcgctct tattgaccac acctctaccg gcatgccgag 8880
   caaatgcctg caaatcgctc cccatttcac ccaattgtag atatgctaac tccagcaatg 8940
   agttgatgaa tctcggtgtg tattttatgt cctcagagga caacacctgt ggta 8994
<210> 52
   <211> 753
   <212> DNA
   <213> saccharomyces cerevisiae
<400> 52
   gcatgcttgc atttagtcgt gcaatgtatg actttaagat ttgtgagcag gaagaaaagg 60
   gagaatcttc taacgataaa cccttgaaaa actgggtaga ctacgctatg ttgagttgct 120
   acgcaggctg cacaattaca cgagaatgct cccgcctagg atttaaggct aagggacgtg 180
   caatgcagac gacagatcta aatgaccgtg tcggtgaagt gttcgccaaa cttttcggtt 240
   aacacatgca gtgatgcacg cgcgatggtg ctaagttaca tatatatata tatagccata 300
   gtgatgtcta agtaaccttt atggtatatt tcttaatgtg gaaagatact agcgcgcgca 360
   cccacacaca agcttcgtct tttcttgaag aaaagaggaa gctcgctaaa tgggattcca 420
   ctttccgttc cctgccagct gatggaaaaa ggttagtgga acgatgaaga ataaaaagag 480
   agatccactg aggtgaaatt tcagctgaca gcgagtttca tgatcgtgat gaacaatggt 540
   aacgagttgt ggctgttgcc agggagggtg gttctcaact tttaatgtat ggccaaatcg 600
   ctacttgggt ttgttatata acaaagaaga aataatgaac tgattctctt cctccttctt 660
   gtcctttctt aattctgttg taattacctt cctttgtaat tttttttgta attattcttc 720
   ttaataatcc aaacaaacac acatattaca ata 753
<210> 53
   <211> 316
   <212> DNA
   <213> Saccharomyces cerevisiae
<400> 53
   gagtaagcga atttcttatg atttatgatt tttattatta aataagttat aaaaaaaata      60
   agtgtataca aattttaaag tgactcttag gttttaaaac gaaaattctt attcttgagt     120
   aactctttcc tgtaggtcag gttgctttct caggtatagc atgaggtcgc tcttattgac     180
   cacacctcta ccggcatgcc gagcaaatgc ctgcaaatcg ctccccattt cacccaattg     240
   tagatatgct aactccagca atgagttgat gaatctcggt gtgtatttta tgtcctcaga     300
   ggacaacacc tgtggt                                                     316
<210> 54
   <211> 39
   <212> DNA
   <213> artificial sequence
<220>
   <223> primer
<400> 54
   cacacatatt acaatagcta gctgaggatg aaagctctg 39
<210> 55
   <211> 39
   <212> DNA
   <213> artificial sequence
<220>
   <223> primer
<400> 55
   cagagctttc atcctcagct agctattgta atatgtgtg 39
<210> 56
   <211> 9491
   <212> DNA
   <213> artificial sequence
<220>
   <223> constructed plasmid
<400> 56
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accataaatt cccgttttaa gagcttggtg agcgctagga gtcactgcca ggtatcgttt 240
   gaacacggca ttagtcaggg aagtcataac acagtccttt cccgcaattt tctttttcta 300
   ttactcttgg cctcctctag tacactctat atttttttat gcctcggtaa tgattttcat 360
   tttttttttt cccctagcgg atgactcttt ttttttctta gcgattggca ttatcacata 420
   atgaattata cattatataa agtaatgtga tttcttcgaa gaatatacta aaaaatgagc 480
   aggcaagata aacgaaggca aagatgacag agcagaaagc cctagtaaag cgtattacaa 540
   atgaaaccaa gattcagatt gcgatctctt taaagggtgg tcccctagcg atagagcact 600
   cgatcttccc agaaaaagag gcagaagcag tagcagaaca ggccacacaa tcgcaagtga 660
   ttaacgtcca cacaggtata gggtttctgg accatatgat acatgctctg gccaagcatt 720
   ccggctggtc gctaatcgtt gagtgcattg gtgacttaca catagacgac catcacacca 780
   ctgaagactg cgggattgct ctcggtcaag cttttaaaga ggccctactg gcgcgtggag 840
   taaaaaggtt tggatcagga tttgcgcctt tggatgaggc actttccaga gcggtggtag 900
   atctttcgaa caggccgtac gcagttgtcg aacttggttt gcaaagggag aaagtaggag 960
   atctctcttg cgagatgatc ccgcattttc ttgaaagctt tgcagaggct agcagaatta 1020
   ccctccacgt tgattgtctg cgaggcaaga atgatcatca ccgtagtgag agtgcgttca 1080
   aggctcttgc ggttgccata agagaagcca cctcgcccaa tggtaccaac gatgttccct 1140
   ccaccaaagg tgttcttatg tagtgacacc gattatttaa agctgcagca tacgatatat 1200
   atacatgtgt atatatgtat acctatgaat gtcagtaagt atgtatacga acagtatgat 1260
   actgaagatg acaaggtaat gcatcattct atacgtgtca ttctgaacga ggcgcgcttt 1320
   cctttttcct ttttgctttt tctttttttt tctcttgaac tcgacggatc tatgcggtgt 1380
   gaaataccgc acagatgcgt aaggagaaaa taccgcatca ggaaattgta aacgttaata 1440
   ttttgttaaa attcgcgtta aatttttgtt aaatcagctc attttttaac caataggccg 1500
   aaatcggcaa aatcccttat aaatcaaaag aatagaccga gatagggttg agtgttgttc 1560
   cagtttggaa caagagtcca ctattaaaga acgtggactc caacgtcaaa gggcgaaaaa 1620
   ccgtctatca gggcgatggc ccactacgtg aaccatcacc ctaatcaagt tttttggggt 1680
   cgaggtgccg taaagcacta aatcggaacc ctaaagggag cccccgattt agagcttgac 1740
   ggggaaagcc ggcgaacgtg gcgagaaagg aagggaagaa agcgaaagga gcgggcgcta 1800
   gggcgctggc aagtgtagcg gtcacgctgc gcgtaaccac cacacccgcc gcgcttaatg 1860
   cgccgctaca gggcgcgtcg cgccattcgc cattcaggct gcgcaactgt tgggaagggc 1920
   gatcggtgcg ggcctcttcg ctattacgcc agctggcgaa agggggatgt gctgcaaggc 1980
   gattaagttg ggtaacgcca gggttttccc agtcacgacg ttgtaaaacg acggccagtg 2040
   agcgcgcgta atacgactca ctatagggcg aattgggtac cgggcccccc ctcgaggtcg 2100
   acggcgcgcc actggtagag agcgactttg tatgccccaa ttgcgaaacc cgcgatatcc 2160
   ttctcgattc tttagtaccc gaccaggaca aggaaaagga ggtcgaaacg tttttgaaga 2220
   aacaagagga actacacgga agctctaaag atggcaacca gccagaaact aagaaaatga 2280
   agttgatgga tccaactggc accgctggct tgaacaacaa taccagcctt ccaacttctg 2340
   taaataacgg cggtacgcca gtgccaccag taccgttacc tttcggtata cctcctttcc 2400
   ccatgtttcc aatgcccttc atgcctccaa cggctactat cacaaatcct catcaagctg 2460
   acgcaagccc taagaaatga ataacaatac tgacagtact aaataattgc ctacttggct 2520
   tcacatacgt tgcatacgtc gatatagata ataatgataa tgacagcagg attatcgtaa 2580
   tacgtaatag ttgaaaatct caaaaatgtg tgggtcatta cgtaaataat gataggaatg 2640
   ggattcttct atttttcctt tttccattct agcagccgtc gggaaaacgt ggcatcctct 2700
   ctttcgggct caattggagt cacgctgccg tgagcatcct ctctttccat atctaacaac 2760
   tgagcacgta accaatggaa aagcatgagc ttagcgttgc tccaaaaaag tattggatgg 2820
   ttaataccat ttgtctgttc tcttctgact ttgactcctc aaaaaaaaaa aatctacaat 2880
   caacagatcg cttcaattac gccctcacaa aaactttttt ccttcttctt cgcccacgtt 2940
   aaattttatc cctcatgttg tctaacggat ttctgcactt gatttattat aaaaagacaa 3000
   agacataata cttctctatc aatttcagtt attgttcttc cttgcgttat tcttctgttc 3060
   ttcttttctt tttgtcatat ataaccataa ccaagtaata catattcaaa ctagtatgac 3120
   tgacaaaaaa actcttaaag acttaagaaa tcgtagttct gtttacgatt caatggttaa 3180
   atcacctaat cgtgctatgt tgcgtgcaac tggtatgcaa gatgaagact ttgaaaaacc 3240
   tatcgtcggt gtcatttcaa cttgggctga aaacacacct tgtaatatcc acttacatga 3300
   ctttggtaaa ctagccaaag tcggtgttaa ggaagctggt gcttggccag ttcagttcgg 3360
   aacaatcacg gtttctgatg gaatcgccat gggaacccaa ggaatgcgtt tctccttgac 3420
   atctcgtgat attattgcag attctattga agcagccatg ggaggtcata atgcggatgc 3480
   ttttgtagcc attggcggtt gtgataaaaa catgcccggt tctgttatcg ctatggctaa 3540
   catggatatc ccagccattt ttgcttacgg cggaacaatt gcacctggta atttagacgg 3600
   caaagatatc gatttagtct ctgtctttga aggtgtcggc cattggaacc acggcgatat 3660
   gaccaaagaa gaagttaaag ctttggaatg taatgcttgt cccggtcctg gaggctgcgg 3720
   tggtatgtat actgctaaca caatggcgac agctattgaa gttttgggac ttagccttcc 3780
   gggttcatct tctcacccgg ctgaatccgc agaaaagaaa gcagatattg aagaagctgg 3840
   tcgcgctgtt gtcaaaatgc tcgaaatggg cttaaaacct tctgacattt taacgcgtga 3900
   agcttttgaa gatgctatta ctgtaactat ggctctggga ggttcaacca actcaaccct 3960
   tcacctctta gctattgccc atgctgctaa tgtggaattg acacttgatg atttcaatac 4020
   tttccaagaa aaagttcctc atttggctga tttgaaacct tctggtcaat atgtattcca 4080
   agacctttac aaggtcggag gggtaccagc agttatgaaa tatctcctta aaaatggctt 4140
   ccttcatggt gaccgtatca cttgtactgg caaaacagtc gctgaaaatt tgaaggcttt 4200
   tgatgattta acacctggtc aaaaggttat tatgccgctt gaaaatccta aacgtgaaga 4260
   tggtccgctc attattctcc atggtaactt ggctccagac ggtgccgttg ccaaagtttc 4320
   tggtgtaaaa gtgcgtcgtc atgtcggtcc tgctaaggtc tttaattctg aagaagaagc 4380
   cattgaagct gtcttgaatg atgatattgt tgatggtgat gttgttgtcg tacgttttgt 4440
   aggaccaaag ggcggtcctg gtatgcctga aatgctttcc ctttcatcaa tgattgttgg 4500
   taaagggcaa ggtgaaaaag ttgcccttct gacagatggc cgcttctcag gtggtactta 4560
   tggtcttgtc gtgggtcata tcgctcctga agcacaagat ggcggtccaa tcgcctacct 4620
   gcaaacagga gacatagtca ctattgacca agacactaag gaattacact ttgatatctc 4680
   cgatgaagag ttaaaacatc gtcaagagac cattgaattg ccaccgctct attcacgcgg 4740
   tatccttggt aaatatgctc acatcgtttc gtctgcttct aggggagccg taacagactt 4800
   ttggaagcct gaagaaactg gcaaaaaatg ttgtcctggt tgctgtggtt aagcggccgc 4860
   gttaattcaa attaattgat atagtttttt aatgagtatt gaatctgttt agaaataatg 4920
   gaatattatt tttatttatt tatttatatt attggtcggc tcttttcttc tgaaggtcaa 4980
   tgacaaaatg atatgaagga aataatgatt tctaaaattt tacaacgtaa gatattttta 5040
   caaaagccta gctcatcttt tgtcatgcac tattttactc acgcttgaaa ttaacggcca 5100
   gtccactgcg gagtcatttc aaagtcatcc taatcgatct atcgtttttg atagctcatt 5160
   ttggagttcg cgattgtctt ctgttattca caactgtttt aatttttatt tcattctgga 5220
   actcttcgag ttctttgtaa agtctttcat agtagcttac tttatcctcc aacatattta 5280
   acttcatgtc aatttcggct cttaaatttt ccacatcatc aagttcaaca tcatctttta 5340
   acttgaattt attctctagc tcttccaacc aagcctcatt gctccttgat ttactggtga 5400
   aaagtgatac actttgcgcg caatccaggt caaaactttc ctgcaaagaa ttcaccaatt 5460
   tctcgacatc atagtacaat ttgttttgtt ctcccatcac aatttaatat acctgatgga 5520
   ttcttatgaa gcgctgggta atggacgtgt cactctactt cgcctttttc cctactcctt 5580
   ttagtacgga agacaatgct aataaataag agggtaataa taatattatt aatcggcaaa 5640
   aaagattaaa cgccaagcgt ttaattatca gaaagcaaac gtcgtaccaa tccttgaatg 5700
   cttcccaatt gtatattaag agtcatcaca gcaacatatt cttgttatta aattaattat 5760
   tattgatttt tgatattgta taaaaaaacc aaatatgtat aaaaaaagtg aataaaaaat 5820
   accaagtatg gagaaatata ttagaagtct atacgttaaa ccaccgcggt ggagctccag 5880
   cttttgttcc ctttagtgag ggttaattgc gcgcttggcg taatcatggt catagctgtt 5940
   tcctgtgtga aattgttatc cgctcacaat tccacacaac ataggagccg gaagcataaa 6000
   gtgtaaagcc tggggtgcct aatgagtgag gtaactcaca ttaattgcgt tgcgctcact 6060
   gcccgctttc cagtcgggaa acctgtcgtg ccagctgcat taatgaatcg gccaacgcgc 6120
   ggggagaggc ggtttgcgta ttgggcgctc ttccgcttcc tcgctcactg actcgctgcg 6180
   ctcggtcgtt cggctgcggc gagcggtatc agctcactca aaggcggtaa tacggttatc 6240
   cacagaatca ggggataacg caggaaagaa catgtgagca aaaggccagc aaaaggccag 6300
   gaaccgtaaa aaggccgcgt tgctggcgtt tttccatagg ctccgccccc ctgacgagca 6360
   tcacaaaaat cgacgctcaa gtcagaggtg gcgaaacccg acaggactat aaagatacca 6420
   ggcgtttccc cctggaagct ccctcgtgcg ctctcctgtt ccgaccctgc cgcttaccgg 6480
   atacctgtcc gcctttctcc cttcgggaag cgtggcgctt tctcatagct cacgctgtag 6540
   gtatctcagt tcggtgtagg tcgttcgctc caagctgggc tgtgtgcacg aaccccccgt 6600
   tcagcccgac cgctgcgcct tatccggtaa ctatcgtctt gagtccaacc cggtaagaca 6660
   cgacttatcg ccactggcag cagccactgg taacaggatt agcagagcga ggtatgtagg 6720
   cggtgctaca gagttcttga agtggtggcc taactacggc tacactagaa ggacagtatt 6780
   tggtatctgc gctctgctga agccagttac cttcggaaaa agagttggta gctcttgatc 6840
   cggcaaacaa accaccgctg gtagcggtgg tttttttgtt tgcaagcagc agattacgcg 6900
   cagaaaaaaa ggatctcaag aagatccttt gatcttttct acggggtctg acgctcagtg 6960
   gaacgaaaac tcacgttaag ggattttggt catgagatta tcaaaaagga tcttcaccta 7020
   gatcctttta aattaaaaat gaagttttaa atcaatctaa agtatatatg agtaaacttg 7080
   gtctgacagt taccaatgct taatcagtga ggcacctatc tcagcgatct gtctatttcg 7140
   ttcatccata gttgcctgac tccccgtcgt gtagataact acgatacggg agggcttacc 7200
   atctggcccc agtgctgcaa tgataccgcg agacccacgc tcaccggctc cagatttatc 7260
   agcaataaac cagccagccg gaagggccga gcgcagaagt ggtcctgcaa ctttatccgc 7320
   ctccatccag tctattaatt gttgccggga agctagagta agtagttcgc cagttaatag 7380
   tttgcgcaac gttgttgcca ttgctacagg catcgtggtg tcacgctcgt cgtttggtat 7440
   ggcttcattc agctccggtt cccaacgatc aaggcgagtt acatgatccc ccatgttgtg 7500
   caaaaaagcg gttagctcct tcggtcctcc gatcgttgtc agaagtaagt tggccgcagt 7560
   gttatcactc atggttatgg cagcactgca taattctctt actgtcatgc catccgtaag 7620
   atgcttttct gtgactggtg agtactcaac caagtcattc tgagaatagt gtatgcggcg 7680
   accgagttgc tcttgcccgg cgtcaatacg ggataatacc gcgccacata gcagaacttt 7740
   aaaagtgctc atcattggaa aacgttcttc ggggcgaaaa ctctcaagga tcttaccgct 7800
   gttgagatcc agttcgatgt aacccactcg tgcacccaac tgatcttcag catcttttac 7860
   tttcaccagc gtttctgggt gagcaaaaac aggaaggcaa aatgccgcaa aaaagggaat 7920
   aagggcgaca cggaaatgtt gaatactcat actcttcctt tttcaatatt attgaagcat 7980
   ttatcagggt tattgtctca tgagcggata catatttgaa tgtatttaga aaaataaaca 8040
   aataggggtt ccgcgcacat ttccccgaaa agtgccacct gaacgaagca tctgtgcttc 8100
   attttgtaga acaaaaatgc aacgcgagag cgctaatttt tcaaacaaag aatctgagct 8160
   gcatttttac agaacagaaa tgcaacgcga aagcgctatt ttaccaacga agaatctgtg 8220
   cttcattttt gtaaaacaaa aatgcaacgc gagagcgcta atttttcaaa caaagaatct 8280
   gagctgcatt tttacagaac agaaatgcaa cgcgagagcg ctattttacc aacaaagaat 8340
   ctatacttct tttttgttct acaaaaatgc atcccgagag cgctattttt ctaacaaagc 8400
   atcttagatt actttttttc tcctttgtgc gctctataat gcagtctctt gataactttt 8460
   tgcactgtag gtccgttaag gttagaagaa ggctactttg gtgtctattt tctcttccat 8520
   aaaaaaagcc tgactccact tcccgcgttt actgattact agcgaagctg cgggtgcatt 8580
   ttttcaagat aaaggcatcc ccgattatat tctataccga tgtggattgc gcatactttg 8640
   tgaacagaaa gtgatagcgt tgatgattct tcattggtca gaaaattatg aacggtttct 8700
   tctattttgt ctctatatac tacgtatagg aaatgtttac attttcgtat tgttttcgat 8760
   tcactctatg aatagttctt actacaattt ttttgtctaa agagtaatac tagagataaa 8820
   cataaaaaat gtagaggtcg agtttagatg caagttcaag gagcgaaagg tggatgggta 8880
   ggttatatag ggatatagca cagagatata tagcaaagag atacttttga gcaatgtttg 8940
   tggaagcggt attcgcaata ttttagtagc tcgttacagt ccggtgcgtt tttggttttt 9000
   tgaaagtgcg tcttcagagc gcttttggtt ttcaaaagcg ctctgaagtt cctatacttt 9060
   ctagagaata ggaacttcgg aataggaact tcaaagcgtt tccgaaaacg agcgcttccg 9120
   aaaatgcaac gcgagctgcg cacatacagc tcactgttca cgtcgcacct atatctgcgt 9180
   gttgcctgta tatatatata catgagaaga acggcatagt gcgtgtttat gcttaaatgc 9240
   gtacttatat gcgtctattt atgtaggatg aaaggtagtc tagtacctcc tgtgatatta 9300
   tcccattcca tgcggggtat cgtatgcttc cttcagcact accctttagc tgttctatat 9360
   gctgccactc ctcaattgga ttagtctcat ccttcaatgc tatcatttcc tttgatattg 9420
   gatcatctaa gaaaccatta ttatcatgac attaacctat aaaaataggc gtatcacgag 9480
   gccctttcgt c 9491
<210> 57
   <211> 1000
   <212> DNA
   <213> Saccharymoces cerevisiae
<400> 57
   gttaattcaa attaattgat atagtttttt aatgagtatt gaatctgttt agaaataatg 60
   gaatattatt tttatttatt tatttatatt attggtcggc tcttttcttc tgaaggtcaa 120
   tgacaaaatg atatgaagga aataatgatt tctaaaattt tacaacgtaa gatattttta 180
   caaaagccta gctcatcttt tgtcatgcac tattttactc acgcttgaaa ttaacggcca 240
   gtccactgcg gagtcatttc aaagtcatcc taatcgatct atcgtttttg atagctcatt 300
   ttggagttcg cgattgtctt ctgttattca caactgtttt aatttttatt tcattctgga 360
   actcttcgag ttctttgtaa agtctttcat agtagcttac tttatcctcc aacatattta 420
   acttcatgtc aatttcggct cttaaatttt ccacatcatc aagttcaaca tcatctttta 480
   acttgaattt attctctagc tcttccaacc aagcctcatt gctccttgat ttactggtga 540
   aaagtgatac actttgcgcg caatccaggt caaaactttc ctgcaaagaa ttcaccaatt 600
   tctcgacatc atagtacaat ttgttttgtt ctcccatcac aatttaatat acctgatgga 660
   ttcttatgaa gcgctgggta atggacgtgt cactctactt cgcctttttc cctactcctt 720
   ttagtacgga agacaatgct aataaataag agggtaataa taatattatt aatcggcaaa 780
   aaagattaaa cgccaagcgt ttaattatca gaaagcaaac gtcgtaccaa tccttgaatg 840
   cttcccaatt gtatattaag agtcatcaca gcaacatatt cttgttatta aattaattat 900
   tattgatttt tgatattgta taaaaaaacc aaatatgtat aaaaaaagtg aataaaaaat 960
   accaagtatg gagaaatata ttagaagtct atacgttaaa 1000
<210> 58
   <211> 1713
   <212> DNA
   <213> Streptococcus mutans
<400> 58
   atgactgaca aaaaaactct taaagactta agaaatcgta gttctgttta cgattcaatg 60
   gttaaatcac ctaatcgtgc tatgttgcgt gcaactggta tgcaagatga agactttgaa 120
   aaacctatcg tcggtgtcat ttcaacttgg gctgaaaaca caccttgtaa tatccactta 180
   catgactttg gtaaactagc caaagtcggt gttaaggaag ctggtgcttg gccagttcag 240
   ttcggaacaa tcacggtttc tgatggaatc gccatgggaa cccaaggaat gcgtttctcc 300
   ttgacatctc gtgatattat tgcagattct attgaagcag ccatgggagg tcataatgcg 360
   gatgcttttg tagccattgg cggttgtgat aaaaacatgc ccggttctgt tatcgctatg 420
   gctaacatgg atatcccagc catttttgct tacggcggaa caattgcacc tggtaattta 480
   gacggcaaag atatcgattt agtctctgtc tttgaaggtg tcggccattg gaaccacggc 540
   gatatgacca aagaagaagt taaagctttg gaatgtaatg cttgtcccgg tcctggaggc 600
   tgcggtggta tgtatactgc taacacaatg gcgacagcta ttgaagtttt gggacttagc 660
   cttccgggtt catcttctca cccggctgaa tccgcagaaa agaaagcaga tattgaagaa 720
   gctggtcgcg ctgttgtcaa aatgctcgaa atgggcttaa aaccttctga cattttaacg 780
   cgtgaagctt ttgaagatgc tattactgta actatggctc tgggaggttc aaccaactca 840
   acccttcacc tcttagctat tgcccatgct gctaatgtgg aattgacact tgatgatttc 900
   aatactttcc aagaaaaagt tcctcatttg gctgatttga aaccttctgg tcaatatgta 960
   ttccaagacc tttacaaggt cggaggggta ccagcagtta tgaaatatct ccttaaaaat 1020
   ggcttccttc atggtgaccg tatcacttgt actggcaaaa cagtcgctga aaatttgaag 1080
   gcttttgatg atttaacacc tggtcaaaag gttattatgc cgcttgaaaa tcctaaacgt 1140
   gaagatggtc cgctcattat tctccatggt aacttggctc cagacggtgc cgttgccaaa 1200
   gtttctggtg taaaagtgcg tcgtcatgtc ggtcctgcta aggtctttaa ttctgaagaa 1260
   gaagccattg aagctgtctt gaatgatgat attgttgatg gtgatgttgt tgtcgtacgt 1320
   tttgtaggac caaagggcgg tcctggtatg cctgaaatgc tttccctttc atcaatgatt 1380
   gttggtaaag ggcaaggtga aaaagttgcc cttctgacag atggccgctt ctcaggtggt 1440
   acttatggtc ttgtcgtggg tcatatcgct cctgaagcac aagatggcgg tccaatcgcc 1500
   tacctgcaaa caggagacat agtcactatt gaccaagaca ctaaggaatt acactttgat 1560
   atctccgatg aagagttaaa acatcgtcaa gagaccattg aattgccacc gctctattca 1620
   cgcggtatcc ttggtaaata tgctcacatc gtttcgtctg cttctagggg agccgtaaca 1680
   gacttttgga agcctgaaga aactggcaaa aaa 1713
<210> 59
   <211> 571
   <212> PRT
   <213> streptococcus mutans
<400> 59
   Met Thr Asp Lys Lys Thr Leu Lys Asp Leu Arg Asn Arg Ser Ser Val
   1               5                   10                  15
   Tyr Asp Ser Met Val Lys Ser Pro Asn Arg Ala Met Leu Arg Ala Thr
               20                  25                  30
   Gly Met Gln Asp Glu Asp Phe Glu Lys Pro Ile Val Gly Val Ile Ser
           35                  40                  45
   Thr Trp Ala Glu Asn Thr Pro Cys Asn Ile His Leu His Asp Phe Gly
       50                  55                  60
   Lys Leu Ala Lys Val Gly Val Lys Glu Ala Gly Ala Trp Pro Val Gln
   65                  70                  75                  80
   Phe Gly Thr Ile Thr Val Ser Asp Gly Ile Ala Met Gly Thr Gln Gly
                   85                  90                  95
   Met Arg Phe Ser Leu Thr Ser Arg Asp Ile Ile Ala Asp Ser Ile Glu
               100                 105                 110
   Ala Ala Met Gly Gly His Asn Ala Asp Ala Phe Val Ala Ile Gly Gly
           115                 120                 125
   Cys Asp Lys Asn Met Pro Gly Ser Val Ile Ala Met Ala Asn Met Asp
       130                 135                 140
   Ile Pro Ala Ile Phe Ala Tyr Gly Gly Thr Ile Ala Pro Gly Asn Leu
   145                 150                 155                 160
   Asp Gly Lys Asp Ile Asp Leu Val Ser Val Phe Glu Gly Val Gly His
                   165                 170                 175
   Trp Asn His Gly Asp Met Thr Lys Glu Glu Val Lys Ala Leu Glu Cys
               180                 185                 190
   Asn Ala Cys Pro Gly Pro Gly Gly Cys Gly Gly Met Tyr Thr Ala Asn
           195                 200                 205
   Thr Met Ala Thr Ala Ile Glu Val Leu Gly Leu Ser Leu Pro Gly Ser
       210                 215                 220
   Ser Ser His Pro Ala Glu Ser Ala Glu Lys Lys Ala Asp Ile Glu Glu
   225                 230                 235                 240
   Ala Gly Arg Ala Val Val Lys Met Leu Glu Met Gly Leu Lys Pro Ser
                   245                 250                 255
   Asp Ile Leu Thr Arg Glu Ala Phe Glu Asp Ala Ile Thr Val Thr Met
               260                 265                 270
   Ala Leu Gly Gly Ser Thr Asn Ser Thr Leu His Leu Leu Ala Ile Ala
           275                 280                 285
   His Ala Ala Asn Val Glu Leu Thr Leu Asp Asp Phe Asn Thr Phe Gln
       290                 295                 300
   Glu Lys Val Pro His Leu Ala Asp Leu Lys Pro Ser Gly Gln Tyr Val
   305                 310                 315                 320
   Phe Gln Asp Leu Tyr Lys Val Gly Gly Val Pro Ala Val Met Lys Tyr
                   325                 330                 335
   Leu Leu Lys Asn Gly Phe Leu His Gly Asp Arg Ile Thr Cys Thr Gly
               340                 345                 350
   Lys Thr Val Ala Glu Asn Leu Lys Ala Phe Asp Asp Leu Thr Pro Gly
           355                 360                 365
   Gln Lys Val Ile Met Pro Leu Glu Asn Pro Lys Arg Glu Asp Gly Pro
       370                 375                 380
   Leu Ile Ile Leu His Gly Asn Leu Ala Pro Asp Gly Ala Val Ala Lys
   385                 390                 395                 400
   Val Ser Gly Val Lys Val Arg Arg His Val Gly Pro Ala Lys Val Phe
                   405                 410                 415
   Asn Ser Glu Glu Glu Ala Ile Glu Ala Val Leu Asn Asp Asp Ile Val
               420                 425                 430
   Asp Gly Asp Val Val Val Val Arg Phe Val Gly Pro Lys Gly Gly Pro
           435                 440                 445
   Gly Met Pro Glu Met Leu Ser Leu Ser Ser Met Ile Val Gly Lys Gly
       450                 455                 460
   Gln Gly Glu Lys Val Ala Leu Leu Thr Asp Gly Arg Phe Ser Gly Gly
   465                 470                 475                 480
   Thr Tyr Gly Leu Val Val Gly His Ile Ala Pro Glu Ala Gln Asp Gly
                   485                 490                 495
   Gly Pro Ile Ala Tyr Leu Gln Thr Gly Asp Ile Val Thr Ile Asp Gln
               500                 505                 510
   Asp Thr Lys Glu Leu His Phe Asp Ile Ser Asp Glu Glu Leu Lys His
           515                 520                 525
   Arg Gln Glu Thr Ile Glu Leu Pro Pro Leu Tyr Ser Arg Gly Ile Leu
       530                 535                 540
   Gly Lys Tyr Ala His Ile Val Ser Ser Ala Ser Arg Gly Ala Val Thr
   545                 550                 555                 560
   Asp Phe Trp Lys Pro Glu Glu Thr Gly Lys Lys
   565 570
<210> 60
   <211> 2145
   <212> DNA
   <213> artificial sequence
<220>
   <223> constructed chimeric gene
<400> 60
   gcatgcttgc atttagtcgt gcaatgtatg actttaagat ttgtgagcag gaagaaaagg 60
   gagaatcttc taacgataaa cccttgaaaa actgggtaga ctacgctatg ttgagttgct 120
   acgcaggctg cacaattaca cgagaatgct cccgcctagg atttaaggct aagggacgtg 180
   caatgcagac gacagatcta aatgaccgtg tcggtgaagt gttcgccaaa cttttcggtt 240
   aacacatgca gtgatgcacg cgcgatggtg ctaagttaca tatatatata tatatatata 300
   tatagccata gtgatgtcta agtaaccttt atggtatatt tcttaatgtg gaaagatact 360
   agcgcgcgca cccacacaca agcttcgtct tttcttgaag aaaagaggaa gctcgctaaa 420
   tgggattcca ctttccgttc cctgccagct gatggaaaaa ggttagtgga acgatgaaga 480
   ataaaaagag agatccactg aggtgaaatt tcagctgaca gcgagtttca tgatcgtgat 540
   gaacaatggt aacgagttgt ggctgttgcc agggagggtg gttctcaact tttaatgtat 600
   ggccaaatcg ctacttgggt ttgttatata acaaagaaga aataatgaac tgattctctt 660
   cctccttctt gtcctttctt aattctgttg taattacctt cctttgtaat tttttttgta 720
   attattcttc ttaataatcc aaacaaacac acatattaca atagctagct gaggatgaag 780
   gcattagttt atcatgggga tcacaaaatt tcgttagaag acaaaccaaa acccactctg 840
   cagaaaccaa cagacgttgt ggttagggtg ttgaaaacaa caatttgcgg tactgacttg 900
   ggaatataca aaggtaagaa tcctgaagtg gcagatggca gaatcctggg tcatgagggc 960
   gttggcgtca ttgaagaagt gggcgaatcc gtgacacaat tcaaaaaggg ggataaagtt 1020
   ttaatctcct gcgttactag ctgtggatcg tgtgattatt gcaagaagca actgtattca 1080
   cactgtagag acggtggctg gattttaggt tacatgatcg acggtgtcca agccgaatac 1140
   gtcagaatac cacatgctga caattcattg tataagatcc cgcaaactat cgatgatgaa 1200
   attgcagtac tactgtccga tattttacct actggacatg aaattggtgt tcaatatggt 1260
   aacgttcaac caggcgatgc tgtagcaatt gtaggagcag gtcctgttgg aatgtcagtt 1320
   ttgttaactg ctcaatttta ctcgcctagt accattattg ttatcgacat ggacgaaaac 1380
   cgtttacaat tagcgaagga gcttggggcc acacacacta ttaactccgg tactgaaaat 1440
   gttgtcgaag ctgtgcatcg tatagcagcc gaaggagtgg atgtagcaat agaagctgtt 1500
   ggtatacccg caacctggga catctgtcag gaaattgtaa aacccggcgc tcatattgcc 1560
   aacgtgggag ttcatggtgt taaggtggac tttgaaattc aaaagttgtg gattaagaat 1620
   ctaaccatca ccactggttt ggttaacact aatactaccc caatgttgat gaaggtagcc    1680
   tctactgata aattgccttt aaagaaaatg attactcaca ggtttgagtt agctgaaatc 1740
   gaacacgcat atcaggtttt cttgaatggc gctaaagaaa aagctatgaa gattattcta 1800
   tctaatgcag gtgccgccta attaattaag agtaagcgaa tttcttatga tttatgattt 1860
   ttattattaa ataagttata aaaaaaataa gtgtatacaa attttaaagt gactcttagg 1920
   ttttaaaacg aaaattctta ttcttgagta actctttcct gtaggtcagg ttgctttctc 1980
   aggtatagca tgaggtcgct cttattgacc acacctctac cggcatgccg agcaaatgcc 2040
   tgcaaatcgc tccccatttc acccaattgt agatatgcta actccagcaa tgagttgatg 2100
   aatctcggtg tgtattttat gtcctcagag gacaacacct gtggt 2145
<210> 61
   <211> 4280
   <212> DNA
   <213> artificial sequence
<220>
   <223> vector
<400> 61
   ggggatcctc tagagtcgac ctgcaggcat gcaagcttgg cgtaatcatg gtcatagctg 60
   tttcctgtgt gaaattgtta tccgctcaca attccacaca acatacgagc cggaagcata 120
   aagtgtaaag cctggggtgc ctaatgagtg agctaactca cattaattgc gttgcgctca 180
   ctgcccgctt tccagtcggg aaacctgtcg tgccagctgc attaatgaat cggccaacgc 240
   gcggggagag gcggtttgcg tattgggcgc tcttccgctt cctcgctcac tgactcgctg 300
   cgctcggtcg ttcggctgcg gcgagcggta tcagctcact caaaggcggt aatacggtta 360
   tccacagaat caggggataa cgcaggaaag aacatgtgag caaaaggcca gcaaaaggcc 420
   aggaaccgta aaaaggccgc gttgctggcg tttttccata ggctccgccc ccctgacgag 480
   catcacaaaa atcgacgctc aagtcagagg tggcgaaacc cgacaggact ataaagatac 540
   caggcgtttc cccctggaag ctccctcgtg cgctctcctg ttccgaccct gccgcttacc 600
   ggatacctgt ccgcctttct cccttcggga agcgtggcgc tttctcatag ctcacgctgt 660
   aggtatctca gttcggtgta ggtcgttcgc tccaagctgg gctgtgtgca cgaacccccc 720
   gttcagcccg accgctgcgc cttatccggt aactatcgtc ttgagtccaa cccggtaaga 780
   cacgacttat cgccactggc agcagccact ggtaacagga ttagcagagc gaggtatgta 840
   ggcggtgcta cagagttctt gaagtggtgg cctaactacg gctacactag aaggacagta 900
   tttggtatct gcgctctgct gaagccagtt accttcggaa aaagagttgg tagctcttga 960
   tccggcaaac aaaccaccgc tggtagcggt ggtttttttg tttgcaagca gcagattacg 1020
   cgcagaaaaa aaggatctca agaagatcct ttgatctttt ctacggggtc tgacgctcag 1080
   tggaacgaaa actcacgtta agggattttg gtcatgagat tatcaaaaag gatcttcacc 1140
   tagatccttt taaattaaaa atgaagtttt aaatcaatct aaagtatata tgagtaaact 1200
   tggtctgaca gttaccaatg cttaatcagt gaggcaccta tctcagcgat ctgtctattt 1260
   cgttcatcca tagttgcctg actccccgtc gtgtagataa ctacgatacg ggagggctta 1320
   ccatctggcc ccagtgctgc aatgataccg cgagacccac gctcaccggc tccagattta 1380
   tcagcaataa accagccagc cggaagggcc gagcgcagaa gtggtcctgc aactttatcc 1440
   gcctccatcc agtctattaa ttgttgccgg gaagctagag taagtagttc gccagttaat 1500
   agtttgcgca acgttgttgc cattgctaca ggcatcgtgg tgtcacgctc gtcgtttggt 1560
   atggcttcat tcagctccgg ttcccaacga tcaaggcgag ttacatgatc ccccatgttg 1620
   tgcaaaaaag cggttagctc cttcggtcct ccgatcgttg tcagaagtaa gttggccgca 1680
   gtgttatcac tcatggttat ggcagcactg cataattctc ttactgtcat gccatccgta 1740
   agatgctttt ctgtgactgg tgagtactca accaagtcat tctgagaata gtgtatgcgg 1800
   cgaccgagtt gctcttgccc ggcgtcaata cgggataata ccgcgccaca tagcagaact 1860
   ttaaaagtgc tcatcattgg aaaacgttct tcggggcgaa aactctcaag gatcttaccg 1920
   ctgttgagat ccagttcgat gtaacccact cgtgcaccca actgatcttc agcatctttt 1980
   actttcacca gcgtttctgg gtgagcaaaa acaggaaggc aaaatgccgc aaaaaaggga 2040
   ataagggcga cacggaaatg ttgaatactc atactcttcc tttttcaata ttattgaagc 2100
   atttatcagg gttattgtct catgagcgga tacatatttg aatgtattta gaaaaataaa 2160
   caaatagggg ttccgcgcac atttccccga aaagtgccac ctgacgtcta agaaaccatt 2220
   attatcatga cattaaccta taaaaatagg cgtatcacga ggccctttcg tctcgcgcgt 2280
   ttcggtgatg acggtgaaaa cctctgacac atgcagctcc cggagacggt cacagcttgt 2340
   ctgtaagcgg atgccgggag cagacaagcc cgtcagggcg cgtcagcggg tgttggcggg 2400
   tgtcggggct ggcttaacta tgcggcatca gagcagattg tactgagagt gcaccatatg 2460
   cggtgtgaaa taccgcacag atgcgtaagg agaaaatacc gcatcaggcg ccattcgcca 2520
   ttcaggctgc gcaactgttg ggaagggcga tcggtgcggg cctcttcgct attacgccag 2580
   ctggcgaaag ggggatgtgc tgcaaggcga ttaagttggg taacgccagg gttttcccag 2640
   tcacgacgtt gtaaaacgac ggccagtgaa ttcgagctcg gtacccccgg ctctgagaca 2700
   gtagtaggtt agtcatcgct ctaccgacgc gcaggaaaag aaagaagcat tgcggattac 2760
   gtattctaat gttcagcccg cggaacgcca gcaaatcacc acccatgcgc atgatactga 2820
   gtcttgtaca cgctgggctt ccagtgtact gagagtgcac cataccacag cttttcaatt 2880
   caattcatca tttttttttt attctttttt ttgatttcgg tttctttgaa atttttttga 2940
   ttcggtaatc tccgaacaga aggaagaacg aaggaaggag cacagactta gattggtata 3000
   tatacgcata tgtagtgttg aagaaacatg aaattgccca gtattcttaa cccaactgca 3060
   cagaacaaaa acctgcagga aacgaagata aatcatgtcg aaagctacat ataaggaacg 3120
   tgctgctact catcctagtc ctgttgctgc caagctattt aatatcatgc acgaaaagca 3180
   aacaaacttg tgtgcttcat tggatgttcg taccaccaag gaattactgg agttagttga 3240
   agcattaggt cccaaaattt gtttactaaa aacacatgtg gatatcttga ctgatttttc 3300
   catggagggc acagttaagc cgctaaaggc attatccgcc aagtacaatt ttttactctt 3360
   cgaagacaga aaatttgctg acattggtaa tacagtcaaa ttgcagtact ctgcgggtgt 3420
   atacagaata gcagaatggg cagacattac gaatgcacac ggtgtggtgg gcccaggtat 3480
   tgttagcggt ttgaagcagg cggcagaaga agtaacaaag gaacctagag gccttttgat 3540
   gttagcagaa ttgtcatgca agggctccct atctactgga gaatatacta agggtactgt 3600
   tgacattgcg aagagcgaca aagattttgt tatcggcttt attgctcaaa gagacatggg 3660
   tggaagagat gaaggttacg attggttgat tatgacaccc ggtgtgggtt tagatgacaa 3720
   gggagacgca ttgggtcaac agtatagaac cgtggatgat gtggtctcta caggatctga 3780
   cattattatt gttggaagag gactatttgc aaagggaagg gatgctaagg tagagggtga 3840
   acgttacaga aaagcaggct gggaagcata tttgagaaga tgcggccagc aaaactaaaa 3900
   aactgtatta taagtaaatg catgtatact aaactcacaa attagagctt caatttaatt 3960
   atatcagtta ttaccctatg cggtgtgaaa taccgcacag atgcgtaagg agaaaatacc 4020
   gcatcaggaa attgtaaacg ttaatatttt gttaaaattc gcgttaaatt tttgttaaat 4080
   cagctcattt tttaaccaat aggccgaaat cggcaaaatc ttcagcccgc ggaacgccag 4140
   caaatcacca cccatgcgca tgatactgag tcttgtacac gctgggcttc cagtgatgat 4200
   acaacgagtt agccaaggtg agcacggatg tctaaattag aattacgttt taatatcttt 4260
   ttttccatat ctagggctag 4280
<210> 62
   <211> 30
   <212> DNA
   <213> artificial sequence
<220>
   <223> primer
<400> 62
   gcatgcttgc atttagtcgt gcaatgtatg 30
<210> 63
   <211> 54
   <212> DNA
   <213> artificial sequence
<220>
   <223> primer
<400> 63
   gaacattaga atacgtaatc cgcaatgcac tagtaccaca ggtgttgtcc tctg 54
<210> 64
   <211> 54
   <212> DNA
   <213> artificial sequence
<220>
   <223> primer
<400> 64
   cagaggacaa cacctgtggt actagtgcat tgcggattac gtattctaat gttc 54
<210> 65
   <211> 28
   <212> DNA
   <213> artificial sequence
<220>
   <223> primer
<400> 65
   caccttggct aactcgttgt atcatcac 28
<210> 66
   <211> 100
   <212> DNA
   <213> artificial sequence
<220>
   <223> primer
<400> 66
   ttttaagccg aatgagtgac agaaaaagcc cacaacttat caagtgatat tgaacaaagg 60
   gcgaaacttc gcatgcttgc atttagtcgt gcaatgtatg 100
<210> 67
   <211> 98
   <212> DNA
   <213> artificial sequence
<220>
   <223> primer
<400> 67
   cccaattggt aaatattcaa caagagacgc gcagtacgta acatgcgaat tgcgtaattc 60
   acggcgataa caccttggct aactcgttgt atcatcac 98
<210> 68
   <211> 29
   <212> DNA
   <213> artificial sequence
<220>
   <223> primer
<400> 68
   caaaagccca tgtcccacac caaaggatg 29
<210> 69
   <211> 26
   <212> DNA
   <213> artificial sequence
<220>
   <223> primer
<400> 69
   caccatcgcg cgtgcatcac tgcatg 26
<210> 70
   <211> 28
   <212> DNA
   <213> artificial sequence
<220>
   <223> primer
<400> 70
   tcggtttttg caatatgacc tgtgggcc 28
<210> 71
   <211> 22
   <212> DNA
   <213> artificial sequence
<220>
   <223> primer
<400> 71
   gagaagatgc ggccagcaaa ac 22
<210> 72
   <211> 2745
   <212> DNA
   <213> artificial sequence
<220>
   <223> constructed coding region-terminator segment
<400> 72
   atgactgaca aaaaaactct taaagactta agaaatcgta gttctgttta cgattcaatg     60
   gttaaatcac ctaatcgtgc tatgttgcgt gcaactggta tgcaagatga agactttgaa    120
   aaacctatcg tcggtgtcat ttcaacttgg gctgaaaaca caccttgtaa tatccactta    180
   catgactttg gtaaactagc caaagtcggt gttaaggaag ctggtgcttg gccagttcag    240
   ttcggaacaa tcacggtttc tgatggaatc gccatgggaa cccaaggaat gcgtttctcc    300
   ttgacatctc gtgatattat tgcagattct attgaagcag ccatgggagg tcataatgcg    360
   gatgcttttg tagccattgg cggttgtgat aaaaacatgc ccggttctgt tatcgctatg    420
   gctaacatgg atatcccagc catttttgct tacggcggaa caattgcacc tggtaattta    480
   gacggcaaag atatcgattt agtctctgtc tttgaaggtg tcggccattg gaaccacggc    540
   gatatgacca aagaagaagt taaagctttg gaatgtaatg cttgtcccgg tcctggaggc    600
   tgcggtggta tgtatactgc taacacaatg gcgacagcta ttgaagtttt gggacttagc    660
   cttccgggtt catcttctca cccggctgaa tccgcagaaa agaaagcaga tattgaagaa    720
   gctggtcgcg ctgttgtcaa aatgctcgaa atgggcttaa aaccttctga cattttaacg    780
   cgtgaagctt ttgaagatgc tattactgta actatggctc tgggaggttc aaccaactca    840
   acccttcacc tcttagctat tgcccatgct gctaatgtgg aattgacact tgatgatttc    900
   aatactttcc aagaaaaagt tcctcatttg gctgatttga aaccttctgg tcaatatgta    960
   ttccaagacc tttacaaggt cggaggggta ccagcagtta tgaaatatct ccttaaaaat   1020
   ggcttccttc atggtgaccg tatcacttgt actggcaaaa cagtcgctga aaatttgaag   1080
   gcttttgatg atttaacacc tggtcaaaag gttattatgc cgcttgaaaa tcctaaacgt 1140
   gaagatggtc cgctcattat tctccatggt aacttggctc cagacggtgc cgttgccaaa 1200
   gtttctggtg taaaagtgcg tcgtcatgtc ggtcctgcta aggtctttaa ttctgaagaa 1260
   gaagccattg aagctgtctt gaatgatgat attgttgatg gtgatgttgt tgtcgtacgt 1320
   tttgtaggac caaagggcgg tcctggtatg cctgaaatgc tttccctttc atcaatgatt 1380
   gttggtaaag ggcaaggtga aaaagttgcc cttctgacag atggccgctt ctcaggtggt 1440
   acttatggtc ttgtcgtggg tcatatcgct cctgaagcac aagatggcgg tccaatcgcc 1500
   tacctgcaaa caggagacat agtcactatt gaccaagaca ctaaggaatt acactttgat 1560
   atctccgatg aagagttaaa acatcgtcaa gagaccattg aattgccacc gctctattca 1620
   cgcggtatcc ttggtaaata tgctcacatc gtttcgtctg cttctagggg agccgtaaca 1680
   gacttttgga agcctgaaga aactggcaaa aaatgttgtc ctggttgctg tggttaagcg 1740
   gccgcgttaa ttcaaattaa ttgatatagt tttttaatga gtattgaatc tgtttagaaa 1800
   taatggaata ttatttttat ttatttattt atattattgg tcggctcttt tcttctgaag 1860
   gtcaatgaca aaatgatatg aaggaaataa tgatttctaa aattttacaa cgtaagatat 1920
   ttttacaaaa gcctagctca tcttttgtca tgcactattt tactcacgct tgaaattaac 1980
   ggccagtcca ctgcggagtc atttcaaagt catcctaatc gatctatcgt ttttgatagc 2040
   tcattttgga gttcgcgatt gtcttctgtt attcacaact gttttaattt ttatttcatt 2100
   ctggaactct tcgagttctt tgtaaagtct ttcatagtag cttactttat cctccaacat 2160
   atttaacttc atgtcaattt cggctcttaa attttccaca tcatcaagtt caacatcatc 2220
   ttttaacttg aatttattct ctagctcttc caaccaagcc tcattgctcc ttgatttact 2280
   ggtgaaaagt gatacacttt gcgcgcaatc caggtcaaaa ctttcctgca aagaattcac 2340
   caatttctcg acatcatagt acaatttgtt ttgttctccc atcacaattt aatatacctg 2400
   atggattctt atgaagcgct gggtaatgga cgtgtcactc tacttcgcct ttttccctac 2460
   tccttttagt acggaagaca atgctaataa ataagagggt aataataata ttattaatcg 2520
   gcaaaaaaga ttaaacgcca agcgtttaat tatcagaaag caaacgtcgt accaatcctt 2580
   gaatgcttcc caattgtata ttaagagtca tcacagcaac atattcttgt tattaaatta 2640
   attattattg atttttgata ttgtataaaa aaaccaaata tgtataaaaa aagtgaataa 2700
   aaaataccaa gtatggagaa atatattaga agtctatacg ttaaa 2745
<210> 73
   <211> 99
   <212> DNA
   <213> artificial sequence
<220>
   <223> primer
<400> 73
   tcctttctca attattattt tctactcata acctcacgca aaataacaca gtcaaatcaa 60
   tcaaagtatg actgacaaaa aaactcttaa agacttaag 99
<210> 74
   <211> 77
   <212> DNA
   <213> artificial sequence
<220>
   <223> primer
<400> 74
   gaacattaga atacgtaatc cgcaatgctt ctttcttttc cgtttaacgt atagacttct 60
   aatatatttc tccatac 77
<210> 75
   <211> 45
   <212> DNA
   <213> artificial sequence
<220>
   <223> primer
<400> 75
   aaacggaaaa gaaagaagca ttgcggatta cgtattctaa tgttc 45
<210> 76
   <211> 88
   <212> DNA
   <213> artificial sequence
<220>
   <223> primer
<400> 76
   tatttttcgt tacataaaaa tgcttataaa actttaacta ataattagag attaaatcgc 60
   caccttggct aactcgttgt atcatcac 88
<210> 77
   <211> 27
   <212> DNA
   <213> artificial sequence
<220>
   <223> primer
<400> 77
   gacttttgga agcctgaaga aactggc 27
<210> 78
   <211> 20
   <212> DNA
   <213> artificial sequence
<220>
   <223> primer
<400> 78
   cttggcagca acaggactag 20
<210> 79
   <211> 26
   <212> DNA
   <213> artificial sequence
<220>
   <223> primer
<400> 79
   ccaggccaat tcaacagact gtcggc 26
<210> 80
   <211> 26
   <212> DNA
   <213> artificial sequence
<220>
   <223> primer
<400> 80
   gacttgaata atgcagcggc gcttgc 26
<210> 81
   <211> 30
   <212> DNA
   <213> artificial sequence
<220>
   <223> primer
<400> 81
   ccaccctctt caattagcta agatcatagc 30
<210> 82
   <211> 25
   <212> DNA
   <213> artificial sequence
<220>
   <223> primer
<400> 82
   aaaaattgat tctcatcgta aatgc 25
<210> 83
   <211> 20
   <212> DNA
   <213> artificial sequence
<220>
   <223> primer
<400> 83
   ctgcagcgag gagccgtaat 20
<210> 84
   <211> 2347
   <212> DNA
   <213> artificial sequence
<220>
   <223> constructed URSA3 marker with flanking homologous repeat sequences for HIS gene replacement and marker excision
<400> 84
   gcattgcgga ttacgtattc taatgttcag gtgctggaag aagagctgct taaccgccgc    60
   gcccagggtg aagatccacg ctactttacc ctgcgtcgtc tggatttcgg cggctgtcgt   120
   ctttcgctgg caacgccggt tgatgaagcc tgggacggtc cgctctcctt aaacggtaaa   180
   cgtatcgcca cctcttatcc tcacctgctc aagcgttatc tcgaccagaa aggcatctct   240
   tttaaatcct gcttactgaa cggttctgtt gaagtcgccc cgcgtgccgg actggcggat   300
   gcgatttgcg atctggtttc caccggtgcc acgctggaag ctaacggcct gcgcgaagtc   360
   gaagttatct atcgctcgaa agcctgcctg attcaacgcg atggcgaaat ggaagaatcc   420
   aaacagcaac tgatcgacaa actgctgacc cgtattcagg gtgtgatcca ggcgcgcgaa   480
   tcaaaataca tcatgatgca cgcaccgacc gaacgtctgg atgaagtcat ggtacctact   540
   gagagtgcac cataccacag cttttcaatt caattcatca tttttttttt attctttttt   600
   ttgatttcgg tttctttgaa atttttttga ttcggtaatc tccgaacaga aggaagaacg   660
   aaggaaggag cacagactta gattggtata tatacgcata tgtagtgttg aagaaacatg   720
   aaattgccca gtattcttaa cccaactgca cagaacaaaa acctgcagga aacgaagata   780
   aatcatgtcg aaagctacat ataaggaacg tgctgctact catcctagtc ctgttgctgc   840
   caagctattt aatatcatgc acgaaaagca aacaaacttg tgtgcttcat tggatgttcg   900
   taccaccaag gaattactgg agttagttga agcattaggt cccaaaattt gtttactaaa   960
   aacacatgtg gatatcttga ctgatttttc catggagggc acagttaagc cgctaaaggc  1020
   attatccgcc aagtacaatt ttttactctt cgaagacaga aaatttgctg acattggtaa  1080
   tacagtcaaa ttgcagtact ctgcgggtgt atacagaata gcagaatggg cagacattac  1140
   gaatgcacac ggtgtggtgg gcccaggtat tgttagcggt ttgaagcagg cggcagaaga  1200
   agtaacaaag gaacctagag gccttttgat gttagcagaa ttgtcatgca agggctccct  1260
   atctactgga gaatatacta agggtactgt tgacattgcg aagagcgaca aagattttgt  1320
   tatcggcttt attgctcaaa gagacatggg tggaagagat gaaggttacg attggttgat  1380
   tatgacaccc ggtgtgggtt tagatgacaa gggagacgca ttgggtcaac agtatagaac  1440
   cgtggatgat gtggtctcta caggatctga cattattatt gttggaagag gactatttgc  1500
   aaagggaagg gatgctaagg tagagggtga acgttacaga aaagcaggct gggaagcata  1560
   tttgagaaga tgcggccagc aaaactaaaa aactgtatta taagtaaatg catgtatact  1620
   aaactcacaa attagagctt caatttaatt atatcagtta ttaccctatg cggtgtgaaa  1680
   taccgcacag atgcgtaagg agaaaatacc gcatcaggaa attgtaaacg ttaatatttt  1740
   gttaaaattc gcgttaaatt tttgttaaat cagctcattt tttaaccaat aggccgaaat  1800
   cggcaaaatc tctagagtgc tggaagaaga gctgcttaac cgccgcgccc agggtgaaga  1860
   tccacgctac tttaccctgc gtcgtctgga tttcggcggc tgtcgtcttt cgctggcaac  1920
   gccggttgat gaagcctggg acggtccgct ctccttaaac ggtaaacgta tcgccacctc  1980
   ttatcctcac ctgctcaagc gttatctcga ccagaaaggc atctctttta aatcctgctt 2040
   actgaacggt tctgttgaag tcgccccgcg tgccggactg gcggatgcga tttgcgatct 2100
   ggtttccacc ggtgccacgc tggaagctaa cggcctgcgc gaagtcgaag ttatctatcg 2160
   ctcgaaagcc tgcctgattc aacgcgatgg cgaaatggaa gaatccaaac agcaactgat 2220
   cgacaaactg ctgacccgta ttcagggtgt gatccaggcg cgcgaatcaa aatacatcat 2280
   gatgcacgca ccgaccgaac gtctggatga agtcatccag tgatgataca acgagttagc 2340
   caaggtg 2347
<210> 85
   <211> 80
   <212> DNA
   <213> artificial sequence
<220>
   <223> primer
<400> 85
   cttcgaagaa tatactaaaa aatgagcagg caagataaac gaaggcaaag gcattgcgga 60
   ttacgtattc taatgttcag 80
<210> 86
   <211> 80
   <212> DNA
   <213> artificial sequence
<220>
   <223> primer
<400> 86
   cttcgaagaa tatactaaaa aatgagcagg caagataaac gaaggcaaag gcattgcgga 60
   ttacgtattc taatgttcag 80

## Description

### FIELD OF THE INVENTION

The invention relates to the field of biofuels. More specifically, the invention relates to a method for producing butanol through microbial fermentation, in which gas is passed through the fermentation medium to form a butanol containing gas phase and the butanol product is removed by extraction into a water immiscible organic extractant during the fermentation and by recovering butanol from the butanol containing gas phase.

### BACKGROUND OF THE INVENTION

Butanol is an important industrial chemical, with a variety of applications, such as use as a fuel additive, as a feedstock chemical in the plastics industry, and as a foodgrade extractant in the food and flavor industry. Each year 10 to12 billion pounds of butanol are produced by petrochemical means and the need for this chemical will likely increase.

Several chemical synthetic methods are known; however, these methods of producing butanol use starting materials derived from petrochemicals and are generally expensive and are not environmentally friendly. Several methods of producing butanol by fermentation are also known, for example the ABE process which is the fermentive process producing a mixture of acetone, 1-butanol and ethanol. Acetone-butanol-ethanol (ABE) fermentation by *Clostridium acetobutylicum* is one of the oldest known industrial fermentations; as is also the pathways and genes responsible for the production of these solvents. Production of 1-butanol by the ABE process is limited by the toxic effect of the 1-butanol on *Clostridium acetobutylicum. In situ* extractive fermentation methods using specific organic extractants which are nontoxic to the bacterium have been reported to enhance the production of 1-butanol by fermentation using *Clostridium acetobutylicum* (Roffler et al Biotechnol. Bioeng. 31:135-143, 1988; Roffler et al., Bioprocess Engineering 2:1-12, 1987; and Evans et al., Appl. Environ. Microbiol. 54:1662-1667, 1988).

Davison and Thompson (Applied Biochemistry and Biotechnology, 1993, 39/40, pp 415-26) relates to the continuous direct solvent extraction of butanol in a fermenting fluidized-bed bioreactor with immobilized *Clostridium acetobutylicum.* Ezeji et al (Cur. Op. Biotechnol., 2007, 18, pp 220-7) in "Bioproduction of butanol from biomass: from genes to bioreactors" list gas stripping as a technique that can be applied for in situ butanol recovery. Egan et al (Ind. Eng. Chem. Res., 1988, 27, pp1330-2 relates to the solvent extraction and the recovery of ethanol from aqueous solutions.

In contrast to the native *Clostridium acetobutylicum* described above, recombinant microbial production hosts expressing 1-butanol, 2-butanol, and isobutanol biosynthetic pathways have also been described. These recombinant hosts have the potential of producing butanol in higher yields compared to the ABE process because they do not produce byproducts such as acetone and ethanol. However, the problem with these recombinant hosts is that biological production of butanol appears to be limited by butanol toxicity thresholds to the host microorganism used in the fermentation. Extractive fermentation methods have not been applied to the production of butanols using recombinant microbial strains.

The present invention satisfies the above need and provides a method of making butanol from at least one fermentable carbon source that overcome the issues of toxicity resulting in an increase in the effective titer, the effective rate, and the effective yield of butanol production by fermentation utilizing a recombinant microbial host wherein the butanol is extracted into specific organic extractants during fermentation and wherein gas is passed through the fermentation medium.

### SUMMARY OF THE INVENTION

Embodiments of the present invention are defined in the claims. In particular, the present invention relates to a method for recovering butanol from a fermentation medium, the method comprising:
a) providing a fermentation medium comprising butanol, water, and a genetically modified microorganism that produces butanol from a fermentation medium comprising at least one fermentable carbon source;
b) passing a gas through the fermentation medium to form a butanol-containing gas phase;
c) contacting the fermentation medium with i) at least one first water immiscible organic extractant selected from the group consisting of C₁₂ to C₂₂ fatty alcohols, C₁₂ to C₂₂ fatty acids, esters of C₁₂ to C₂₂ fatty acids, C₁₂ to C₂₂ fatty aldehydes, and mixtures thereof, and optionally (ii) a second water immiscible organic extractant selected from the group consisting of C₁₂ to C₁₂ fatty alcohols, C₁₂ to C₁₂ fatty acids, esters of C₁₂ to C₂₂ fatty acids, C₁₂ to C₂₂ fatty aldehydes, and mixtures thereof to form a two-phase mixture comprising an aqueous phase and a butanol-containing organic phase;
d) separating the butanol-containing organic phase from the aqueous phase; and
e) recovering the butanol from the butanol-containing organic phase and the butanol-containing gas phase to produce recovered butanol.

The present invention also relates to a method for the production of butanol comprising the steps of:
a) providing a genetically modified microorganism that produces butanol from a fermentation medium comprising at least one fermentable carbon source;
b) passing a gas through the fermentation medium to form a butanol-containing gas phase;
c) growing the microorganism in a biphasic fermentation medium comprising an aqueous phase and a water immiscible organic extractant selected from the group consisting of C₁₂ to C₂₂ fatty alcohols, C₁₂ to C₂₂ fatty acids, esters of C₁₂ to C₂₂ fatty acids, C₁₂ to C₂₂ fatty aldehydes, and mixtures thereof, wherein said biphasic fermentation medium comprises from about 3% to about 60% by volume of said water immiscible organic extractant, for a time sufficient to allow extraction of the butanol into the organic extractant to form a butanol-containing organic phase;
d) separating the butanol-containing organic phase from the aqueous phase; and
e) recovering the butanol from the butanol-containing organic phase and the butanol-containing gas phase to produce recovered butanol.

The present invention also relates to a method for the production of butanol comprising the steps of:
a) providing a genetically modified microorganism that produces butanol from a fermentation medium comprising at least one fermentable carbon source;
b) growing the microorganism in a fermentation medium wherein the microorganism produces said butanol into the fermentation medium to produce a butanol-containing fermentation medium
c) passing a gas through the fermentation medium to form a butanol-containing gas phase;
d) contacting the butanol-containing fermentation medium with i) at least one first water immiscible organic extractant selected from the group consisting of C₁₂ to C₂₂ fatty alcohols, C₁₂ to C₂₂ fatty acids, esters of C₁₂ to C₂₂ fatty acids, C₁₂ to C₂₂ fatty aldehydes, and mixtures thereof, and optionally (ii) a second water immiscible organic extractant selected from the group consisting of C₁₂ to C₂₂ fatty alcohols, C₁₂ to C₂₂ fatty acids, esters of C₁₂ to C₂₂ fatty acids, C₁₂ to C₂₂ fatty aldehydes, and mixtures thereof to form a two-phase mixture comprising an aqueous phase and a butanol-containing organic phase;
e) separating the butanol-containing organic phase from the aqueous phase; and
f) recovering the butanol from the butanol-containing organic phase and the butanol-containing gas phase.

The present invention also relates to a method for the production of butanol comprising the steps of:
a) providing a genetically modified microorganism that produces butanol from a fermentation medium comprising at least one fermentable carbon source;
b) growing the microorganism in a fermentation medium under aerobic conditions for a time sufficient to reach a preselected growth level;
c) switching to microaerobic or anaerobic conditions to stimulate butanol production into the fermentation medium to form a butanol-containing fermentation medium;
d) passing a gas through the fermentation medium to form a butanol-containing gas phase;
e) contacting the butanol-containing fermentation medium with i) at least one first water immiscible organic extractant selected from the group consisting of C₁₂ to C₂₂ fatty alcohols, C₁₂ to C₂₂ fatty acids, esters of C₁₂ to C₂₂ fatty acids, C₁₂ to C₂₂ fatty aldehydes, and mixtures thereof, and optionally (ii) a second water immiscible organic extractant selected from the group consisting of C₁₂ to C₂₂ fatty alcohols, C₁₂ to C₂₂ fatty acids, esters of C₁₂ to C₂₂ fatty acids, C₁₂ to C₂₂ fatty aldehydes, and mixtures thereof to form a two-phase mixture comprising an aqueous phase and a butanol-containing organic phase;
f) separating the butanol-containing organic phase from the aqueous phase; and
g) recovering the butanol from the butanol-containing organic phase and the butanol-containing gas phase.

The present invention also relates to a method for the production of butanol comprising the steps of:
a) providing a fermentation medium comprising butanol, water, and a genetically modified microorganism that produces butanol from a fermentation medium comprising at least one fermentable carbon source;
b) passing a gas through the fermentation medium to form a butanol-containing gas phase;
c) contacting the fermentation medium via a co-current or countercurrent extractant stream with i) at least one first water immiscible organic extractant selected from the group consisting of C₁₂ to C₂₂ fatty alcohols, C₁₂ to C₂₂ fatty acids, esters of C₁₂ to C₂₂ fatty acids, C₁₂ to C₁₂ fatty aldehydes, and mixtures thereof, and optionally (ii) a second water immiscible organic extractant selected from the group consisting of C₁₂ to C₂₂ fatty alcohols, C₁₂ to C₂₂ fatty acids, esters of C₁₂ to C₂₂ fatty acids, C₁₂ to C₂₂ fatty aldehydes, and mixtures thereof to form a two-phase mixture comprising an aqueous phase and a butanol-containing organic phase;
d) separating the butanol-containing organic phase from the aqueous phase; and
e) recovering the butanol from the butanol-containing organic phase and the butanol-containing gas phase to produce recovered butanol.

Preferably, the organic extractant is selected from the group consisting of oleyl alcohol, behenyl alcohol, cetyl alcohol, lauryl alcohol, myristyl alcohol, stearyl alcohol, 1-undecanol, oleic acid, lauric acid, myristic acid, stearic acid, methyl myristate, methyl oleate, undecanal, lauric aldehyde, 2-methylundecanal, and mixtures thereof, more preferably the organic extractant comprises oleyl alcohol.

Preferably the recovered butanol has an effective titer of at least about 37 g per liter of the aqueous phase. Preferably the fermentation medium further comprises ethanol, and the butanol-containing organic phase contains ethanol. Preferably the butanol is isobutanol.

Preferably the genetically modified microorganism is selected from the group consisting of bacteria, cyanobacteria, filamentous fungi and yeasts, such as *Zymomonas, Escherichia, Salmonella, Rhodococcus, Pseudomonas, Bacillus, Lactobacillus, Enterococcus, Pediococcus, Alcaligenes, Klebsiella, Paenibacillus, Arthrobacter,Corynebacterium, and Brevibacterium, Pichia, Candida, Hansenula* and *Saccharomyces.*

The present extractive fermentation methods provide butanol, including all butanol isomers, which is known to have an energy content similar to that of gasoline and which can be blended with any fossil fuel. Butanol is favored as a fuel or fuel additive as it yields only CO₂ and little or no SOₓ or NOₓ when burned in the standard internal combustion engine. Additionally butanol is less corrosive than ethanol, the most preferred fuel additive to date.

In addition to its utility as a biofuel or fuel additive, the butanol produced from the present methods has the potential of impacting hydrogen distribution problems in the emerging fuel cell industry. Fuel cells today are plagued by safety concerns associated with hydrogen transport and distribution. Butanol can be easily reformed for its hydrogen content and can be distributed through existing gas stations in the purity required for either fuel cells or vehicles.

Finally, the present methods produce butanol from plant derived carbon sources, avoiding the negative environmental impact associated with standard petrochemical processes for butanol production.

### BRIEF DESCRIPTION OF THE FIGURE AND SEQUENCE

### DESCRIPTIONS

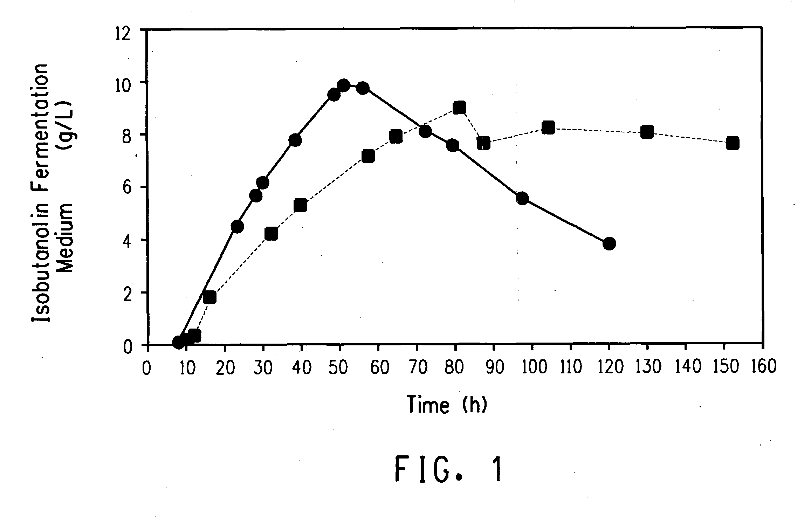

**Figure 1** is a graph showing the concentration of isobutanol in the fermentation medium (i.e., aqueous phase) during a fermentation using oleyl alcohol as the organic extractant with gas stripping (■) as described in Example 6, and during a fermentation with gas stripping alone (●), as described in Example 7. Figure 1 represents data generated using a recombinant *Escherichia coli* producing isobutanol.

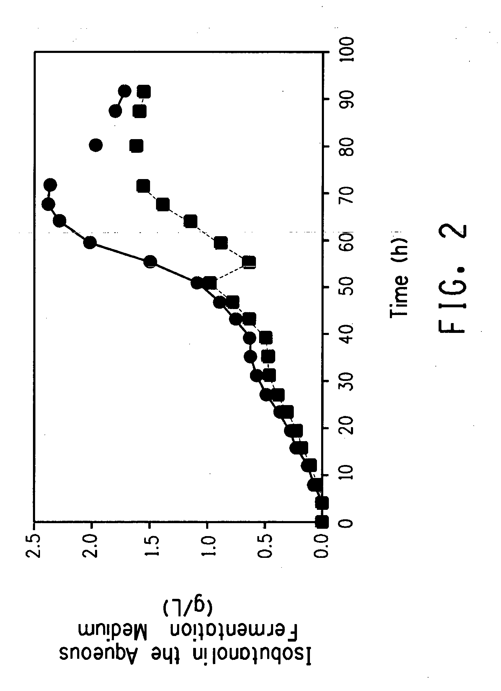

**Figure 2** is a graph showing the concentration of isobutanol in the fermentation medium (i.e., aqueous phase) during a fermentation using oleyl alcohol as the organic extractant with gas stripping (■) as described in Example 8, and during a fermentation with gas stripping alone (●), as described in Example 9. Figure 2 represents data generated using a recombinant *Saccharomyces cerevisiae* producing isobutanol.

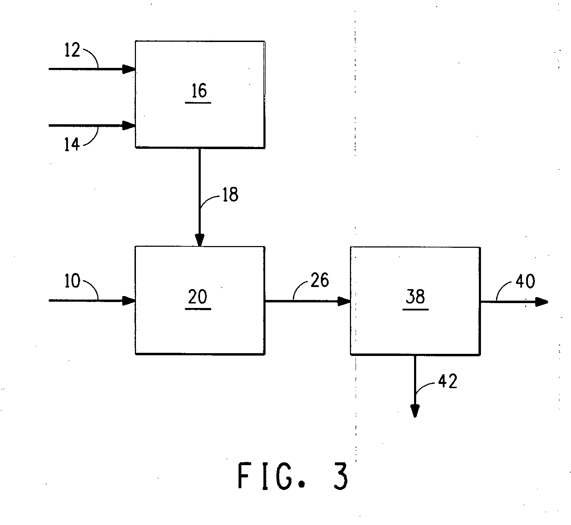

**Figure 3** schematically illustrates one example of the methods disclosed herein, in which the first water immiscible extractant and the optional second water immiscible extractant are combined in a vessel prior to contacting the fermentation medium with the extractant in a fermentation vessel.

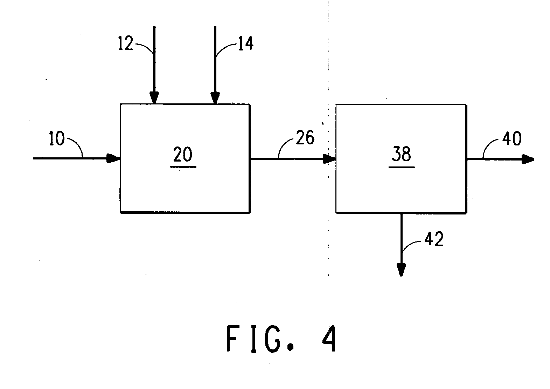

**Figure 4** schematically illustrates one example of the methods disclosed herein, in which the first water immiscible extractant and the optional second water immiscible extractant are added separately to a fermentation vessel in which the fermentation medium is contacted with the extractant.

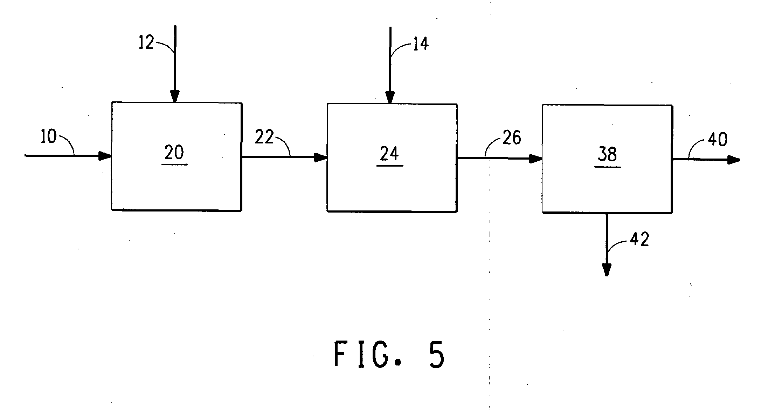

**Figure 5** schematically illustrates one example of the methods disclosed herein, in which the first water immiscible extractant and the optional second water immiscible extractant are added separately to different fermentation vessels for contacting of the fermentation medium with the extractant.

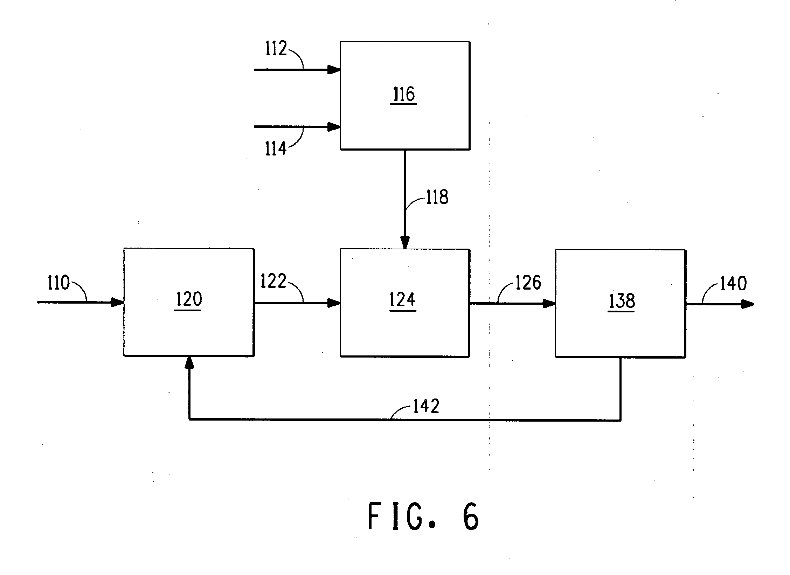

**Figure 6** schematically illustrates one example of the methods disclosed herein, in which extraction of the product occurs downstream of the fermentor and the first water immiscible extractant and the optional second water immiscible extractant are combined in a vessel prior to contacting the fermentation medium with the extractant in a different vessel.

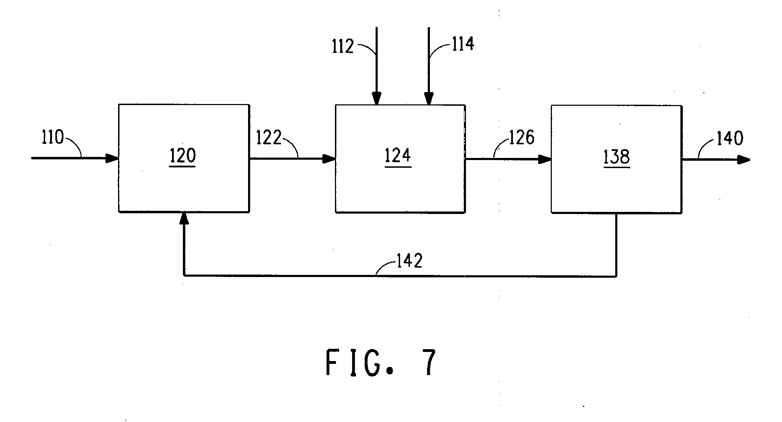

**Figure 7** schematically illustrates one example of the methods disclosed herein, in which extraction of the product occurs downstream of the fermentor and the first water immiscible extractant and the optional second water immiscible extractant are added separately to a vessel in which the fermentation medium is contacted with the extractant.

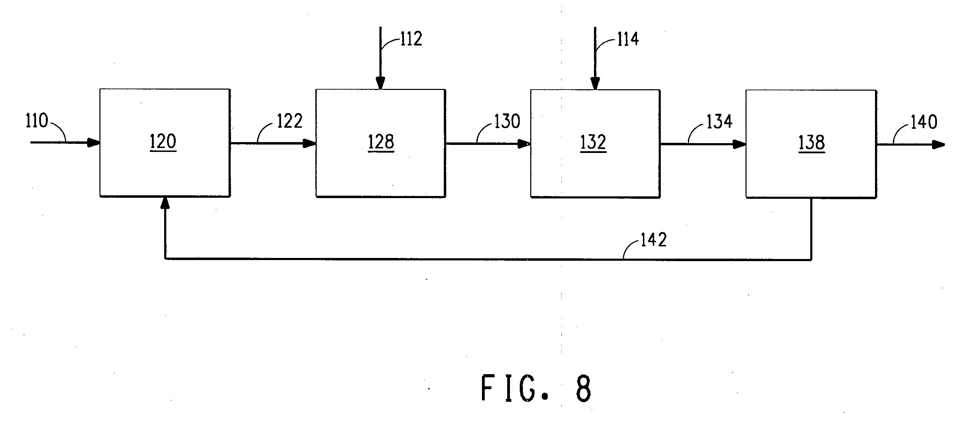

**Figure 8** schematically illustrates one example of the methods disclosed herein, in which extraction of the product occurs downstream of the fermentor and the first water immiscible extractant and the optional second water immiscible extractant are added separately to different vessels for contacting of the fermentation medium with the extractant.

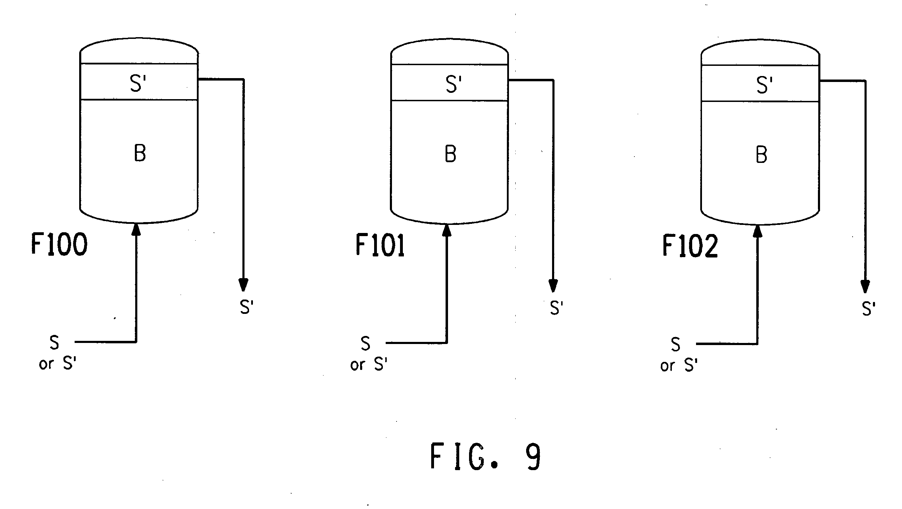

**Figure 9** schematically illustrates one example of the methods disclosed herein, in which extraction of the product occurs in at least one batch fermentor via co-current flow of an extractant at or near the bottom of a fermentation mash to fill the fermentor with extractant which flows out of the fermentor at a point at or near the top of the fermentor.

The following sequences are consistent with World Intellectual Property Organization (WIPO) Standard ST.25 (1998) and the sequence listing requirements of the EPO and PCT (Rules 5.2 and 49.5(a bis), and Section 208 and Annex C of the Administrative Instructions).

**Table 1**

| Summary of Gene and Protein SEQ ID Numbers | | |
|---|---|---|
| Description | SEQ ID NO: Nucleic acid | SEQ ID NO: Peptide |
| *Klebsiella pneumoniae budB* (acetolactate synthase) | 1 | 2 |
| *E. coli ilvC* (acetohydroxy acid reductoisomerase) | 3 | 4 |
| *E. coli ilvD* (acetohydroxy acid dehydratase) | 5 | 6 |
| *Lactococcus lactis kivD* (branched-chain α-keto acid decarboxylase), codon optimized | 7 | 8 |
| *Achromobacter xylosoxidans.* butanol dehydrogenase (sadB) gene | 9 | 10 |
| *Bacillus subtilis alsS* (acetolactate synthase) | 32 | 33 |
| Pf5.IIvC-Z4B8 (KARI) | 36 | 37 |
| *S*. *cerevisiae ILV5* (acetohydroxy acid reductoisomerase; KARI) | 40 | 41 |
| *B. subtilis* ketoisovalerate decarboxylase (KivD) codon optimized | 43 | 44 |
| Horse liver alcohol dehydrogenase (HADH) codon optimized | 45 | 46 |
| *Streptococcus mutans ilvD acetohydroxy acid dehydratase* | 58 | 59 |

SEQ ID NOs:11-22 are the nucleotide sequences of the primers used to construct the recombinant *Escherichia coli* strain described in the General Methods section of the Examples herein below.
SEQ ID NO:23 is the nucleotide sequence of the *pflB* gene from *Escherichia coli* strain K-12 MG1655.
SEQ ID NO:24 is the nucleotide sequence of the *ldhA* gene from *Escherichia coli* strain K-12 MG1655.
SEQ ID NO:25 is the nucleotide sequence of the *adhE* gene from *Escherichia coli* strain K-12 MG1655.
SEQ ID NO:26 is the nucleotide sequence of the *frdA* gene from *Escherichia coli* strain K-12 MG1655.
SEQ ID NO:27 is the nucleotide sequence of the *frdB* gene from *Escherichia coli* strain K-12 MG1655.
SEQ ID NO:28 is the nucleotide sequence of the *frdC* gene from *Escherichia coli* strain K-12 MG1655.
SEQ ID NO:29 is the nucleotide sequence of the *frdD* gene from *Escherichia coli* strain K-12 MG1655.
SEQ ID NO; 30 is the nucleotide sequence of pLH475-Z4B8.
SEQ ID NO; 31 is the nucleotide sequence of the CUP1 promoter.
SEQ ID NO; 34 is the nucleotide sequence of the CYC1 terminator.
SEQ ID NO; 35 is the nucleotide sequence of the ILV5 promoter.
SEQ ID NO; 38 is the nucleotide sequence of the ILV5 terminator.
SEQ ID NO; 39 is the nucleotide sequence of the FBA1 promoter.
SEQ ID NO; 42 is the nucleotide sequence of pLH468.
SEQ ID NO; 47 is the nucleotide sequence of pNY8.
SEQ ID NO; 48 is the nucleotide sequence of the GPD1 promoter.
SEQ ID NOs:49,50, 54, 55, 62-71, 73-83 and 85-86 are the nucleotide sequences of primers used in the examples.
SEQ ID NO; 51 is the nucleotide sequence of pRS425::GPM-sadB.
SEQ ID NO; 52 is the nucleotide sequence of the GPM1 promoter.
SEQ ID NO:53 is the nucleotide sequence of the ADH1 terminator.
SEQ ID NO:56 is the nucleotide sequence of pRS423 FBA ilvD(Strep).
SEQ ID NO:57 is the nucleotide sequence of the FBA terminator.
SEQ ID NO:60 is the nucleotide sequence of the *GPM-sadB-ADHt* segment.
SEQ ID NO:61 is the nucleotide sequence of pUC19-URA3r.
SEQ ID NO:72 is the nucleotide sequence of the *ilvD-FBA1t* segment.
SEQ ID NO:84 is the nucleotide sequence of the URA3r2 template DNA.

### DETAILED DESCRIPTION

As used above and throughout the description, the following terms, unless otherwise indicated, shall be defined as follows:
The term "butanol" as used herein, refers to 1-butanol, 2-butanol, isobutanol, or mixtures thereof.
The term "aerobic conditions" as used herein, means growth conditions in the presence of oxygen.
The term "microaerobic conditions" as used herein, means growth conditions with low levels of oxygen (i.e., below normal atmospheric oxygen levels).
The term "anaerobic conditions" as used herein, means growth conditions in the absence of oxygen.
The term "fermentable carbon source" as used herein, refers to a carbon source capable of being metabolized by the microorganisms disclosed herein. Suitable fermentable carbon sources include, but are not limited to, monosaccharides, such as glucose or fructose; disaccharides, such as lactose or sucrose; oligosaccharides; polysaccharides, such as starch or cellulose; one carbon substrates; and mixtures thereof.
The term "extractant" as used herein refers to organic solvent used to extract any butanol isomer.
The term "biphasic fermentation medium" as used herein, refers to a two-phase growth medium comprising a fermentation medium (i.e., the aqueous phase) and a suitable amount of a water immiscible organic extractant.
The term "butanol biosynthetic pathway" as used herein, refers to an enzyme pathway to produce 1-butanol, 2-butanol, or isobutanol.
The term "1-butanol biosynthetic pathway" as used herein, refers to an enzyme pathway to produce 1-butanol from acetyl-coenzyme A (acetyl-CoA).
The term "2-butanol biosynthetic pathway" as used herein, refers to an enzyme pathway to produce 2-butanol from pyruvate.
The term "isobutanol biosynthetic pathway" as used herein, refers to an enzyme pathway to produce isobutanol from pyruvate.
The term "fatty acid" as used herein, refers to a carboxylic acid having a long, aliphatic chain (i.e., C₁₁ to C₂₂), which is either saturated or unsaturated.
The term "fatty alcohol" as used herein, refers to an alcohol having a long, aliphatic chain (i.e., C₁₁ to C₂₂), which is either saturated or unsaturated.
The term "fatty aldehyde" as used herein, refers to an aldehyde having a long, aliphatic chain (i.e., C₁₁ to C₂₂), which is either saturated or unsaturated.
The term "effective titer" as used herein, refers to the total amount of butanol produced by fermentation per liter of fermentation medium. The total amount of butanol includes the amount of butanol in the fermentation medium, and the amount of butanol recovered from the organic extractant and from the gas phase, if gas stripping is used.
The term "minimal media" as used herein, refers to growth media that contain the minimum nutrients possible for growth, generally without the presence of amino acids. A minimal medium typically contains a fermentable carbon source and various salts, which may vary among microorganisms and growing conditions; these salts generally provide essential elements such as magnesium, nitrogen, phosphorus, and sulfur to allow the microorganism to synthesize proteins and nucleic acids.
The term "defined media" as used herein, refers to growth media that have known quantities of all ingredients. e.g., a defined carbon source and nitrogen source, and trace elements and vitamins required by the microorganism.
The term "OD" as used herein, refers to optical density.
The term "OD₆₀₀" as used herein, refers to the optical density at a wavelength of 600 nm.
The term "id" as used herein, refers to internal diameter.
The term "Aq" as used herein, refers to aqueous phase.
The term "Org" as used herein, refers to organic phase.
The term "IPTG" as used herein, refers to isopropyl β-D-thiogalactopyranoside.
The term "vvm" as used herein, refers to volume to volume per minute.
The term "ATCC" as used herein, refers to the American Type Culture Collection, Manassas, VA.
The term "vol" means volume.
The term "rpm" means revolutions per minute.
The term "sec" means second(s).
The term "min" means minute(s).
The term "h" means hour(s).
The term "µL" means microliter.
The term "mL" means milliliter(s).
The term "L" means liter(s).
The term "mL/min" means milliliters per minute.
The term "mmol" means millimole(s).
The term "mM" means millimolar.
The term "M" means molar.
The term "µm" means micrometer.
The term "g" means grams.
The term "µg" means microgram.
The term "g/g" means gram per gram.
The term "g/L" means grams per liter.
The term "µg/mL" means microgram per liter.
The term "mg/L" means micgram per liter.
The term "mmol/min/mg" means millimole per minute per milligram.
The term "g/L/h" means grams per liter per hour.
The term "HPLC" means high pressure liquid chromatography.
The term "GC" means gas chromatography.

### Genetically Modified Microorganisms

Microbial hosts for butanol production may be selected from bacteria, cyanobacteria, filamentous fungi and yeasts. The microbial host used should be tolerant to the butanol product produced, so that the yield is not limited by toxicity of the product to the host. The selection of a microbial host for butanol production is described in detail below.

Microbes that are metabolically active at high titer levels of butanol are not well known in the art. Although butanol-tolerant mutants have been isolated from solventogenic *Clostridia,* little information is available concerning the butanol tolerance of other potentially useful bacterial strains. Most of the studies on the comparison of alcohol tolerance in bacteria suggest that butanol is more toxic than ethanol (de Cavalho et al., Microsc. Res. Tech. 64:215-22 (2004) and Kabelitz et al., FEMS Microbiol. Lett. 220:223-227 (2003)). Tomas et al. (J. Bacteriol. 186:2006-2018 (2004)) report that the yield of 1-butanol during fermentation in *Clostridium acetobutylicum* may be limited by butanol toxicity. The primary effect of 1-butanol on *Clostridium acetobutylicum* is disruption of membrane functions (Hermann et al., Appl. Environ. Microbiol. 50:1238-1243 (1985)).

The microbial hosts selected for the production of butanol should be tolerant to butanol and should be able to convert carbohydrates to butanol using the introduced biosynthetic pathway as described below. The criteria for selection of suitable microbial hosts include the following: intrinsic tolerance to butanol, high rate of carbohydrate utilization, availability of genetic tools for gene manipulation, and the ability to generate stable chromosomal alterations.

Suitable host strains with a tolerance for butanol may be identified by screening based on the intrinsic tolerance of the strain. The intrinsic tolerance of microbes to butanol may be measured by determining the concentration of butanol that is responsible for 50% inhibition of the growth rate (IC50) when grown in a minimal medium. The IC50 values may be determined using methods known in the art. For example, the microbes of interest may be grown in the presence of various amounts of butanol and the growth rate monitored by measuring the optical density at 600 nanometers. The doubling time may be calculated from the logarithmic part of the growth curve and used as a measure of the growth rate. The concentration of butanol that produces 50% inhibition of growth may be determined from a graph of the percent inhibition of growth versus the butanol concentration. Preferably, the host strain should have an IC50 for butanol of greater than about 0.5%. More suitable is a host strain with an IC50 for butanol that is greater than about 1.5%. Particularly suitable is a host strain with an IC50 for butanol that is greater than about 2.5%.

The microbial host for butanol production should also utilize glucose and/or other carbohydrates at a high rate. Most microbes are capable of utilizing carbohydrates. However, certain environmental microbes cannot efficiently use carbohydrates, and therefore would not be suitable hosts.

The ability to genetically modify the host is essential for the production of any recombinant microorganism. Modes of gene transfer technology that may be used include by electroporation, conjugation, transduction or natural transformation. A broad range of host conjugative plasmids and drug resistance markers are available. The cloning vectors used with an organism are tailored to the host organism based on the nature of antibiotic resistance markers that can function in that host.

The microbial host also may be manipulated in order to inactivate competing pathways for carbon flow by inactivating various genes. This requires the availability of either transposons or chromosomal integration vectors to direct inactivation. Additionally, production hosts that are amenable to chemical mutagenesis may undergo improvements in intrinsic butanol tolerance through chemical mutagenesis and mutant screening.

Based on the criteria described above, suitable microbial hosts for the production of butanol include, but are not limited to, members of the genera, *Zymomonas, Escherichia, Salmonella, Rhodococcus, Pseudomonas, Bacillus, Lactobacillus, Enterococcus, Pediococcus, Alcaligenes, Klebsiella, Paenibacillus, Arthrobacter, Corynebacterium, Brevibacterium, Pichia, Candida, Hansenula* and *Saccharomyces.* Preferred hosts include: *Escherichia coli, Alcaligenes eutrophus, Bacillus licheniformis, Paenibacillus macerans, Rhodococcus erythropolis, Pseudomonas putida, Lactobacillus plantarum, Enterococcus faecium, Enterococcus gallinarium, Enterococcus faecalis, Pediococcus pentosaceus, Pediococcus acidilactici, Bacillus subtilis* and *Saccharomyces cerevisiae.*

Microorganisms mentioned above may be genetically modified to convert fermentable carbon sources into butanol, specifically 1-butanol, 2-butanol, or isobutanol, using methods known in the art. Particularly suitable microorganisms include *Escherichia Lactobacillus,* and *Saccharomyces,* where *E. coli, L. plantarum* and *S*. *cerevisiae* are particularly preferred. Additionally, the microorganism may be a butanol-tolerant strain of one of the microorganisms listed above that is isolated using the method described by Bramucci et al. (commonly owned U.S. Patent Application No. 11/761497 (see US 7 541 173, Jun 2, 2009); and WO 2007/146377). An example of one such strain is *Lactobacillus plantarum* strain PN0512 (ATCC: PTA-7727, biological deposit made July 12, 2006 for U.S. Patent Application No. 11/761497).

These microorganisms can be genetically modified to contain a 1-butanol biosynthetic pathway to produce 1-butanol, as described by Donaldson et al. in WO 2007/041269. For example, the microorganism may be genetically modified to express a 1-butanol biosynthetic pathway comprising the following enzyme-catalyzed substrate to product conversions:
a) acetyl-CoA to acetoacetyl-CoA;
b) acetoacetyl-CoA to 3-hydroxybutyryl-CoA;
c) 3-hydroxybutyryl-CoA to crotonyl-CoA;
d) crotonyl-CoA to butyryl-CoA;
e) butyryl-CoA to butyraldehyde; and
f) butyraldehyde to 1-butanol.

The microorganisms may also be genetically modified to express a 2-butanol biosynthetic pathway to produce 2-butanol, as described by Donaldson et al. in U.S. Patent Application Publication Nos. 2007/0259410 and 2007/0292927, WO 2007/130518 and WO 2007/130521. For example, in one embodiment the microorganism may be genetically modified to express a 2-butanol biosynthetic pathway comprising the following enzyme-catalyzed substrate to product conversions:
a) pyruvate to alpha-acetolactate;
b) alpha-acetolactate to acetoin;
c) acetoin to 2,3-butanediol;
d) 2,3-butanediol to 2-butanone; and
e) 2-butanone to 2-butanol.

The microorganisms may also be genetically modified to express an isobutanol biosynthetic pathway to produce isobutanol, as described by Donaldson et al. in U.S. Patent Application Publication No. 2007/0092957 and WO 2007/050671. For example, the microorganism may be genetically modified to contain an isobutanol biosynthetic pathway comprising the following enzyme-catalyzed substrate to product conversions:
a) pyruvate to acetolactate;
b) acetolactate to 2,3-dihydroxyisovalerate;
c) 2,3-dihydroxyisovalerate to α-ketoisovalerate,;
d) α-ketoisovalerate to isobutyraldehyde; and
e) isobutyraldehyde to isobutanol.

The microorganism genetically modified to be capable of converting fermentable carbon sources into butanol may be a recombinant *Escherichia coli* strain that comprises an isobutanol biosynthetic pathway, as described above, and deletions of the following genes to eliminate competing pathways that limit isobutanol production, *pflB,* given as SEQ ID NO:23, (encoding for pyruvate formate lyase), *IdhA,* given as SEQ ID NO:24, (encoding for lactate dehydrogenase), *adhE,* given as SEQ ID NO:25, (encoding for alcohol dehydrogenase), and at least one gene comprising the *frdABCD* operon (encoding for fumarate reductase), specifically, *frdA,* given as SEQ ID NO:26, *frdB*, given as SEQ ID NO:27, *frdC,* given as SEQ ID NO:28, and *frdD,* given as SEQ ID NO:29,

The *Escherichia coli* strain may comprise: (a) an isobutanol biosynthetic pathway encoded by the following genes: *budB* (given as SEQ ID NO:1) from *Klebsiella pneumoniae* encoding acetolactate synthase (given as SEQ ID NO:2), *ilvC* (given as SEQ ID NO:3) from *E. coli* encoding acetohydroxy acid reductoisomerase (given as SEQ ID NO:4), *ilvD* (given as SEQ ID NO:5) from *E. coli* encoding acetohydroxy acid dehydratase (given as SEQ ID NO:6), *kivD* (given as SEQ ID NO:7) from *Lactococcus lactis* encoding the branched-chain keto acid decarboxylase (given as SEQ ID NO:8), and *sadB* (given as SEQ ID NO:9) from *Achromobacter xylosoxidans* encoding a butanol dehydrogenase (given as SEQ ID NO:10); and (b) deletions of the following genes: *pflB* (SEQ ID NO:23), *ldhA* (SEQ ID NO:24) *adhE* (SEQ ID NO:25), and *frdB* (SEQ ID NO:27). The enzymes encoded by the genes of the isobutanol biosynthetic pathway catalyze the substrate to product conversions for converting pyruvate to isobutanol, as described above. Specifically, acetolactate synthase catalyzes the conversion of pyruvate to acetolactate, acetohydroxy acid reductoisomerase catalyzes the conversion of acetolactate to 2,3-dihydroxyisovalerate, acetohydroxy acid dehydratase catalyzes the conversion of 2,3-dihydroxyisovalerate to α-ketoisovalerate, branched-chain keto acid decarboxylase catalyzes the conversion of α-ketoisovalerate to isobutyraldehyde, and butanol dehydrogenase catalyzes the conversion of isobutyraldehyde to isobutanol. This recombinant *Escherichia coli* strain can be constructed using methods known in the art, as exemplified in the General Methods Section of the Examples herein below.

The *Saccharomyces cerevisiae* strain may comprise: an isobutanol biosynthetic pathway encoded by the following genes: *alsS* coding region from *Bacillus subtilis* (SEQ ID NO:32) encoding acetolactate synthase (SEQ ID NO:33), *ILV5* from *S. cerevisiae* (SEQ ID NO:40) encoding acetohydroxy acid reductoisomerase (KARI; SEQ ID NO:41) and/or a mutant KARI such as encoded by Pf5.IIvC-Z4B8 (SEQ ID NO:36; protein SEQ ID NO:37), *ilvD* from *Streptococcus mutans* (SEQ ID NO:58) encoding acetohydroxy acid dehydratase (SEQ ID NO:59), *kivD* from *Bacillus subtilis* (SEQ ID NO:43) encoding the branched-chain keto acid decarboxylase (SEQ ID NO:44), and *sadB* from *Achromobacter xylosoxidans* (SEQ ID NO:9) encoding a butanol dehydrogenase (SEQ ID NO:10). The enzymes encoded by the genes of the isobutanol biosynthetic pathway catalyze the substrate to product conversions for converting pyruvate to isobutanol, as described herein.

A preferred yeast strain expressing an isobutanol pathway has acetolactate synthase (ALS) activity in the cytosol and has deletions of the endogenous pyruvate decarboxylase (PDC) genes as described in commonly owned and co-pending US Patent Application #61/058970. This combination of cytosolic ALS and reduced PDC expression was found to greatly increase flux from pyruvate to acetolactate, which then flows to the pathway for production of isobutanol.

This recombinant *Saccharomyces cerevisiae* strain can be constructed using methods known in the art, as exemplified in the General Methods section of the Examples herein below.

### Organic Extractants

Extractants useful in the methods described herein are water immiscible organic solvents. Suitable organic extractants should meet the criteria for an ideal solvent for a commercial two-phase extractive fermentation for the production or recovery of butanol. Specifically, the extractant should (i) be nontoxic to the butanol-producing microorganisms such as, for example, genetically modified *Escherichia coli, Lactobacillus plantarum,* and *Saccharomyces cerevisiae,* (ii) be substantially immiscible with the fermentation medium, (iii) have a high partition coefficient for the extraction of butanol, (iv) have a low partition coefficient for the extraction of nutrients, (v) have a low tendency to form emulsions with the fermentation medium, and (vi) be low cost and nonhazardous. Suitable organic extractants for use in the Methods disclosed herein are selected from the group consisting of C₁₂ to C₂₂ fatty alcohols, C₁₂ to C₂₂ fatty acids, esters of C₁₂ to C₂₂ fatty acids, C₁₂ to C₂₂ fatty aldehydes, and mixtures thereof. As used herein, the term "mixtures thereof" encompasses both mixtures within and mixtures between these group members, for example mixtures within C₁₂ to C₂₂ fatty alcohols, and also mixtures between C₁₂ to C₂₂ fatty alcohols and C₁₂ to C₂₂ fatty acids, for example.

In some instances extractants having less than 12-carbon chain lengths can be harmful to the microorganism and therefore harmful to the process of providing butanol via a biosynthetic path. In the case of an 11-carbon extractant, the effect on a microorganism can be dependent on the conditions, but can be harmful. In the case where a C₁₁ fatty alcohol, C₁₁ fatty acid, an ester of a C₁₂ fatty acid, a C₁₁ aldehyde, and mixtures thereof can be deleterious to the process, for example in the case where a microorganism is adversely affected by the C₁₁ compound under the conditions used, such use is to be avoided. Suitable organic extractants are further selected from the group consisting of oleyl alcohol (CAS No. 143-28-2), behenyl alcohol (CAS No. 661-19-8), cetyl alcohol (CAS No. 36653-82-4), lauryl alcohol, also referred to as 1-dodecanol (CAS No. 112-53-8), myristyl alcohol (112-72-1), stearyl alcohol (CAS No. 112-92-5), 1-undecanol (CAS No. 112-42-5), oleic acid (CAS No. 112-80-1), lauric acid (CAS No. 143-07-7), myristic acid (CAS No. 544-63-8), stearic acid (CAS No. 57-11-4), methyl myristate CAS No. 124-10-7), methyl oleate (CAS No. 112-62-9), undecanal (CAS No. 112-44-7), lauric aldehyde (CAS No. 112-54-9), 2-methylundecanal (CAS No. 110-41-8), and mixtures thereof. These organic extractants are available commercially from various sources, such as Sigma-Aldrich (St. Louis, MO), in various grades, many of which may be suitable for use in extractive fermentation to produce or recover butanol. Technical grades contain a mixture of compounds, including the desired component and higher and lower fatty components. For example, one commercially available technical grade oleyl alcohol contains about 65% oleyl alcohol and a mixture of higher and lower fatty alcohols.

One of reasonable skill in the art can appreciate that it may be advantageous to use a mixture of the organic extractants. For example, solvent mixtures may be used to increase the partition coefficient of the product. Additionally, solvent mixtures may be used to adjust and optimize physical characteristics of the solvent, such as the density, boiling point, and viscosity.

### Methods for Producing Butanol Using Two-Phase Extractive Fermentation

The microorganism may be cultured in a suitable fermentation medium in a suitable fermentor to produce butanol. Any suitable fermentor may be used including a stirred tank fermentor, an airlift fermentor, a bubble fermentor, or any combination thereof. Materials and methods for the maintenance and growth of microbial cultures are well known to those skilled in the art of microbiology or fermentation science (See for example, Bailey et al., Biochemical Engineering Fundamentals, second edition, McGraw Hill, New York, 1986). Consideration must be given to appropriate fermentation medium, pH, temperature, and requirements for aerobic, microaerobic, or anaerobic conditions, depending on the specific requirements of the microorganism, the fermentation, and the process. The fermentation medium used is not critical, but it must support growth of the microorganism used and promote the biosynthetic pathway necessary to produce the desired butanol product. A conventional fermentation medium may be used, including, but not limited to, complex media containing organic nitrogen sources such as yeast extract or peptone and at least one fermentable carbon source; minimal media; and defined media. Suitable fermentable carbon sources include, but are not limited to, monosaccharides, such as glucose or fructose; disaccharides, such as lactose or sucrose; oligosaccharides; polysaccharides, such as starch or cellulose; one carbon substrates; and mixtures thereof. In addition to the appropriate carbon source, the fermentation medium may contain a suitable nitrogen source, such as an ammonium salt, yeast extract or peptone, minerals, salts, cofactors, buffers and other components, known to those skilled in the art (Bailey et al. *supra*). Suitable conditions for the extractive fermentation depend on the particular microorganism used and may be readily determined by one skilled in the art using routine experimentation.

### Methods for Recovering Butanol Using Two-Phase Extractive Fermentation

Butanol is recovered from a fermentation medium containing butanol, water, at least one fermentable carbon source, and a microorganism that has been genetically modified (that is, genetically engineered) to produce butanol via a biosynthetic pathway from at least one carbon source. Such genetically modified microorganisms can be selected from the group consisting of *Escherichia coli, Lactobacillus plantarum,* and *Saccharomyces cerevisiae.* The first step in the process is contacting the fermentation medium with a water immiscible organic extractant, described above, to form a two-phase mixture comprising an aqueous phase and a butanol-containing organic phase. "Contacting" means the fermentation medium and the organic extractant are brought into physical contact at any time during the fermentation process. In one embodiment, the fermentation medium further comprises ethanol, and the butanol-containing organic phase can contain ethanol.

The organic extractant may contact the fermentation medium at the start of the fermentation forming a biphasic fermentation medium. Alternatively, the organic extractant may contact the fermentation medium after the microorganism has achieved a desired amount of growth, which can be determined by measuring the optical density of the culture.

Further, the organic extractant may contact the fermentation medium at a time at which the butanol level in the fermentation medium reaches a preselected level, for example, before the butanol concentration reaches a toxic level. The butanol concentration may be monitored during the fermentation using methods known in the art, such as gas chromatography or high performance liquid chromatography.

Fermentation may be run under aerobic conditions for a time sufficient for the culture to achieve a preselected level of growth, as determined by optical density measurement. An inducer may then be added to induce the expression of the butanol biosynthetic pathway in the modified microorganism, and fermentation conditions are switched to microaerobic or anaerobic conditions to stimulate butanol production, as described in detail in Example 6 herein below. The extractant is added after the switch to microaerobic or anaerobic conditions.

After contacting the fermentation medium with the organic extractant, the butanol product partitions into the organic extractant, decreasing the concentration in the aqueous phase containing the microorganism, thereby limiting the exposure of the production microorganism to the inhibitory butanol product. The volume of the organic extractant to be used depends on a number of factors, including the volume of the fermentation medium, the size of the fermentor, the partition coefficient of the extractant for the butanol product, and the fermentation mode chosen, as described below. The volume of the organic extractant is about 3% to about 60% of the fermentor working volume.

The next step is separating the butanol-containing organic phase from the aqueous phase using methods known in the art, including but not limited to, siphoning, decantation, centrifugation, using a gravity settler, membrane-assisted phase splitting, and the like. Recovery of the butanol from the butanol-containing organic phase can be done using methods known in the art, including but not limited to, distillation, adsorption by resins, separation by molecular sieves, pervaporation, and the like. Specifically, distillation may be used to recover the butanol from the butanol-containing organic phase.

In the methods of the invention, gas stripping may be used concurrently with the organic extractant to remove the butanol product from the fermentation medium. Gas stripping may be done by passing a gas such as air, nitrogen, or carbon dioxide through the fermentation medium, thereby forming a butanol-containing gas phase. The butanol product may be recovered from the butanol-containing gas phase using methods known in the art, such as using a chilled water trap to condense the butanol, or scrubbing the gas phase with a solvent. Thus, the methods of the invention include gas stripping as defined in independent claims 1-5.

Any butanol remaining in the fermentation medium after the fermentation run is completed may be recovered by continued extraction using fresh or recycled organic extractant. Alternatively, the butanol can be recovered from the fermentation medium using methods known in the art, such as distillation, azeotropic distillation, liquid-liquid extraction, adsorption, gas stripping, membrane evaporation, pervaporation, and the like.

The two-phase extractive fermentation method may be carried out in a continuous mode in a stirred tank fermentor. In this mode, the mixture of the fermentation medium and the butanol-containing organic extractant is removed from the fermentor. The two phases are separated by means known in the art including, but not limited to, siphoning, decantation, centrifugation, using a gravity settler, membrane-assisted phase splitting, and the like, as described above. After separation, the fermentation medium may be recycled to the fermentor or may be replaced with fresh medium. Then, the extractant is treated to recover the butanol product as described above. The extractant may then be recycled back into the fermentor for further extraction of the product. Alternatively, fresh extractant may be continuously added to the fermentor to replace the removed extractant. This continuous mode of operation offers several advantages. Because the product is continually removed from the reactor, a smaller volume of organic extractant is required enabling a larger volume of the fermentation medium to be used. This results in higher production yields. The volume of the organic extractant may be about 3% to about 50% of the fermentor working volume; 3% to about 20% of the fermentor working volume; or 3% to about 10% of the fermentor working volume. It is beneficial to use the smallest amount of extractant in the fermentor as possible to maximize the volume of the aqueous phase, and therefore, the amount of cells in the fermentor. The process may be operated in an entirely continuous mode in which the extractant is continuously recycled between the fermentor and a separation apparatus and the fermentation medium is continuously removed from the fermentor and replenished with fresh medium. In this entirely continuous mode, the butanol product is not allowed to reach the critical toxic concentration and fresh nutrients are continuously provided so that the fermentation may be carried out for long periods of time. The apparatus that may be used to carryout these modes of two-phase extractive fermentations are well known in the art. Examples are described, for example, by Kollerup et al. in U.S. Patent No. 4,865,973.

Batchwise fermentation mode may also be used. Batch fermentation, which is well known in the art, is a closed system in which the composition of the fermentation medium is set at the beginning of the fermentation and is not subjected to artificial alterations during the process. In this mode, a volume of organic extractant is added to the fermentor and the extractant is not removed during the process. Although this mode is simpler than the continuous or the entirely continuous modes described above, it requires a larger volume of organic extractant to minimize the concentration of the inhibitory butanol product in the fermentation medium. Consequently, the volume of the fermentation medium is less and the amount of product produced is less than that obtained using the continuous mode. The volume of the organic solvent in the batchwise mode may be 20% to about 60% of the fermentor working volume; or 30% to about 60% of the fermentor working volume. It is beneficial to use the smallest volume of extractant in the fermentor as possible, for the reason described above.

Fed-batch fermentation mode may also be used. Fed-batch fermentation is a variation of the standard batch system, in which the nutrients, for example glucose, are added in increments during the fermentation. The amount and the rate of addition of the nutrient may be determined by routine experimentation. For example, the concentration of critical nutrients in the fermentation medium may be monitored during the fermentation. Alternatively, more easily measured factors such as pH, dissolved oxygen, and the partial pressure of waste gases, such as carbon dioxide, may be monitored. From these measured parameters, the rate of nutrient addition may be determined. The amount of organic solvent used in this mode is the same as that used in the batch-wise mode, described above.

Extraction of the product may be done downstream of the fermentor, rather than *in situ*. In this external mode, the extraction of the butanol product into the organic extractant is carried out on the fermentation medium removed from the fermentor. The amount of organic solvent used is about 20% to about 60% of the fermentor working volume; or 30% to about 60% of the fermentor working volume. The fermentation medium may be removed from the fermentor continuously or periodically, and the extraction of the butanol product by the organic extractant may be done with or without the removal of the cells from the fermentation medium. The cells may be removed from the fermentation medium by means known in the art including, but not limited to, filtration or centrifugation. After separation of the fermentation medium from the extractant by means described above, the fermentation medium may be recycled into the fermentor, discarded, or treated for the removal of any remaining butanol product. Similarly, the isolated cells may also be recycled into the fermentor. After treatment to recover the butanol product, the extractant may be recycled for use in the extraction process. Alternatively, fresh extractant may be used. In this mode the solvent is not present in the fermentor, so the toxicity of the solvent is much less of a problem. If the cells are separated from the fermentation medium before contacting with the solvent, the problem of solvent toxicity is further reduced. Furthermore, using this external mode there is less chance of forming an emulsion and evaporation of the solvent is minimized, alleviating environmental concerns.

A method for the production of butanol is provided, wherein a microorganism that has been genetically modified of being capable of converting at least one fermentable carbon source into butanol, is grown in a biphasic fermentation medium. Gas is passed through the fermentation medium to form a butanol-containing gas phase. The biphasic fermentation medium comprises an aqueous phase and a water immiscible organic extractant, as described above, wherein the biphasic fermentation medium comprises from about 3% to about 60% by volume of the organic extractant. The microorganism may be grown in the biphasic fermentation medium for a time sufficient to extract butanol into the extractant to form a butanol-containing organic phase. In the case where the fermentation medium further comprises ethanol, the butanol-containing organic phase may contain ethanol. The butanol-containing organic phase is then separated from the aqueous phase, as described above. Subsequently, the butanol is recovered from the butanol-containing organic phase and the butanol-containing gas phase, as described above.

Isobutanol may be produced by extractive fermentation with the use of a modified *Escherichia coli* or *Saccharomyces cerevisiae* strain in combination with oleyl alcohol as the organic extractant. Using the method described herein provides a high effective titer for isobutanol. Atsumi et al. (Nature 451 (3):86-90, 2008) report isobutanol titers up to 22 g/L using fermentation with an *Escherichia coli* that was genetically modified to contain an isobutanol biosynthetic pathway. Butanol produced by the method disclosed herein has an effective titer of greater than 22 g per liter of the fermentation medium. Alternatively, the butanol produced by methods disclosed has an effective titer of at least 25 g per liter of the fermentation medium. Alternatively, the butanol produced by methods described herein has an effective titer of at least 30 g per liter of the fermentation medium.
Alternatively, the butanol produced by methods described herein has an effective titer of at least 37 g per liter of the fermentation medium.

Without being held to theory, it is believed that the higher butanol titer obtained with the extractive fermentation method disclosed herein results, in part, from the removal of the toxic butanol product from the fermentation medium, thereby keeping the level below that which is toxic to the microorganism.

The use of the organic extractant oleyl alcohol has an additional beneficial effect that is surprising and not well understood at the time of presenting this invention. Specifically, the use of oleyl alcohol as the extractant in combination with gas stripping provides significantly higher titers than gas stripping alone, even though gas stripping alone is effective in keeping the butanol below toxic levels. Organic extractants comprising or consisting essentially of oleyl alcohol can provide improved titers in the processes described herein.

Disclosed herein in FIG. 3, there is shown a schematic representation of one example of processes for producing and recovering butanol using in situ extractive fermentation. An aqueous stream **10** of at least one fermentable carbon source is introduced into a fermentor **20**, which contains at least one microorganism (not shown) genetically modified to convert the at least one fermentable carbon source into butanol. A stream of the first water immiscible extractant **12** and a stream of the optional second water immiscible extractant **14** are introduced to a vessel **16**, in which the extractants are combined to form the extractant **18**. A stream of the extractant **18** is introduced into the fermentor **20**, whereby contact between the fermentation medium and the extractant to form a two-phase mixture comprising an aqueous phase and a butanol-containing organic phase occurs. A stream **26** comprising both the aqueous and organic phases is introduced into a vessel **38**, in which separation of the aqueous and organic phases is performed to produce a butanol-containing organic phase **40** and an aqueous phase **42**.

Disclosed herein in FIG. 4, there is shown a schematic representation of one example of processes for producing and recovering butanol using in situ extractive fermentation. An aqueous stream **10** of at least one fermentable carbon source is introduced into a fermentor **20**, which contains at least one microorganism (not shown) genetically modified to convert the at least one fermentable carbon source into butanol. A stream of the first water immiscible extractant **12** and a stream of the optional second water immiscible extractant **14** are introduced separately to the fermentor **20**, whereby contact between the fermentation medium and the extractant to form a two-phase mixture comprising an aqueous phase and a butanol-containing organic phase occurs. A stream **26** comprising both the aqueous and organic phases is introduced into a vessel **38**, in which separation of the aqueous and organic phases is performed to produce a butanol-containing organic phase **40** and an aqueous phase **42**.

Disclosed herein in FIG. 5, there is shown a schematic representation of one example of processes for producing and recovering butanol using in situ extractive fermentation. An aqueous stream **10** of at least one fermentable carbon source is introduced into a first fermentor **20**, which contains at least one microorganism (not shown) genetically modified to convert the at least one fermentable carbon source into butanol. A stream of the first water immiscible extractant **12** is introduced to the fermentor **20**, and a stream **22** comprising a mixture of the first solvent and the contents of fermentor **20** is introduced into a second fermentor **24**. A stream of the optional second water immiscible extractant **14** is introduced into the second fermentor **24**, whereby contact between the fermentation medium and the extractant to form a two-phase mixture comprising an aqueous phase and a butanol-containing organic phase occurs. A stream **26** comprising both the aqueous and organic phases is introduced into a vessel **38**, in which separation of the aqueous and organic phases is performed to produce a butanol-containing organic phase **40** and an aqueous phase **42**.

Disclosed herein in FIG. 6, there is shown a schematic representation of one example of processes for producing and recovering butanol in which extraction of the product is performed downstream of the fermentor, rather than in situ. An aqueous stream **110** of at least one fermentable carbon source is introduced into a fermentor **120**, which contains at least one microorganism (not shown) genetically modified to convert the at least one fermentable carbon source into butanol. A stream of the first water immiscible extractant **112** and a stream of the optional second water immiscible extractant **114** are introduced to a vessel **116**, in which the water immiscible extractants are combined to form the extractant **118**. At least a portion, shown as stream **122**, of the fermentation medium in fermentor **120** is introduced into vessel **124**. A stream of the extractant **118** is also introduced into vessel **124,** whereby contact between the fermentation medium and the extractant to form a two-phase mixture comprising an aqueous phase and a butanol-containing organic phase occurs. A stream **126** comprising both the aqueous and organic phases is introduced into a vessel **138,** in which separation of the aqueous and organic phases is performed to produce a butanol-containing organic phase **140** and an aqueous phase **142.**

Disclosed herein in FIG. 7, there is shown a schematic representation of one example of processes for producing and recovering butanol in which extraction of the product is performed downstream of the fermentor, rather than in situ. An aqueous stream **110** of at least one fermentable carbon source is introduced into a fermentor **120,** which contains at least one microorganism (not shown) genetically modified to convert the at least one fermentable carbon source into butanol. A stream of the first water immiscible extractant **112** and a stream of the optional second water immiscible extractant **114** are introduced separately to a vessel **124,** in which the water immiscible extractants are combined to form the extractant **118.** At least a portion, shown as stream **122,** of the fermentation medium in fermentor **120** is also introduced into vessel **124,** whereby contact between the fermentation medium and the extractant to form a two-phase mixture comprising an aqueous phase and a butanol-containing organic phase occurs. A stream **126** comprising both the aqueous and organic phases is introduced into a vessel **138**, in which separation of the aqueous and organic phases is performed to produce a butanol-containing organic phase **140** and an aqueous phase **142**.

Disclosed herein in FIG. 8, there is shown a schematic representation of one example of processes for producing and recovering butanol in which extraction of the product is performed downstream of the fermentor, rather than in situ. An aqueous stream **110** of at least one fermentable carbon source is introduced into a fermentor **120,** which contains at least one microorganism (not shown) genetically modified to convert the at least one fermentable carbon source into butanol. A stream of the first water immiscible extractant **112** is introduced to a vessel **128**, and at least a portion, shown as stream **122**, of the fermentation medium in fermentor **120** is also introduced into vessel **128.** A stream **130** comprising a mixture of the first water immiscible extractant and the contents of fermentor **120** is introduced into a second vessel **132**. A stream of the optional second water immiscible extractant **114** is introduced into the second vessel **132**, whereby contact between the fermentation medium and the extractant to form a two-phase mixture comprising an aqueous phase and a butanol-containing organic phase occurs. A stream **134** comprising both the aqueous and organic phases is introduced into a vessel **138**, in which separation of the aqueous and organic phases is performed to produce a butanol-containing organic phase **140** and an aqueous phase **142**.

The extractive processes described herein can be run as batch processes or can be run in a continuous mode where fresh extractant is added and used extractant is pumped out such that the amount of extractant in the fermentor remains constant during the entire fermentation process. Such continuous extraction of products and byproducts from the fermentation can increase effective rate, titer and yield.

It is also possible to operate the liquid-liquid extraction in a flexible co-current or, alternatively, counter-current way that accounts for the difference in batch operating profiles when a series of batch fermentors are used. In this scenario the fermentors are filled with fermentable mash which provides at least one fermentable carbon source and microorganism in a continuous fashion one after another for as long as the plant is operating. Disclosed herein in Figure 9, once Fermentor F100 fills with mash and microorganism, the mash and microorganism feeds advance to Fermentor F101 and then to Fermentor F102 and then back to Fermentor F100 in a continuous loop. The fermentation in any one fermentor begins once mash and microorganism are present together and continues until the fermentation is complete. The mash and microorganism fill time equals the number of fermentors divided by the total cycle time (fill, ferment, empty and clean). If the total cycle time is 60 hours and there are 3 fermentors then the fill time is 20 hours. If the total cycle time is 60 hours and there are 4 fermentors then the fill time is 15 hours.

Adaptive co-current extraction follows the fermentation profile assuming the fermentor operating at the higher broth phase titer can utilize the extracting solvent stream richest in butanol concentration and the fermentor operating at the lowest broth phase titer will benefit from the extracting solvent stream leanest in butanol concentration. For example, referring again to Figure 9, consider the case where Fermentor F100 is at the start of a fermentation and operating at relatively low butanol broth phase (B) titer, Fermentor F101 is in the middle of a fermentation operating at relatively moderate butanol broth phase titer and Fermentor F102 is near the end of a fermentation operating at relatively high butanol broth phase titer. In this case, lean extracting solvent (S), with minimal or no extracted butanol, can be fed to Fermentor F100, the "solvent out" stream (S') from Fermentor F100 having an extracted butanol component can then be fed to Fermentor F101 as its "solvent in" stream and the solvent out stream from F101 can then e fed to Fermentor F102 as its solvent in stream. The solvent out stream from F102 can then be sent to be processed to recover the butanol present in the stream. The processed solvent stream from which most of the butanol is removed can be returned to the system as lean extracting solvent and would be the solvent in feed to Fermentor F100 above.

As the fermentations proceed in an orderly fashion the valves in the extracting solvent manifold can be repositioned to feed the leanest extracting solvent to the fermentor operating at the lowest butanol broth phase titer. For example, assume (a) Fermentor F102 completes its fermentation and has been reloaded and fermentation begins anew, (b) Fermentor F100 is in the middle of its fermentation operating at moderate butanol broth phase titer and (c) Fermentor F101 is near the end of its fermentation operating at relatively higher butanol broth phase titer. In this scenario the leanest extracting solvent would feed F102, the extracting solvent leaving F102 would feed Fermentor F100 and the extracting solvent leaving Fermentor F100 would feed Fermentor F101.

The advantage of operating this way can be to maintain the broth phase butanol titer as low as possible for as long as possible to realize improvements in productivity. Additionally, it can be possible to drop the temperature in the other fermentors that have progressed further into fermentation that are operating at higher butanol broth phase titers. The drop in temperature can allow for improved tolerance to the higher butanol broth phase titers.

### EXAMPLES

All solvents (that is, extractants) were obtained from Sigma-Aldrich (St. Louis, MO) and were used without further purification. The oleyl alcohol used was technical grade, which contained a mixture of oleyl alcohol (65%) and higher and lower fatty alcohols. The purity of the other solvents used was as follows: oleic acid, 65 to 88%; octanoic acid, 98%; nonanol, 98%; 1-dodecanol, 98%; 1-nonanal, 95%, and 1-decanol, 98%. Isobutanol was obtained from Sigma-Aldrich and was used without further purification.

### GENERAL METHODS

### Construction of Recombinant Escherichia coli Strain NGCI-031

A recombinant *Escherichia coli strain* comprising an isobutanol biosynthetic pathway and deletions of the following genes, *pflB* (SEQ ID NO:23, encoding for pyruvate formate lyase), *ldhA* (SEQ ID NO:24, encoding for lactate dehydrogenase), *adhE* (SEQ ID NO:25, encoding for alcohol dehydrogenase), and *frdB* (SEQ ID NO:27, encoding a subunit of fumarate reductase), was constructed as described below. The genes in the isobutanol biosynthetic pathway were *budB* from *Klebsiella pneumoniae* (given as SEQ ID NO:1), *ilvC* from *Escherichia coli* (given as SEQ ID NO:3), *ilvD* from *Escherichia coli* (given as SEQ ID NO:5), *kivD* from *Lactococcus lactis* (given as SEQ ID NO:7), and *sadB* from *Achromobacter xylosoxidans* (given as SEQ ID NO:9). The construction of the recombinant strain was done in two steps. First, an *Escherichia coli* strain having the aforementioned gene deletions was constructed. Then, the genes encoding the isobutanol biosynthetic pathway were introduced into the strain.

### Construction of Recombinant Escherichia coli Strain Having Deletions of pflB, ldhA, adhE and frdB Genes

The Keio collection of *E. coli* strains (Baba et al., Mol. Syst. Biol., 2:1-11, 2006) was used for the production of the *E*. *coli* strain having the intended gene deletions, which is referred to herein as the four-knock out *E*. *coli* strain. The Keio collection is a library of single gene knockouts created in strain *E*. *coli* BW25113 by the method of Datsenko and Wanner (Datsenko, K. A. & Wanner, B. L., Proc. Natl. Acad. Sci., U.S.A. 97 6640-6645, 2000). In the collection, each deleted gene was replaced with a FRT-flanked kanamycin marker that was removable by Flp recombinase. The four-knock out *E*. *coli* strain was constructed by moving the knockout-kanamycin marker from the Keio donor strain by P1 transduction to a recipient strain. After each P1 transduction to produce a knockout, the kanamycin marker was removed by Flp recombinase. This markerless strain acted as the new donor strain for the next P1 transduction.

The four-knock out *E. coli* strain was constructed in Keio strain JW0886 by P1ᵥᵢᵣ transductions with P1 phage lysates prepared from three Keio strains in addition to JW0886. The Keio strains used are listed below:
- JW0886: the kan marker is inserted in the *pflB* gene
- JW4114: the kan marker is inserted in the *frdB* gene
- JW1375: the kan marker is inserted in the *IdhA* gene
- JW1228: the kan marker is inserted in the *adhE* gene

P1ᵥᵢᵣ transductions were carried out as described by Miller with some modifications (Miller, J. H. 1992. A Short Course in Bacterial Genetics. Cold Spring Harbor Laboratory, Cold Spring Harbor, N.Y). Briefly, to prepare a transducing lysate, cells of the donor strain were grown overnight in Luria-Bertani (LB) medium at 37 °C while shaking. An overnight growth of these cells was sub-cultured into LB medium containing 0.005 M CaCl₂ and placed in a 37 °C water bath with no aeration. One hour prior to adding phage, the cells were incubated at 37 °C with shaking. After final growth of the cells, a 1.0 mL aliquot of the culture was dispensed into 14-mL tubes and approximately 10⁷ P1ᵥᵢᵣ phage was added. The tubes were incubated in a 37 °C water bath for 20 min, after which 2.5 mL of 0.8% LB top agar was added to each tube. The contents of the tubes were spread on an LB agar plate and were incubated at 37 °C. The following day the soft agar layer was scraped into a centrifuge tube. The surface of the plate was washed with LB medium and added to the centrifuge tube, followed by a few drops of CHCl₃ and then the tube was vigorously agitated using a vortex mixer. After centrifugation at 4,000 rpm for 10 min, the supernatant containing the P1ᵥᵢᵣ lysate was collected.

For transduction, the recipient strain was grown overnight in 1-2 mL of LB medium at 37 °C with shaking. Cultures were pelleted by centrifugation in a microcentrifuge (Eppendorf) at 10,000 rpm for 1 min at room temperature. The cell pellet was resuspended in an equal volume of MC buffer (0.1 M MgSO₄, 0.005 M CaCl₂), dispensed into tubes in 0.1 mL aliquots and 0.1 mL and 0.01 mL of P1ᵥᵢᵣ lysate was added. A control tube containing no P1ᵥᵢᵣ lysate was also included. The tubes were incubated for 20 min at 37 °C after which time, 0.2 mL of 0.1 M sodium citrate was added to stop the P1 infection. One mL of LB medium was added to each tube before the tubes were incubated at 37 °C for 1 h. After incubation the cells were pelleted as described above, resuspended in 50-200 µL of LB prior to spreading on the LB plates containing 25 µg/mL of kanamycin and were incubated overnight at 37 °C. Transductants were screened by colony PCR with chromosome specific primers flanking the region upstream and downstream of the kanamycin marker insertion.

Removal of the kanamycin marker from the chromosome was obtained by transforming the kanamycin-resistant strain with plasmid pCP20 (Cherepanov, P. P. and Wackernagel, W., Gene, 158: 9-14, 1995) followed by spreading onto LB ampicillin (100 µg/mL) plates and incubating at 30 °C. The pCP20 plasmid carries the yeast FLP recombinase under the control of the λ_{PR} promoter. Expression from this promoter is controlled by the cI857 temperature-sensitive repressor residing on the plasmid. The origin of replication of pCP20 is also temperature sensitive. Ampicillin resistant colonies were streaked onto LB agar plates and incubated at 42 °C. The higher incubation temperature simultaneously induced expression of the FLP recombinase and cured the pCP20 plasmid from the cell. Isolated colonies were patched to grids onto the LB plates containing kanamycin (25 µg/mL), and LB ampicillin (100 µg/mL) plates and LB plates. The resulting kanamycin-sensitive, ampicillin-sensitive colonies were screened by colony PCR to confirm removal of the kanamycin marker from the chromosome.

For colony PCR amplifications the HotStarTaq Master Mix (Qiagen, Valencia, CA; catalog no. 71805-3) was used according to the manufacturer's protocol. Into a 25 µL Master Mix reaction containing 0.2 µM of each chromosome specific PCR primer, a small amount of a colony was added. Amplification was carried out in a DNA Thermocycler GeneAmp 9700 (PE Applied Biosystems, Foster City, CA). Typical colony PCR conditions were as follows: 15 min at 95 °C; 30 cycles of 95 °C for 30 sec, annealing temperature ranging from 50-58 °C for 30 sec, primers extended at 72 °C with an extension time of approximately 1 min/kb of DNA; then 10 min at 72 °C followed by a hold at 4 °C. PCR product sizes were determined by gel electrophoresis by comparison with known molecular weight standards.

For transformations, electrocompetent cells of *E*. *coli* were prepared as described by Ausubel, F.M., et al., (Current Protocols in Molecular Biology, 1987, Wiley-Interscience,). Cells were grown in 25-50 mL of LB medium at 30-37 °C and harvested at an OD₆₀₀ of 0.5-0.7 by centrifugation at 10,000 rpm for 10 min. These cells are washed twice in sterile ice-cold water in a volume equal to the original starting volume of the culture. After the final wash cells were resuspended in sterile water and the DNA to be transformed was added. The cells and DNA were transferred to chilled cuvettes and electroporated in a Bio-Rad Gene Pulser II according to manufacturer's instructions (Bio-Rad Laboratories, Inc Hercules, CA).

Strain JW0886 (ΔpflB::kan) was transformed with plasmid pCP20 and spread on LB plates containing 100 µg/mL of ampicillin at 30 °C. Ampicillin resistant transformants were then selected, streaked on LB plates and grown at 42 °C. Isolated colonies were patched onto the ampicillin and kanamycin selective medium plates and LB plates. Kanamycin-sensitive and ampicillin-sensitive colonies were screened by colony PCR with primers pflB CkUp (SEQ ID NO:11) and pflB CkDn (SEQ ID NO:12). A 10 µL aliquot of the PCR reaction mix was analyzed by gel electrophoresis. The expected approximate 0.4 kb PCR product was observed confirming removal of the marker and creating the "JW0886 markerless" strain. This strain had a deletion of the *pflB* gene.

The "JW0886 markerless" strain was transduced with a P1ᵥᵢᵣ lysate from JW4114 (frdB::kan) and streaked onto the LB plates containing 25 µg/mL of kanamycin. The kanamycin-resistant transductants were screened by colony PCR with primers frdB CkUp (SEQ ID NO:13) and frdB CkDn (SEQ ID NO: 14). Colonies that produced the expected approximate 1.6 kb PCR product were made electrocompetent, as described above, and transformed with pCP20 for marker removal as described above. Transformants were first spread onto LB plates containing 100 µg/mL of ampicillin at 30 °C and ampicillin resistant transformants were then selected and streaked on LB plates and grown at 42 °C. Isolated colonies were patched onto ampicillin and the kanamycin selective medium plates and LB plates. Kanamycin-sensitive, ampicillin-sensitive colonies were screened by PCR with primers frdB CkUp (SEQ ID NO:13) and frdB CkDn (SEQ ID NO: 14). The expected approximate 0.4 kb PCR product was observed confirming marker removal and creating the double knockout strain, "ΔpflB frdB".

The double knockout strain was transduced with a P1*ᵥᵢᵣ* lysate from JW1375 (ΔldhA::kan) and spread onto the LB plates containing 25 µg/mL of kanamycin. The kanamycin-resistant transductants were screened by colony PCR with primers IdhA CkUp (SEQ ID NO:15) and IdhA CkDn (SEQ ID NO:16). Clones producing the expected 1.1 kb PCR product were made electrocompetent and transformed with pCP20 for marker removal as described above. Transformants were spread onto LB plates containing 100 µg/mL of ampicillin at 30 °C and ampicillin resistant transformants were streaked on LB plates and grown at 42 °C. Isolated colonies were patched onto ampicillin and kanamycin selective medium plates and LB plates. Kanamycin-sensitive, ampicillin-sensitive colonies were screened by PCR with primers IdhA CkUp (SEQ ID NO:15) and IdhA CkDn (SEQ ID NO:16) for a 0.3 kb product. Clones that produced the expected approximate 0.3 kb PCR product confirmed marker removal and created the triple knockout strain designated the "three-knock out strain" (ΔpflB frdB IdhA).

The "three-knock out strain" was transduced with a P1*ᵥᵢᵣ* lysate from JW1228 (ΔadhE::kan) and spread onto the LB plates containing 25 µg/mL kanamycin. The kanamycin-resistant transductants were screened by colony PCR with primers adhE CkUp (SEQ ID NO: 17) and adhE CkDn (SEQ ID NO:18). Clones that produced the expected 1.6 kb PCR product were made electrocompetent and transformed with pCP20 for marker removal. Transformants were spread onto LB plates containing 100 µg/mL of ampicillin at 30 °C. Ampicillin resistant transformants were streaked on LB plates and grown at 42 °C. Isolated colonies were patched onto ampicillin and kanamycin selective plates and LB plates. Kanamycin-sensitive, ampicillin-sensitive colonies were screened by PCR with the primers adhE CkUp (SEQ ID NO: 17) and adhE CkDn (SEQ ID NO:18). Clones that produced the expected approximate 0.4 kb PCR product were named the "four-knock out strain" (ΔpflB frdB IdhA adhE).

### Introduction of the Set of Genes Encoding an Isobutanol Biosynthetic Pathway into the Four-Knock Out E. coli Strain.

The plasmid pTrc99A::budB-ilvC-ilvD-kivD was constructed as described in Examples 9-14 of copending and commonly owned U.S. Patent Application Publication No. 2007/0092957. This plasmid comprised the following genes, *budB* encoding acetolactate synthase from *Klebsiella pneumoniae* (SEQ ID NO:1),
*ilvC* gene encoding acetohydroxy acid reductoisomerase from *E*. *coli* (SEQ ID NO:3), *ilvD* encoding acetohydroxy acid dehydratase from *E. coli* (SEQ ID NO:5), and *kivD* encoding the branched-chain keto acid decarboxylase from *Lactococcus lactis* (SEQ ID NO:7). The *sadB* gene from *Achromobacter xylosoxidans* encoding a butanol dehydrogenase (SEQ ID NO:9) was subcloned into the pTrc99A::budB-ilvC-ilvD-kivD plasmid as described below.

A DNA fragment encoding a butanol dehydrogenase (DNA: SEQ ID NO:9; protein: SEQ ID NO:10) from *Achromobacterxylosoxidans* was amplified from *A. xylosoxidans* genomic DNA using standard conditions. The DNA was prepared using a Gentra Puregene kit (Gentra Systems, Inc., Minneapolis, MN; catalog number D-5500A) following the recommended protocol for gram negative organisms. PCR amplification was done using forward and reverse primers N473 and N469 (SEQ ID NOs:19 and 20, respectively) with Phusion high Fidelity DNA Polymerase (New England Biolabs, Beverly, MA). The PCR product was TOPO-Blunt cloned into pCR4 BLUNT (Invitrogen) to produce pCR4Blunt::sadB, which was transformed into *E. coli* Mach-1 cells. Plasmid was subsequently isolated from four clones, and the sequence verified.

The *sadB* coding region was then cloned into the vector pTrc99a (Amann et al., Gene 69: 301- 315, 1988). The pCR4Blunt::sadB was digested with EcoRI, releasing the sadB fragment, which was ligated with EcoRI-digested pTrc99a to generate pTrc99a::sadB. This plasmid was transformed into *E*. *coli* Mach 1 cells and the resulting transformant was named Mach1/pTrc99a::sadB. The activity of the enzyme expressed from the *sadB* gene in these cells was determined to be 3.5 mmol/min/mg protein in cell-free extracts when analyzed using isobutyraldehyde as the standard.

Then, the *sadB* gene was subcloned into pTrc99A::budB-ilvC-ilvD-kivD as follows. The *sadB* coding region was amplified from pTrc99a::sadB using primers N695A (SEQ ID NO:21) and N696A (SEQ ID NO:22) with Phusion High Fidelity DNA Polymerase (New England Biolabs, Beverly, MA). Amplification was carried out with an initial denaturation at 98 °C for 1 min, followed by 30 cycles of denaturation at 98 °C for 10 sec, annealing at 62 °C for 30 sec, elongation at 72 °C for 20 sec and a final elongation cycle at 72 °C for 5 min, followed by a 4 °C hold. Primer N695A contained an Avril restriction site for cloning and a RBS (ribosomal binding site) upstream of the ATG start codon of the *sadB* coding region. The N696A primer included an XbaI site for cloning. The 1.1 kb PCR product was digested with AvrII and Xbal (New England Biolabs, Beverly, MA) and gel purified using a Qiaquick Gel Extraction Kit (Qiagen Inc., Valencia, CA)). The purified fragment was ligated with pTrc99A::budB-ilvC-ilvD-kivD, that had been cut with the same restriction enzymes, using T4 DNA ligase (New England Biolabs, Beverly, MA). The ligation mixture was incubated at 16 °C overnight and then transformed into *E*. *coli* Mach 1™ competent cells (Invitrogen) according to the manufacturer's protocol. Transformants were obtained following growth on LB agar with 100 µg/ml of ampicillin. Plasmid DNA from the transformants was prepared with QIAprep Spin Miniprep Kit (Qiagen Inc., Valencia, CA) according to manufacturer's protocols. The resulting plasmid was called pTrc99A::budB-ilvC-ilvD-kivD-sadB. Electrocompetent four-knock out *E*. *coli* cells, prepared as described above, were transformed with pTrc99A::budB-ilvC-ilvD-kivD-sadB. Transformants were streaked onto LB agar plates containing 100 µg/mL of ampicillin. The resulting recombinant *E*. *coli* strain comprised an isobutanol biosynthetic pathway, encoded by plasmid pTrc99A::budB-ilvC-ilvD-kivD-sadB, and deletions *of pflB, frdB, IdhA,* and *adhE* genes and was designated as strain NGCI-031.

### Construction of the yeast strain NGI-049

NGI-049 is a *Saccharomyces cerevisiae* strain with insertion-inactivation of endogenous PDC1, PDC5, and PDC6 genes, and containing expression vectors pLH475-Z4B8 and pLH468. PDC1, PDC5, and PDC6 genes encode the three major isozymes of pyruvate decarboxylase. The strain expresses genes encoding enzymes for an isobutanol biosynthetic pathway that are integrated or on plasmids.

### Expression Vector pLH475-Z4B8

The pLH475-Z4B8 plasmid (SEQ ID NO:30) was constructed for expression of ALS and KARI in yeast. pLH475-Z4B8 is a pHR81 vector (ATCC #87541) containing the following chimeric genes:
1) the CUP1 promoter (SEQ ID NO:31), acetolactate synthase coding region from *Bacillus subtilis* (AlsS; SEQ ID NO:32; protein SEQ ID NO:33) and *CYC1* terminator (SEQ ID NO:34);
2) an *ILV5* promoter (SEQ ID NO:35), Pf5.IIvC-Z4B8 coding region (SEQ ID NO:36; protein SEQ ID NO:37) and *ILV5* terminator (SEQ ID NO:38); and
3) the FBA1 promoter (SEQ ID NO:39), *S*. *cerevisiae* KARI coding region (*ILV5;* SEQ ID NO:40; protein SEQ ID NO:41) and CYC1 terminator.

The Pf5.IIvC-Z4B8 coding region is a sequence encoding KARI derived from *Pseudomonas fluorescens* but containing mutations. The Pf5.IIvC-Z4B8 encoded KARI (SEQ ID NO:37;) has the following amino acid changes as compared to the natural *Pseudomonas fluorescens* KARI:
C33L: cysteine at position 33 changed to leucine,
R47Y: arginine at position 47 changed to tyrosine,
S50A: serine at position 50 changed to alanine,
T52D: threonine at position 52 changed to asparagine,
V53A: valine at position 53 changed to alanine,
L61 F: leucine at position 61 changed to phenylalanine,
T80I: threonine at position 80 changed to isoleucine,
A156V: alanine at position 156 changed to threonine, and
G170A: glycine at position 170 changed to alanine.

The Pf5.IIvC-Z4B8 coding region was was synthesized by DNA 2.0 (Palo Alto, CA; SEQ ID NO:6) based on codons that were optimized for expression in *Saccharomyces cerevisiae.*

### Expression Vector pLH468

The pLH468 plasmid (SEQ ID NO:42) was constructed for expression of DHAD, KivD and HADH in yeast.

Coding regions for *B. subtilis* ketoisovalerate decarboxylase (KivD) and Horse liver alcohol dehydrogenase (HADH) were synthesized by DNA2.0 based on codons that were optimized for expression in *Saccharomyces cerevisiae* (SEQ ID NO:43 and 45, respectively) and provided in plasmids pKivDy-DNA2.0 and pHadhy-DNA2.0. The encoded proteins are SEQ ID NOs:44 and 46, respectively. Individual expression vectors for KivD and HADH were constructed. To assemble pLH467 (pRS426::P_{GPD1}-*kivD*y-*GPD1*t), vector pNY8 (SEQ ID NO:47; also named pRS426.GPD-ald-GPDt, described in commonly owned and co-pending US Patent App. Pub. US2008/0182308, Example 17, was digested with *AscI* and *SfiI* enzymes, thus excising the *GPD1* promoter and the ald coding region. A *GPD1* promoter fragment (SEQ ID NO:48) from pNY8 was PCR amplified to add an *AscI* site at the 5' end, and an *SpeI* site at the 3' end, using 5' primer OT1068 and 3' primer OT1067 (SEQ ID NOs:49 and 50). The *AscI*/*SfiI* digested pNY8 vector fragment was ligated with the *GPD1* promoter PCR product digested with *AscI* and *SpeI*, and the *SpeI-SfiI* fragment containing the codon optimized *kivD* coding region isolated from the vector pKivD-DNA2.0. The triple ligation generated vector pLH467 (pRS426::P*_{GPD1}*-*kivD*y*-GPD*1t). pLH467 was verified by restriction mapping and sequencing.

pLH435 (pRS425::P*_{GPM1}*-Hadhy-ADH1t) was derived from vector pRS425::GPM-sadB (SEQ ID NO:51) which is described in commonly owned and co-pending US Patent App. #61/058970, Example 3. pRS425::GPM-sadB is the pRS425 vector (ATCC #77106) with a chimeric gene containing the GPM1 promoter (SEQ ID NO:52), coding region from a butanol dehydrogenase of Achromobacter xylosoxidans (sadB; SEQ ID NO:9; protein SEQ ID NO:10: disclosed in commonly owned and co-pending US Patent App. #61/048291), and ADH1 terminator (SEQ ID NO:53). pRS425::GPMp-sadB contains *Bbvl* and *PacI* sites at the 5' and 3' ends of the sadB coding region, respectively. A *Nhel* site was added at the 5' end of the sadB coding region by site-directed mutagenesis using primers OT1074 and OT1075 (SEQ ID NO:54 and 55) to generate vector pRS425-GPMp-sadB-NheI, which was verified by sequencing. pRS425::P_{GPM1}-*sadB*-*Nhe*I was digested with *NheI* and *PacI* to drop out the *sadB* coding region, and ligated with the *NheI-PacI* fragment containing the codon optimized HADH coding region from vector pHadhy-DNA2.0 to create pLH435.

To combine KivD and HADH expression cassettes in a single vector, yeast vector pRS411 (ATCC # 87474) was digested with *SacI* and *NotI,* and ligated with the *SacI-SalI* fragment from pLH467 that contains the *P_{GPD1}-kivD*y-*GPD1*t cassette together with the *SalI-NotI* fragment from pLH435 that contains the *P_{GPM1}-Hadhy-ADH1*t cassette in a triple ligation reaction. This yielded the vector pRS411::P*_{GPD1}*-*kivDy*-P*_{GPM1}*-H*adh*y (pLH441), which was verified by restriction mapping.

In order to generate a co-expression vector for all three genes in the lower isobutanol pathway: *ilvD*, *kivD*y and H*adhy*, we used pRS423 FBA ilvD(Strep) (SEQ ID NO:56), as the source of the IlvD gene.

This shuttle vector contains an F1 origin of replication (nt 1423 to 1879) for maintenance in *E*. *coli* and a 2 micron origin (nt 8082 to 9426) for replication in yeast. The vector has an FBA promoter (nt 2111 to 3108; SEQ ID NO:39) and FBA terminator (nt 4861 to 5860; SEQ ID NO:57). In addition, it carries the His marker (nt 504 to 1163) for selection in yeast and ampicillin resistance marker (nt 7092 to 7949) for selection in *E*. *coli.* The ilvD coding region (nt 3116 to 4828; SEQ ID NO:58; protein SEQ ID NO:59) from Streptococcus mutans UA159 (ATCC #700610) is between the FBA promoter and FBA terminator forming a chimeric gene for expression. In addition there is a lumio tag fused to the ilvD coding region (nt 4829-4849).

The first step was to linearize pRS423 FBA ilvD(Strep) (also called pRS423-FBA(SpeI)-IlvD(*Streptococcus mutans*)-Lumio) with *SacI* and *SacII* (with *SacII* site blunt ended using T4 DNA polymerase), to give a vector with total length of 9,482 bp. The second step was to isolate the *kivD*y-*hADH*y cassette from pLH441 with *SacI* and *KpnI* (with *KpnI* site blunt ended using T4 DNA polymerase), which gives a 6,063 bp fragment. This fragment was ligated with the 9,482 bp vector fragment from pRS423-FBA(SpeI)-IlvD(*Streptococcus mutans*)-Lumio. This generated vector pLH468 (pRS423::P*_{FBA1}*-*ilvD*(Strep)Lumio-*FBA1*t-P*_{GPD1}-kivDy*-*GPD1*t-P_{*GPM1*-}h*adh*y-*ADH1*t), which was confirmed by restriction mapping and sequencing.

### Construction of pdc6::GPMp1-sadB integration cassette and PDC6 deletion:

A *pdc6::GPM1p-sad8-ADH1t-URA3r* integration cassette was made by joining the *GPM-sadB-ADHt* segment (SEQ ID NO:60) from pRS425::*GPM-sadB* (described above) to the *URA3r* gene from pUC19-URA3r. pUC19-URA3r (SEQ ID NO:61) contains the *URA3* marker from pRS426 (ATCC # 77107) flanked by 75 bp homologous repeat sequences to allow homologous recombination *in vivo* and removal of the *URA3* marker. The two DNA segments were joined by SOE PCR (as described by Horton et al. (1989) Gene 77:61-68) using as template pRS425::*GPM-sadB* and pUC19-URA3r plasmid DNAs, with Phusion DNA polymerase (New England Biolabs Inc., Beverly, MA; catalog no. F-540S) and primers 114117-11A through 114117-11 D (SEQ ID NOs:62, 63, 64 and 65), and 114117-13A and 114117-13B (SEQ ID NOs:66 and 67).

The outer primers for the SOE PCR (114117-13A and 114117-13B) contained 5' and 3'~50 bp regions homologous to regions upstream and downstream of the *PDC6* promoter and terminator, respectively. The completed cassette PCR fragment was transformed into BY4700 (ATCC # 200866) and transformants were maintained on synthetic complete media lacking uracil and supplemented with 2% glucose at 30°C using standard genetic techniques (Methods in Yeast Genetics, 2005, Cold Spring Harbor Laboratory Press, Cold Spring Harbor, NY, pp. 201-202). Transformants were screened by PCR using primers 112590-34G and 112590-34H (SEQ ID NOs:68 and 69), and 112590-34F and 112590-49E (SEQ ID NOs:70 and 71) to verify integration at the *PDC6* locus with deletion of the *PDC6* coding region. The *URA3r* marker was recycled by plating on synthetic complete media supplemented with 2% glucose and 5-FOA at 30°C following standard protocols. Marker removal was confirmed by patching colonies from the 5-FOA plates onto SD -URA media to verify the absence of growth. The resulting identified strain has the genotype: BY4700 *pdc6::*P*_{GPM1}-sadB-ADH1*t.

### Construction of pdc1::PDC1-ilvD integration cassette and PDC1 deletion:

*A pdc1::PDC1p-ilvD-FBA1t-URA3*r integration cassette was made by joining the *ilvD-FBA1t* segment (SEQ ID NO:72) from pLH468 (described above) to the *URA3*r gene from pUC19-URA3r by SOE PCR (as described by Horton et al. (1989) Gene 77:61-68) using as template pLH468 and pUC19-URA3r plasmid DNAs, with Phusion DNA polymerase (New England Biolabs Inc., Beverly, MA; catalog no. F-540S) and primers 114117-27A through 114117-27D (SEQ ID NOs:73, 74, 75 and 76).

The outer primers for the SOE PCR (114117-27A and 114117-27D) contained 5' and 3'~50 bp regions homologous to regions downstream of the *PDC1* promoter and downstream of the *PDC1* coding sequence. The completed cassette PCR fragment was transformed into BY4700 *pdc6::P_{GPM1}-sadB-ADH1*t and transformants were maintained on synthetic complete media lacking uracil and supplemented with 2% glucose at 30°C using standard genetic techniques (Methods in Yeast Genetics, 2005, Cold Spring Harbor Laboratory Press, Cold Spring Harbor, NY, pp. 201-202). Transformants were screened by PCR using primers 114117-36D and 135 (SEQ ID NOs:77 and 78), and primers 112590-49E and 112590-30F (SEQ ID NOs:70 and 79) to verify integration at the *PDC*1 locus with deletion of the *PDC1* coding sequence. The *URA3r* marker was recycled by plating on synthetic complete media supplemented with 2% glucose and 5-FOA at 30°C following standard protocols. Marker removal was confirmed by patching colonies from the 5-FOA plates onto SD -URA media to verify the absence of growth. The resulting identified strain "NYLA67" has the genotype: BY4700 *pdc6::GPM1p-sadB-ADH1*t *pdc1::PDC1p-ilvD-FBA1t*.

### HIS3 deletion

To delete the endogenous HIS3 coding region, a *his3::URA3r2* cassette was PCR-amplified from URA3r2 template DNA (SEQ ID NO:84). URA3r2 contains the *URA3* marker from pRS426 (ATCC # 77107) flanked by 500 bp homologous repeat sequences to allow homologous recombination *in vivo* and removal of the *URA3* marker. PCR was done using Phusion DNA polymerase and primers 114117-45A and 114117-45B (SEQ ID NOs:85 and 86) which generated a ~2.3 kb PCR product. The *HIS3* portion of each primer was derived from the 5' region upstream of the *HIS3* promoter and 3' region downstream of the coding region such that integration of the *URA3r2* marker results in replacement of the *HIS3* coding region. The PCR product was transformed into NYLA67 using standard genetic techniques (Methods in Yeast Genetics, 2005, Cold Spring Harbor Laboratory Press, Cold Spring Harbor, NY, pp. 201-202) and transformants were selected on synthetic complete media lacking uracil and supplemented with 2% glucose at 30°C. Transformants were screened to verify correct integration by replica plating of transformants onto synthetic complete media lacking histidine and supplemented with 2% glucose at 30°C. The *URA3r* marker was recycled by plating on synthetic complete media supplemented with 2% glucose and 5-FOA at 30°C following standard protocols. Marker removal was confirmed by patching colonies from the 5-FOA plates onto SD -URA media to verify the absence of growth. The resulting identified strain "NYLA73" has the genotype: BY4700 *pdc6::GPM1p-sadB-ADH1t pdc1::PDC1p-ilvD-FBA1t Δhis3*.

### Construction of pdc5::kanMX integration cassette and PDC5 deletion:

A *pdc5::kanMX4* cassette was PCR-amplified from strain YLR134W chromosomal DNA (ATCC No. 4034091) using Phusion DNA polymerase and primers PDC5::KanMXF and PDC5::KanMXR (SEQ ID NOs:80 and 81) which generated a ~2.2 kb PCR product. The *PDC5* portion of each primer was derived from the 5' region upstream of the *PDC5* promoter and 3' region downstream of the coding region such that integration of the *kanMX4* marker results in replacement of the *PDC5* coding region. The PCR product was transformed into NYLA73 using standard genetic techniques (Methods in Yeast Genetics, 2005, Cold Spring Harbor Laboratory Press, Cold Spring Harbor, NY, pp. 201-202) and transformants were selected on YP media supplemented with 1% ethanol and geneticin (200 µg/ml) at 30°C. Transformants were screened by PCR to verify correct integration at the *PDC* locus with replacement of the *PDC5* coding region using primers PDC5kofor and N175 (SEQ ID NOs:82 and 83). The identified correct transformants have the genotype: BY4700 *pdc6::GPM1p-sadB-ADH1t pdc1::PDC1p-ilvD-FBA1t Δhis3 pdc5::kanMX4.*

Plasmid vectors pLH468 and pLH475-Z4B8 were simultaneously transformed into strain BY4700 *pdc6::GPM1*p*-sadB-ADH1*t *pdc1::PDC1*p-*ilvD-FBA1t Δhis3 pdc5::kanMX4* using standard genetic techniques *(*Methods in Yeast Genetics, 2005, Cold Spring Harbor Laboratory Press, Cold Spring Harbor, NY).and maintained on synthetic complete media lacking histidine and uracil, and supplemented with 1% ethanol at 30°C.

### GC Method for Determination of Isobutanol

The following GC method was used to determine the amount of isobutanol in the aqueous phase and organic phase in Examples 1-7 described below. The GC method utilized an HP-InnoWax column (30 m x 0.32 mm ID, 0.25 µm film) from Agilent Technologies (Santa Clara, CA). The carrier gas was helium at a flow rate of 1 mL/min measured at 150 °C with constant head pressure; injector split was 1:10 at 200 °C; oven temperature was 45 °C for 1 min, 45 °C to 230 °C at 10 °C/min, and 230 °C for 30 sec. Flame ionization detection was used at 260 °C with 40 mL/min helium makeup gas. Culture broth samples were filtered through 0.2 µm spin filters before injection. Depending on the analytical sensitivity desired, either 0.1 µL or 0.5 µL injection volumes were used. Calibrated standard curves were generated for the following compounds: ethanol, isobutanol, acetoin, meso-2,3-butanediol, and (2S,3S)-2,3-butanediol. Analytical standards were also utilized to identify retention times for isobutyraldehyde, isobutyric acid, and isoamyl alcohol. Under these conditions, the isobutanol retention time was about 5.33 minutes.

### HPLC Method for Determination of Glucose and Isobutanol in the Aqueous Phase

For Examples 7 and 8, isobutanol in the organic phase was determined using the GC method described above. For Examples 7 and 8, isobutanol and glucose concentrations in the aqueous phase were measured by HPLC (Waters Alliance Model, Milford, MA or Agilent 1100 Series, Santa Clara, CA) using a Shodex sugar SH1011 column, 8.0 mm x 300 mm, (Showa Denko K.K., Kanagawa, Japan (through Thompson Instruments, Clear Brook, VA)) using 0.01 N aqueous sulfuric acid, isocratic, as the eluant. The sample was passed through a 0.2 µm syringe filter (PALL GHP membrane) into an HPLC vial. The HPLC run conditions were as follows:
injection volume: 10 µL
Flow rate: 0.80 mL/minute
Run time: 32 minutes
Column Temperature: 50 °C
Detector: refractive index
Detector temperature: 40 °C
UV detection: 210 nm, 4 nm bandwidth
After the run, concentrations in the sample were determined from standard curves for each of the compounds. The retention times were 27.0 and 8.7 minutes for isobutanol and glucose, respectively.

### EXAMPLE 1

### Screening of Solvents

The purpose of this Example was to screen various organic solvents for use in the extractive fermentation of butanols. Solvent characteristics that were investigated were the partitioning of isobutanol between the solvent and an aqueous phase, the emulsion forming tendency of the solvent in the two-phase system, and the biocompatibility of the solvent with a wild-type *Saccharomyces cerevisiae* strain.

The partitioning of isobutanol between water and the following solvents: oleic acid (CAS No. 112-80-1), oleyl alcohol (CAS No. 143-28-2), octanoic acid (CAS No. 124-07-2), 1-nonanol (CAS No. 28473-21-4), 1-dodecanol (CAS No. 112-53-8), 1-nonanal (CAS No. 124-19-6), and 1-decanol (CAS No. 112-30-1) was investigated. Isobutanol was added to water to give aqueous solutions having a final isobutanol concentration of 10, 30, 50, and 70 g/L. These aqueous butanol solutions (12 mL) were added to test tubes and 4 mL of the solvent to be tested was added. Each solvent was tested in duplicate at each isobutanol concentration. The tubes were incubated for 3 hours with mixing at 30 °C. After that time, the aqueous phase and the solvent phase from each tube were separated by centrifugation and analyzed for isobutanol using the GC Method described in the General Methods Section herein above. The partition coefficient for isobutanol between each solvent phase and the aqueous phase was calculated, i.e., Kp = [Isobutanol]_{Org} / [isobutanol]_{Aq}. The results are summarized in Table 2.

**Table 2**

| Partition Coefficients for Isobutanol | |
|---|---|
| Solvent | Average Partition Coefficient |
| oleic acid | 2.7 |
| oleyl alcohol | 3.7 |
| octanoic acid | 6.7 |
| nonanol | 6.7 |
| 1-dodecanol | 5.2 |
| 1-nonanal | 5.7 |
| 1-decanol | 4.7 |

As can be seen from the data in the table, all the solvents tested had a favorable partition coefficient for isobutanol.

The biocompatibility of each solvent was determined using shake flask studies with *Saccharomyces cerevisiae* BY4741 obtained from ATCC. Seed shake flasks containing 700 mL of yeast extract/peptone/dextrose (YPD) medium were inoculated with 200 µL of *S*. *cerevisiae* BY4741 inoculum and incubated overnight at 30 °C with shaking at 250 rpm until the OD₆₀₀ reached about 0.5. Samples were withdrawn from the culture for measurement of OD₆₀₀ using a spectrophotometer and glucose concentration using HPLC.

The resulting seed culture was divided into eight 125 mL flasks as follows. Into one flask, which served as a control, 100 mL of the seed culture was added. Into the remaining seven flasks, 75 mL of the seed culture and 25 mL of the solvent to be tested was added. The flasks were incubated at 30 °C, with shaking at 250 rpm in a table top shaker (Innova 4230, New Brunswick Scientific, Edison, NJ) and samples were removed from each flask at various times and the OD₆₀₀ and glucose concentration were measured. The results are summarized in Tables 3 and 4.

**Table 3**

| Optical Density Results | | | | | |
|---|---|---|---|---|---|
| Solvent | OD₆₀₀ | OD₆₀₀ | OD₆₀₀ | OD₆₀₀ | OD₆₀₀ |
| | t = 0 | t = 2h | t = 4 | t = 6 h | t = 24 h |
| none (control) | 0.5 | 1.0 | 2.6 | 3.4 | 4.7 |
| oleic acid | 0.5 | 0.8 | 1.7 | 2.8 | 4.4 |
| oleyl alcohol | 0.5 | 1.0 | 2.0 | 2.4 | 3.5 |
| octanoic acid | 0.5 | 0.9 | 0.8 | 0.7 | 1.0 |
| nonanol | 0.5 | 0.5 | 0.7 | 0.6 | 0.7 |
| 1-dodecanol | 0.5 | 0.9 | 1.6 | 2.8 | 4.2 |
| 1-nonanal | 0.5 | 0.7 | 0.7 | 0.8 | 1.8 |
| 1-decanol | 0.5 | 0.5 | 0.7 | 0.6 | 0.8 |

**Table 4**

| Glucose Results | | | | | |
|---|---|---|---|---|---|
| Solvent | Glucose (g/L) | Glucose (g/L) | Glucose (g/L) | Glucose (g/L) | Glucose (g/L) |
| | t = 0 | t = 2 h | t = 2 h | t = 6 h | t = 24 h |
| none (control) | 17.5 | 14.9 | 9.3 | 2.1 | 0.0 |
| oleic acid | 17.5 | 19.0 | 10.2 | 2.2 | 0.0 |
| oleyl alcohol | 17.5 | 15.3 | 10.4 | 3.4 | 0.0 |
| octanoic acid | 17.5 | 17.1 | 17.6 | 19.2 | 18.5 |
| nonanol | 17.5 | 17.0 | 16.9 | 18.1 | 17.8 |
| 1-dodecanol | 17.5 | 15.4 | 11.6 | 3.9 | 0.0 |
| 1-nonanal | 17.5 | 16.6 | 17.0 | 17.8 | 17.6 |
| 1-decanol | 17.5 | 16.6 | 17.0 | 17.8 | 17.6 |

As can been from the results in Tables 3 and 4, the *S*. *cerevisiae* grown in the presence of oleic acid, oleyl alcohol, and 1-dodecanol exhibited about the same level of glucose utilization and growth as the control without solvent, indicating that these solvents are biocompatible with the S. *cerevisiae* strain tested. The solvents octanoic acid, 1-nonanal, 1-decanol, and nonanol all inhibited growth and glucose utilization of the *S*. *cerevisiae* strain.

### EXAMPLE 2 AND EXAMPLE 3 (COMPARATIVE)

### Growth of Saccharomyces cerevisiae in the Presence of Isobutanol and Oleyl Alcohol

The purpose of these Examples was to demonstrate that oleyl alcohol mitigates the toxicity of isobutanol to *Saccharomyces cerevisiae.* The glucose consumption rate and the growth rate of wild-type *Saccharomyces cerevisiae* BY4741 strain were measured in shake flask cultures containing a high concentration of isobutanol in the presence of oleyl alcohol (Example 2) and the absence of oleyl alcohol (Example 3, Comparative).

Three seed shake flasks containing 600 mL of YPD medium were inoculated with 100, 300, and 1000 µL of *Saccharomyces cerevisiae* BY4741 inoculum, respectively. The flasks were incubated overnight at 30 °C with shaking at 250 rpm until the OD₆₀₀ reached about 0.1. Samples were withdrawn from each culture and the OD₆₀₀ and glucose concentration were measured as described above.

To a 125 mL flask was added 100 mL of the culture which was derived from the 300 µL inoculum (Example 3, Comparative). To another 125 mL flask was added 75 mL of the culture and 25 mL of oleyl alcohol (i.e., 25 vol%) (Example 2). The flasks were incubated at 30 °C with shaking at 100 rpm. Samples were withdrawn for OD₆₀₀ and glucose measurement. When the OD₆₀₀ reached about 0.4, isobutanol was added to both flasks to a final concentration of 30 g/L of the aqueous phase. A third flask containing 75 mL of the culture and 25 vol% of oleyl alcohol, but no added isobutanol, served as a positive control. Samples were withdrawn from all flasks at various times for the determination of OD₆₀₀ and glucose concentration. The optical density results and the glucose results are given in Tables 5 and 6, respectively.

**Table 5**

| Optical Density Results in the Presence and Absence of Oleyl Alcohol | | | |
|---|---|---|---|
| Time (h) | Example 2 OD₆₀₀ | Example 3 (Comparative) OD₆₀₀ | Control OD₆₀₀ |
| 0 (isobutanol addition) | 0.4 | 0.4 | 0.4 |
| 3 | 0.5 | 0.6 | 0.6 |
| 4 | 1.3 | 0.7 | 1.7 |
| 5 | 1.7 | 0.6 | 2.5 |
| 14 | 3.1 | 0.6 | 3.3 |

**Table 6**

| Glucose Concentration in the Presence and Absence of Oleyl Alcohol | | | |
|---|---|---|---|
| Time (h) | Example 2 Glucose (g/L) | Example 3 (Comparative) Glucose (g/L) | Control Glucose (g/L) |
| 0 (isobutanol addition) | 18.9 | 18.9 | 19.7 |
| 3 | 16.6 | 17.5 | 16.0 |
| 4 | 14.4 | 17.7 | 12.9 |
| 5 | 12.0 | 17.4 | 9.2 |
| 14 | 2.7 | 17.4 | 0.3 |

As can be seen from the data in Tables 5 and 6, isobutanol at a concentration of 30 g/L in the absence of oleyl alcohol (Example 3, Comparative) almost completely inhibited glucose utilization and biomass growth. However, the culture was able to grow and utilize glucose In the presence of isobutanol at a concentration of 30 g/L when oleyl alcohol was added to the culture. The growth and glucose utilization rate of the culture containing isobutanol and oleyl alcohol were comparable to those of the control containing only oleyl alcohol. These results demonstrate that oleyl alcohol mitigates the toxicity of isobutanol to the strain of *Saccharomyces cerevisiae* studied. It would be reasonable to expect that oleyl alcohol could be used to mitigate the toxicity of isobutanol, as well as other butanols, to other strains of *Saccharomyces cerevisiae,* including recombinant strains.

### EXAMPLE 4 AND EXAMPLE 5 (COMPARATIVE)

### Growth of Lactobacillus plantarum in the Presence of Isobutanol and Oleyl Alcohol

The purpose of these Examples was to demonstrate that oleyl alcohol mitigates the toxicity of isobutanol to *Lactobacillus plantarum.* The glucose consumption rate and the growth rate of *Lactobacillus plantarum* strain PN0512 were measured in shake flask cultures containing a high concentration of isobutanol in the presence of oleyl alcohol (Example 4) and the absence of oleyl alcohol (Example 5, Comparative).

Three seed shake flasks containing 50 mL of de Man-Rogosa-Sharpe (MRS) medium were inoculated with 200, 500, and 1000 µL, respectively, of *Lactobacillus plantarum* strain PN0512 inoculum (ATCC: PTA-7727, biological deposit made July 12, 2006 for U.S. Patent Application No. 11/761497 US 7541173). The flasks were incubated overnight at 30 °C with shaking at 250 rpm until the OD₆₀₀ was between 2 and 5. A 1-L flask containing 600 mL of MRS medium was inoculated from one of the above seed flasks having an OD₆₀₀ = 3 to an initial OD₆₀₀ = 0.1 and cultivated at 30 °C with shaking at 180 rpm. After 4 to 5 hours of cultivation, the OD₆₀₀ was between 0.5 and 1.0. Samples were withdrawn from each culture and the OD₆₀₀ and glucose concentration were measured as described above.

To a 125 mL flask was added 100 mL of the culture from the above mentioned 1-L flask (Example 3, Comparative). To another 125 mL flask was added 75 mL of the culture and 25 mL of oleyl alcohol (i.e., 25 vol%) (Example 2). The flasks were incubated at 30 °C with shaking at 100 rpm. Samples were withdrawn for OD₆₀₀ and glucose measurement. When the OD₆₀₀ reached about 1.5, isobutanol was added to both flasks to a final concentration of 30 g/L of the aqueous phase. A third flask containing 75 mL of the culture and 25 vol% of oleyl alcohol, but no added isobutanol, served as a positive control. Samples were withdrawn from all flasks at various times for the determination of OD₆₀₀ and glucose concentration. The optical density results and the glucose results are given in Tables 7 and 8, respectively.

**Table 7**

| Optical Density Results in the Presence and Absence of Oleyl Alcohol | | | |
|---|---|---|---|
| Time (h) | Example 4 OD₆₀₀ | Example 5 (Comparative) OD₆₀₀ | Control OD₆₀₀ |
| 0 (isobutanol addition) | 6.2 | 5.7 | 5.6 |
| 3 | 8.8 | 6.8 | 8.9 |
| 4 | 10.4 | 6.9 | 10.4 |
| 5 | 12.6 | 7.2 | 14.1 |
| 14 | 7.4 | 5.3 | 8.8 |

**Table 8**

| Glucose Concentration in the Presence and Absence of Oleyl Alcohol | | | |
|---|---|---|---|
| Time (h) | Example 4 Glucose (g/L) | Example 5 (Comparative) Glucose (g/L) | Control Glucose (g/L) |
| 0 (isobutanol addition) | 12.9 | 12.7 | 12.9 |
| 3 | 7.8 | 11.7 | 7.4 |
| 4 | 5.2 | 11.7 | 4.5 |
| 5 | 2.8 | 11.7 | 1.9 |
| 14 | 0 | 11.6 | 0 |

As can be seen from the data in Tables 7 and 8, isobutanol at a concentration of 30 g/L in the absence of oleyl alcohol (Example 3, Comparative) almost completely inhibited glucose utilization and biomass growth of the *Lactobacillus* strain. However, the culture was able to grow and utilize glucose in the presence of isobutanol at a concentration of 30 g/L when oleyl alcohol was added to the culture. The growth and glucose utilization rate of the culture containing isobutanol and oleyl alcohol were comparable to those of the control containing only oleyl alcohol. These results demonstrate that oleyl alcohol mitigates the toxicity of isobutanol to the strain of *Lactobacillus plantarum* studied. It would be reasonable to expect that oleyl alcohol could be used to mitigate the toxicity of isobutanol, as well as other butanols, to other strains of *Lactobacillus plantarum,* including recombinant strains.

### EXAMPLE 6

### Production of Isobutanol By Recombinant Escherichia coli Using Extractive Fermentation

The purpose of this Example was to demonstrate the production of isobutanol by a recombinant strain of *Escherichia coli* that contains an isobutanol biosynthetic pathway using extractive fermentation with oleyl alcohol as the water immiscible, organic extractant.

The strain used was *Escherichia coli* Strain NGCI-031, constructed as described in the General Methods Section herein above. All seed cultures for inoculum preparation were grown in Luria-Bertani (LB) medium with ampicillin (100 mg/L) as the selection antibiotic. The fermentation medium was a semi-synthetic medium, the composition of which is given in Table 9.

**Table 9**

| Fermentation Medium Composition | |
|---|---|
| Ingredient | Amount/L |
| Phosphoric Acid 85% | 0.75 mL |
| Sulfuric Acid (18 M) | 0.30 mL |
| Balch's w/ Cobalt - 1000X (composition given in Table 10) | 1.00 mL |
| Potassium Phosphate Monobasic | 1.40 g |
| Citric Acid Monohydrate | 200 g |
| Magnesium Sulfate, heptahydrate | 200 g |
| Ferric Ammonium Citrate | 0.33 g |
| Calcium chloride, dihydrate | 0.20 g |
| Yeast Extract^{a} | 5.00 g |
| Antifoam 204^{b} | 0.20 mL |
| Thiamince·HCl, 5g/L stock | 1.00 mL |
| Ampicillin, 25 mg/mL stock | 4.00 mL |
| Glucose 50 wt% stock | 33.3 mL |

| | |
|---|---|
| ^{a}Obtained from BD Diagnostic Systems, Sparks, MD ^{b}Obtained from Sigma-Aldrich | |

**Table 10**

| Balch's Modified Trace Metals - 1000X | |
|---|---|
| Ingredient | Concentration (g/L) |
| Citric Acid Monohydrate | 40.0 |
| MnSO₄·H₂O | 30.0 |
| NaCl | 10.0 |
| FeSO₄·7H₂O | 1.0 |
| CoCl₂·6H₂O | 1.0 |
| ZnSO₄7H₂O | 1.5 |
| CuSO₄·5H₂O | 0.1 |
| Boric Acid (H₃BO₃) | 0.1 |
| Sodium Molybnate (NaMoO₄·2H₂O) | 0.1 |

Ingredients 1-10 from Table 9 were added to water at the prescribed concentration to make a final volume of 1.5 L in the fermentor. The contents of the fermentor were sterilized by autoclaving. Components 11-13 were mixed, filter sterilized and added to the fermentor after the autoclaved medium had cooled. The total final volume of the fermentation medium (the aqueous phase) was about 1.6 L.

The fermentation was done using a Biostat-B DCU-3 fermentor (Braun Biotech International, Melesungen, Germany) with a working volume of 2.0 L. The temperature was maintained at 30 °C during the entire fermentation and the pH was maintained at 6.8 using ammonium hydroxide. Following inoculation of the sterile fermentation medium with seed culture (2-10 vol%), the fermentor was operated aerobically at a 30% dissolved oxygen (DO) set point with 0.5 vvm of air flow by automatic control of the agitation rate (rpm). Once the desired optical density (OD₆₀₀) was reached (i.e., OD₆₀₀=10), the culture was induced with the addition of 0.4-0.5 mM IPTG to overexpress the isobutanol biosynthetic pathway. Four hours post induction, fermentation conditions were switched to microaerobic conditions by decreasing the stirrer speed to 200 rpm. The shift to microaerobic conditions initiated isobutanol production while minimizing the amount of carbon going to biomass production, thereby uncoupling biomass formation from isobutanol production. Oleyl alcohol (about 780 mL) was added during the isobutanol production phase to alleviate the problem of inhibition due to build up of isobutanol in the aqueous phase. Glucose was added as a bolus (50 wt% stock solution) to the fermentor to keep levels of glucose between 30 g/L and 2 g/L.

Because efficient production of isobutanol requires microaerobic conditions to enable redox balance in the biosynthetic pathway, air was continuously supplied to the fermentor at 0.5 vvm. Continuous aeration led to significant stripping of isobutanol from the aqueous phase of the fermentor. To quantify the loss of isobutanol due to stripping, the off-gas from the fermentor was sparged through a chilled (6.5 °C) water trap to condense the isobutanol, which was then quantified using the GC Method described in the General Methods Section herein above. Alternatively, the air stream exiting the fermentor was directly sent to a mass spectrometer (Prima dB mass spectrometer, Thermo Electron Corp., Madison, WI) to quantify the amount of isobutanol in the gas stream. The isobutanol peaks at mass to charge ratios of 74 or 42 were monitored continuously to quantify the amount of isobutanol in the gas stream.

For isobutanol production, the effective titer, the effective rate, and the effective yield, all corrected for the isobutanol lost due to stripping, were 37 g/L, 0.40 g/L/h, and 0.33 g/g, respectively. As can be seen by comparing these results to those obtained without oleyl alcohol as extractant (as shown in Example 7, Comparative below), the use of oleyl alcohol in an extractive fermentation for isobutanol production results in significantly higher effective titer, effective rate, and effective yield. The isobutanol product, which is toxic to the bacterial host, is continuously extracted into the oleyl alcohol phase, decreasing its concentration in the aqueous phase, thereby reducing the toxicity to the microorganism. Additionally, oleyl alcohol appears to have another, unexpected beneficial effect on isobutanol production. This can also be seen by comparison with Example 7 (Comparative). In that Example, gas stripping alone continuously removed isobutanol from the fermentation medium and limited the isobutanol concentration in the fermentation medium to a maximum of about 8-10 g/L, a level that is comparable to the level observed using a combination of extraction with oleyl alcohol and gas stripping (see Figure 1). In both Examples, the isobutanol concentration was below inhibitory levels for most of the fermentation. However, significantly higher isobutanol effective titer, effective rate, and effective yield were obtained with a combination of oleyl alcohol extractive fermentation and gas stripping (Example 6) than with gas stripping alone (Example 7), suggesting the oleyl alcohol has another, unexpected beneficial effect on isobutanol production.

### EXAMPLE 7 (COMPARATIVE)

### Production of Isobutanol By Recombinant Escherichia coli Using Fermentation Without Addition of an Extractant

The purpose of this Comparative Example was to demonstrate that without the addition of oleyl alcohol the production of isobutanol is inhibited due to the toxicity of the product.

The fermentation was done as described in Example 6, except that oleyl alcohol was not added to the fermentation medium during the isobutanol production phase. The following changes were also made. The culture was induced by addition of IPTG when the OD₆₀₀ reached 6, and the switch to microaerobic conditions was made 3 hours post induction. To quantify the amount of isobutanol lost due to stripping, the off-gas from the fermentor was sent directly to a mass spectrometer (Prima dB, Thermo Electron Corp., Madison, WI). The isobutanol peaks at mass to charge ratios of 74 or 42 were continuously monitored to quantify isobutanol loss due to stripping.

For isobutanol production, the effective titer, the effective rate, and the effective yield, all corrected for the isobutanol lost due to stripping, were 13 g/L, 0.21 g/L/h, and 0.22 g/g, respectively. Because isobutanol was continuously removed from the fermentation medium by gas stripping, the isobutanol concentration in the aqueous phase during fermentation was limited to a maximum of 8-10 g/L, and was actually below inhibitory levels for most of the fermentation (see Figure 1).

### EXAMPLE 8

### Production of Isobutanol By Recombinant Saccharomyces cerevisiae Using Extractive Fermentation

The purpose of this Example was to demonstrate the production of isobutanol by a recombinant strain of *Saccharomyces cerevisiae* that contains an isobutanol biosynthetic pathway using extractive fermentation with oleyl alcohol as the water immiscible, organic extractant.

The strain used was *Saccharomyces cerevisiae* Strain NGCI-049, constructed as described in the General Methods Section herein above. All seed cultures for inoculum preparation were grown in Yeast Nitrogen Base (YNB) without amino acids medium (6.7 g/L), supplemented with amino acid dropout mix (1.4 g/L), leucine (100 mg/L) and tryptophan (20 mg/L). Ethanol at 1 % (v/v) was used as the sole carbon source for all seed cultures. The fermentation medium was a semi-synthetic medium, the composition of which is given in Table 11.

**Table 11**

| Fermentation Medium Composition | |
|---|---|
| Ingredient | Amount/L |
| 1.YNB w/o amino acids ^{a} | 6.7 g |
| 2.Sigma Dropout Mix (Y2001) ^{b} | 2.8 g |
| 3.Leucine (10 g/L) | 20 mL |
| 4.Tryptophan (10 g/L) | 4 mL |
| 5.Ethanol | 10 mL |
| 6.Glucose 50 wt% stock | 4 g |

| | |
|---|---|
| ^{a}Obtained from BD Diagnostic Systems, Sparks, MD ^{b}Obtained from Sigma-Aldrich, St. Louis, MO | |

Ingredients 1-4 from Table 11 were added to water at the prescribed concentration to make a final volume of 0.54 L in the fermentor. The contents of the fermentor were sterilized by autoclaving. Components 5 and 6 were mixed, filter sterilized and added to the fermentor after the autoclaved medium had cooled. The total final volume of the fermentation medium (the aqueous phase) was about 0.54 L.

The fermentation was done using a 1 L autoclavable bioreactor, Bio Console ADI 1025 (Applikon, Inc, Holland) with a working volume of 900 mL. The temperature was maintained at 30 °C during the entire fermentation and the pH was maintained at 5.5 using sodium hydroxide. Following inoculation of the sterile fermentation medium with seed culture (10 vol%), the fermentor was operated aerobically at a 30% dissolved oxygen (DO) set point with 0.3 vvm of air flow by automatic control of the agitation rate (rpm). Once the initial batched glucose of 2 g/L was consumed, glucose was fed using a pump at an exponential rate such that glucose never accumulated above 0.2 g/L in the fermentor. Once the desired optical density (OD₆₀₀) was reached (i.e., OD₆₀₀=6), the culture was induced to isobutanol production phase by feeding glucose such that excess glucose (> 2 g/L) was maintained at all times during fermentation. Two hours post glucose excess, 60 mL of filter sterilized 10X Yeast Extract Peptone stock solution (10X YEP= 100 g/L of yeast extract and 200 g/L of peptone) was added. Two hours post addition of YEP, Oleyl alcohol (about 300 mL) was added during the isobutanol production phase to alleviate the problem of inhibition due to build up of isobutanol and other byproducts in the aqueous phase. Glucose was fed (50 wt% stock solution) to the fermentor to keep levels of glucose greater than 2 g/L.

Because efficient production of isobutanol requires microaerobic conditions to enable redox balance in the biosynthetic pathway, air was continuously supplied to the fermentor at 0.3 vvm. Continuous aeration led to significant stripping of isobutanol from the aqueous phase of the fermentor. To quantify the loss of isobutanol due to stripping, the off-gas from the fermentor was directly sent to a mass spectrometer (Prima dB mass spectrometer, Thermo Electron Corp., Madison, WI) to quantify the amount of isobutanol in the gas stream. The isobutanol peaks at mass to charge ratios of 74 or 42 were monitored continuously to quantify the amount of isobutanol in the gas stream.

Glucose and organic acids in the aqueous phase were monitored during the fermentation using HPLC. Glucose was also monitored quickly using a glucose analyzer (YSI, Inc., Yellow Springs, OH). Isobutanol in the aqueous phase was quantified by HPLC and isobutanol in the oleyl alcohol phase were monitored using the GC method described in the General Method Section herein above after the two phases were removed periodically from the fermentor and separated by centrifugation. The concentration of isobutanol in the aqueous phase during the fermentation is shown in Figure 2, where the closed squares (■) refer to the concentrations of Example 8, fermentation using oleyl alcohol as the organic extractant with gas stripping, and the closed circles (●) refer to the concentrations of Example 9 (Comparative), fermentation with gas stripping alone.

For isobutanol production, the effective titer, the effective rate, and the effective yield, all corrected for the isobutanol lost due to stripping, were 5 g/L, 0.06 g/L/h, and 0.16 g/g, respectively. As can be seen by comparing these results to those obtained without oleyl alcohol as extractant (as shown in Example 9, Comparative below), the use of oleyl alcohol in an extractive fermentation for isobutanol production results in significantly higher effective titer, effective rate, and effective yield. The isobutanol product, which is toxic to the host, is continuously extracted into the oleyl alcohol phase, decreasing its concentration in the aqueous phase, thereby reducing the toxicity to the microorganism. Additionally, oleyl alcohol appears to have another, unexpected beneficial effect on isobutanol production. This can also be seen by comparison with Example 9 (Comparative). In that Example, gas stripping alone continuously removed isobutanol from the fermentation medium and limited the isobutanol concentration in the fermentation medium to a maximum of about 4 g/L, a level that is comparable to the level observed using a combination of extraction with oleyl alcohol and gas stripping (see Figure 2). In both Examples, the isobutanol concentration was below inhibitory levels for most of the fermentation. However, significantly higher isobutanol effective titer, effective rate, and effective yield were obtained with a combination of oleyl alcohol extractive fermentation and gas stripping (Example 8) than with gas stripping alone (Example 9), suggesting the oleyl alcohol has another, unexpected beneficial effect on isobutanol production.

### EXAMPLE 9 (COMPARATIVE)

### Production of Isobutanol By Recombinant Saccharomyces cerevisiae Using Fermentation Without Addition of an Extractant

The purpose of this Comparative Example was to demonstrate that without the addition of oleyl alcohol the production of isobutanol is inhibited due to the toxicity of the product.

The fermentation was done as described in Example 8, except that oleyl alcohol was not added to the fermentation medium during the isobutanol production phase. To quantify the amount of isobutanol lost due to stripping, the off-gas from the fermentor was sent directly to a mass spectrometer (Prima dB, Thermo Electron Corp., Madison, WI). The isobutanol peaks at mass to charge ratios of 74 or 42 were continuously monitored to quantify isobutanol loss due to stripping.

For isobutanol production, the effective titer, the effective rate, and the effective yield, all corrected for the isobutanol lost due to stripping, were 3 g/L, 0.04 g/L/h, and 0.16 g/g, respectively. Because isobutanol was continuously removed from the fermentation medium by gas stripping, the isobutanol concentration in the aqueous phase during fermentation was limited to a maximum of 2-3 g/L, and was actually below inhibitory levels for most of the fermentation (see Figure 2).

### EXAMPLE 10

A 2 L fermentor can be used to run a fermentation with recombinant yeast producing isobutanol as in Example 8 except that a portion of the initial batched oleyl alcohol is removed and replaced with fresh oleyl alcohol. This process can be run in a continuous mode where fresh oleyl alcohol is slowly trickled in and spent oleyl alcohol is pumped out such that the amount of oleyl alcohol in the fermentor remains constant during the entire fermentation process. Such continuous extraction of products and byproducts from the fermentation can increase effective rate, titer and yield.

## Claims

1. A method for recovering butanol from a fermentation medium, the method comprising:
a) providing a fermentation medium comprising butanol, water, and a genetically modified microorganism that produces butanol from a fermentation medium comprising at least one fermentable carbon source;
b) passing a gas through the fermentation medium to form a butanol-containing gas phase;
c) contacting the fermentation medium with i) at least one first water immiscible organic extractant selected from the group consisting of C₁₂ to C₂₂ fatty alcohols, C₁₂ to C₂₂ fatty acids, esters of C₁₂ to C₂₂ fatty acids, C₁₂ to C₂₂ fatty aldehydes, and mixtures thereof, and optionally (ii) a second water immiscible organic extractant selected from the group consisting of C₁₂ to C₂₂ fatty alcohols, C₁₂ to C₂₂ fatty acids, esters of C₁₂ to C₂₂ fatty acids, C₁₂ to C₂₂ fatty aldehydes, and mixtures thereof to form a two-phase mixture comprising an aqueous phase and a butanol-containing organic phase;
d) separating the butanol-containing organic phase from the aqueous phase; and
e) recovering the butanol from the butanol-containing organic phase and the butanol-containing gas phase to produce recovered butanol.

2. A method for the production of butanol comprising the steps of:
a) providing a genetically modified microorganism that produces butanol from a fermentation medium comprising at least one fermentable carbon source;
b) passing a gas through the fermentation medium to form a butanol-containing gas phase;
c) growing the microorganism in a biphasic fermentation medium comprising an aqueous phase and a water immiscible organic extractant selected from the group consisting of C₁₂ to C₂₂ fatty alcohols, C₁₂ to C₂₂ fatty acids, esters of C₁₂ to C₂₂ fatty acids, C₁₂ to C₂₂ fatty aldehydes, and mixtures thereof, wherein said biphasic fermentation medium comprises from about 3% to about 60% by volume of said water immiscible organic extractant, for a time sufficient to allow extraction of the butanol into the organic extractant to form a butanol-containing organic phase;
d) separating the butanol-containing organic phase from the aqueous phase; and
e) recovering the butanol from the butanol-containing organic phase and the butanol-containing gas phase to produce recovered butanol.

3. A method for the production of butanol comprising the steps of:
a) providing a genetically modified microorganism that produces butanol from a fermentation medium comprising at least one fermentable carbon source;
b) growing the microorganism in a fermentation medium wherein the microorganism produces said butanol into the fermentation medium to produce a butanol-containing fermentation medium;
c) passing a gas through the fermentation medium to form a butanol-containing gas phase;
d) contacting the butanol-containing fermentation medium with i) at least one first water immiscible organic extractant selected from the group consisting of C₁₂ to C₂₂ fatty alcohols, C₁₂ to C₂₂ fatty acids, esters of C₁₂ to C₂₂ fatty acids, C₁₂ to C₂₂ fatty aldehydes, and mixtures thereof, and optionally (ii) a second water immiscible organic extractant selected from the group consisting of C₁₂ to C₂₂ fatty alcohols, C₁₂ to C₂₂ fatty acids, esters of C₁₂ to C₂₂ fatty acids, C₁₂ to C₂₂ fatty aldehydes, and mixtures thereof to form a two-phase mixture comprising an aqueous phase and a butanol-containing organic phase;
e) separating the butanol-containing organic phase from the aqueous phase; and
f) recovering the butanol from the butanol-containing organic phase and the butanol-containing gas phase.

4. A method for the production of butanol comprising the steps of:
a) providing a genetically modified microorganism that produces butanol from a fermentation medium comprising at least one fermentable carbon source;
b) growing the microorganism in a fermentation medium under aerobic conditions for a time sufficient to reach a preselected growth level;
c) switching to microaerobic or anaerobic conditions to stimulate butanol production into the fermentation medium to form a butanol-containing fermentation medium;
d) passing a gas through the fermentation medium to form a butanol-containing gas phase;
e) contacting the butanol-containing fermentation medium with i) at least one first water immiscible organic extractant selected from the group consisting of C₁₂ to C₂₂ fatty alcohols, C₁₂ to C₂₂ fatty acids, esters of C₁₂ to C₂₂ fatty acids, C₁₂ to C₂₂ fatty aldehydes, and mixtures thereof, and optionally (ii) a second water immiscible organic extractant selected from the group consisting of C₁₂ to C₂₂ fatty alcohols, C₁₂ to C₂₂ fatty acids, esters of C₁₂ to C₂₂ fatty acids, C₁₂ to C₂₂ fatty aldehydes, and mixtures thereof to form a two-phase mixture comprising an aqueous phase and a butanol-containing organic phase;
f) separating the butanol-containing organic phase from the aqueous phase; and
g) recovering the butanol from the butanol-containing organic phase and the butanol-containing gas phase.

5. A method for the production of butanol comprising the steps of:
a) providing a fermentation medium comprising butanol, water, and a genetically modified microorganism that produces butanol from a fermentation medium comprising at least one fermentable carbon source;
b) passing a gas through the fermentation medium to form a butanol-containing gas phase;
c) contacting the fermentation medium via a co-current or countercurrent extractant stream with i) at least one first water immiscible organic extractant selected from the group consisting of C₁₂ to C₂₂ fatty alcohols, C₁₂ to C₂₂ fatty acids, esters of C₁₂ to C₂₂ fatty acids, C₁₂ to C₂₂ fatty aldehydes, and mixtures thereof, and optionally (ii) a second water immiscible organic extractant selected from the group consisting of C₁₂ to C₂₂ fatty alcohols, C₁₂ to C₂₂ fatty acids, esters of C₁₂ to C₂₂ fatty acids, C₁₂ to C₂₂ fatty aldehydes, and mixtures thereof to form a two-phase mixture comprising an aqueous phase and a butanol-containing organic phase;
d) separating the butanol-containing organic phase from the aqueous phase; and
e) recovering the butanol from the butanol-containing organic phase and the butanol-containing gas phase to produce recovered butanol.

6. The method according to any one of Claims 1-5 wherein the organic extractant is selected from the group consisting of oleyl alcohol, behenyl alcohol, cetyl alcohol, lauryl alcohol, myristyl alcohol, stearyl alcohol, 1-undecanol, oleic acid, lauric acid, myristic acid, stearic acid, methyl myristate, methyl oleate, undecanal, lauric aldehyde, 2-methylundecanal, and mixtures thereof.

7. The method according to any one of Claims 1-6 wherein the organic extractant comprises oleyl alcohol.

8. The method according to any one of Claims 1-7 wherein the recovered butanol has an effective titer of at least about 37 g per liter of the aqueous phase.

9. The method according to any one of Claims 1-8, wherein the fermentation medium further comprises ethanol, and the butanol-containing organic phase contains ethanol.

10. The method according to any one of Claims 1-9 wherein the butanol is isobutanol.

11. The method according to any of Claims 1-10 wherein the genetically modified microorganism is selected from the group consisting of bacteria, cyanobacteria, filamentous fungi and yeasts.

12. The method according to Claim 11 wherein the bacteria are selected from the group consisting of *Zymomonas, Escherichia, Salmonella, Rhodococcus, Pseudomonas, Bacillus, Lactobacillus, Enterococcus, Pediococcus, Alcaligenes, Klebsiella, Paenibacillus, Arthrobacter, Corynebacterium, and Brevibacterium.*

13. The method according to Claim 11 wherein the yeast is selected from the group consisting of *Pichia, Candida, Hansenula* and *Saccharomyces.*

## Patentansprüche

1. Verfahren zur Rückgewinnung von Butanol aus einem Fermentationsmedium, wobei das Verfahren aufweist:
a) Bereitstellen eines Fermentationsmediums, das Butanol, Wasser und einen genetisch modifizierten Mikroorganismus aufweist, der Butanol aus einem Fermentationsmedium produziert, das mindestens eine fermentierbare Kohlenstoffquelle aufweist;
b) Durchleiten eines Gases durch das Fermentationsmedium, um eine butanolhaltige Gasphase zu bilden;
c) Inkontaktbringen des Fermentationsmediums mit i) mindestens einem ersten nicht mit Wasser mischbaren organischen Extraktionsmittel, das aus der Gruppe ausgewählt ist, die aus C₁₂-C₂₂-Fettalkoholen, C₁₂-C₂₂-Fettsäuren, Estern von C₁₂-C₂₂-Fettsäuren, C₁₂-C₂₂-Fettaldehyden und deren Gemischen besteht, und wahlweise ii) einem zweiten nicht mit Wasser mischbaren organischen Extraktionsmittel, das aus der Gruppe ausgewählt ist, die aus C₁₂-C₂₂-Fettalkoholen, C₁₂-C₂₂-Fettsäuren, Estern von C₁₂-C₂₂-Fettsäuren, C₁₂-C₂₂-Fettaldehyden und deren Gemischen besteht, um ein Zweiphasengemisch zu bilden, das eine wässrige Phase und eine butanolhaltige organische Phase aufweist;
d) Trennen der butanolhaltigen organischen Phase von der wässrigen Phase; und
e) Rückgewinnen des Butanols aus der butanolhaltigen organischen Phase und der butanolhaltigen Gasphase, um zurückgewonnenes Butanol zu produzieren.

2. Verfahren zur Herstellung von Butanol, das die folgenden Schritte aufweist:
a) Bereitstellen eines genetisch modifizierten Mikroorganismus, der Butanol aus einem Fermentationsmedium produziert, das mindestens eine fermentierbare Kohlenstoffquelle aufweist;
b) Durchleiten eines Gases durch das Fermentationsmedium, um eine butanolhaltige Gasphase zu bilden;
c) Vermehren des Mikroorganismus in einem zweiphasigen Fermentationsmedium, das eine wässrige Phase und ein nicht mit Wasser mischbares organisches Extraktionsmittel aufweist, das aus der Gruppe ausgewählt ist, die aus C₁₂-C₂₂-Fettalkoholen, C₁₂-C₂₂-Fettsäuren, Estern von C₁₂-C₂₂-Fettsäuren, C₁₂-C₂₂-Fettaldehyden und deren Gemischen besteht, wobei das zweiphasige Fermentationsmedium etwa 3 bis etwa 60 Vol.-% des nicht mit Wasser mischbaren organischen Extraktionsmittels aufweist, während einer Zeit, die ausreicht, um Extraktion des Butanols in das organische Extraktionsmittel zu ermöglichen und eine butanolhaltige organische Phase zu bilden;
d) Trennen der butanolhaltigen organischen Phase von der wässrigen Phase; und
e) Rückgewinnen des Butanols aus der butanolhaltigen organischen Phase und der butanolhaltigen Gasphase, um zurückgewonnenes Butanol zu produzieren.

3. Verfahren zur Herstellung von Butanol, das die folgenden Schritte aufweist:
a) Bereitstellen eines genetisch modifizierten Mikroorganismus, der Butanol aus einem Fermentationsmedium produziert, das mindestens eine fermentierbare Kohlenstoffquelle aufweist;
b) Vermehren des Mikroorganismus in einem Fermentationsmedium, wobei der Mikroorganismus Butanol in dem Fermentationsmedium erzeugt, um ein butanolhaltiges Fermentationsmedium zu produzieren;
c) Durchleiten eines Gases durch das Fermentationsmedium, um eine butanolhaltige Gasphase zu bilden;
d) Inkontaktbringen des butanolhaltigen Fermentationsmediums mit i) mindestens einem ersten nicht mit Wasser mischbaren organischen Extraktionsmittel, das aus der Gruppe ausgewählt ist, die aus C₁₂-C₂₂-Fettalkoholen, C₁₂-C₂₂-Fettsäuren, Estern von C₁₂-C₂₂-Fettsäuren, C₁₂-C₂₂-Fettaldehyden und deren Gemischen besteht, und wahlweise ii) einem zweiten nicht mit Wasser mischbaren organischen Extraktionsmittel, das aus der Gruppe ausgewählt ist, die aus C₁₂-C₂₂-Fettalkoholen, C₁₂-C₂₂-Fettsäuren, Estern von C₁₂-C₂₂-Fettsäuren, C₁₂-C₂₂-Fettaldehyden und deren Gemischen besteht, um ein Zweiphasengemisch zu bilden, das eine wässrige Phase und eine butanolhaltige organische Phase aufweist;
e) Trennen der butanolhaltigen organischen Phase von der wässrigen Phase; und
f) Rückgewinnen des Butanols aus der butanolhaltigen organischen Phase und der butanolhaltigen Gasphase.

4. Verfahren zur Herstellung von Butanol, das die folgenden Schritte aufweist:
a) Bereitstellen eines genetisch modifizierten Mikroorganismus, der Butanol aus einem Fermentationsmedium produziert, das mindestens eine fermentierbare Kohlenstoffquelle aufweist;
b) Vermehren des Mikroorganismus in einem Fermentationsmedium unter aeroben Bedingungen während einer Zeit, die ausreicht, um einen vorgewählten Vermehrungsgrad zu erreichen;
c) Wechsel zu mikroaeroben oder anaeroben Bedingungen, um die Butanolproduktion in dem Fermentationsmedium anzuregen und ein butanolhaltiges Fermentationsmedium zu bilden;
d) Durchleiten eines Gases durch das Fermentationsmedium, um eine butanolhaltige Gasphase zu bilden;
e) Inkontaktbringen des butanolhaltigen Fermentationsmediums mit i) mindestens einem ersten nicht mit Wasser mischbaren organischen Extraktionsmittel, das aus der Gruppe ausgewählt ist, die aus C₁₂-C₂₂-Fettalkoholen, C₁₂-C₂₂-Fettsäuren, Estern von C₁₂-C₂₂-Fettsäuren, C₁₂-C₂₂-Fettaldehyden und deren Gemischen besteht, und wahlweise ii) einem zweiten nicht mit Wasser mischbaren organischen Extraktionsmittel, das aus der Gruppe ausgewählt ist, die aus C₁₂-C₂₂-Fettalkoholen, C₁₂-C₂₂-Fettsäuren, Estern von C₁₂-C₂₂-Fettsäuren, C₁₂-C₂₂-Fettaldehyden und deren Gemischen besteht, um ein Zweiphasengemisch zu bilden, das eine wässrige Phase und eine butanolhaltige organische Phase aufweist;
f) Trennen der butanolhaltigen organischen Phase von der wässrigen Phase; und
g) Rückgewinnen des Butanols aus der butanolhaltigen organischen Phase und der butanolhaltigen Gasphase.

5. Verfahren zur Herstellung von Butanol, das die folgenden Schritte aufweist:
a) Bereitstellen eines Fermentationsmediums, das Butanol, Wasser und einen genetisch modifizierten Mikroorganismus aufweist, der Butanol aus einem Fermentationsmedium produziert, das mindestens eine fermentierbare Kohlenstoffquelle aufweist;
b) Durchleiten eines Gases durch das Fermentationsmedium, um eine butanolhaltige Gasphase zu bilden;
c) Inkontaktbringen des Fermentationsmediums über einen in gleicher Richtung oder in Gegenrichtung fließenden Extraktionsmittelstrom mit i) mindestens einem ersten nicht mit Wasser mischbaren organischen Extraktionsmittel, das aus der Gruppe ausgewählt ist, die aus C₁₂-C₂₂-Fettalkoholen, C₁₂-C₂₂-Fettsäuren, Estern von C₁₂-C₂₂-Fettsäuren, C₁₂-C₂₂-Fettaldehyden und deren Gemischen besteht, und wahlweise ii) einem zweiten nicht mit Wasser mischbaren organischen Extraktionsmittel, das aus der Gruppe ausgewählt ist, die aus C₁₂-C₂₂-Fettalkoholen, C₁₂-C₂₂-Fettsäuren, Estern von C₁₂-C₂₂-Fettsäuren, C₁₂-C₂₂-Fettaldehyden und deren Gemischen besteht, um ein Zweiphasengemisch zu bilden, das eine wässrige Phase und eine butanolhaltige organische Phase aufweist;
d) Trennen der butanolhaltigen organischen Phase von der wässrigen Phase; und
e) Rückgewinnen des Butanols aus der butanolhaltigen organischen Phase und der butanolhaltigen Gasphase, um zurückgewonnenes Butanol zu produzieren.

6. Verfahren nach einem der Ansprüche 1-5, wobei das organische Extraktionsmittel aus der Gruppe ausgewählt ist, die aus Oleylalkohol, Behenylalkohol, Cetylalkohol, Laurylalkohol, Myristylalkohol, Stearylalkohol, 1-Undecanol, Oleinsäure, Laurinsäure, Myristinsäure, Stearinsäure, Methylmyristat, Methyloleat, Undecanal, Laurinaldehyd, 2-Methylundecanal und deren Gemischen besteht.

7. Verfahren nach einem der Ansprüche 1-6, wobei das organische Extraktionsmittel Oleylalkohol aufweist.

8. Verfahren nach einem der Ansprüche 1-7, wobei das zurückgewonnene Butanol einen effektiven Titer von mindestens etwa 37 g pro Liter der wässrigen Phase aufweist.

9. Verfahren nach einem der Ansprüche 1-8, wobei das Fermentationsmedium ferner Ethanol aufweist und die butanolhaltige organische Phase Ethanol enthält.

10. Verfahren nach einem der Ansprüche 1-9, wobei das Butanol Isobutanol ist.

11. Verfahren nach einem der Ansprüche 1-10, wobei der genetisch modifizierte Mikroorganismus aus der Gruppe ausgewählt ist, die aus Bakterien, Cyanobakterien, Fadenpilzen und Hefen besteht.

12. Verfahren nach Anspruch 11, wobei die Bakterien aus der Gruppe ausgewählt sind, die aus Zymomonas, Escherichia, Salmonella, Rhodococcus, Pseudomonas, Bacillus, Lactobacillus, Enterococcus, Pediococcus, Alcaligenes, Klebsiella, Paenibacillus, Arthrobacter, Corynebacterium und Brevibacterium besteht.

13. Verfahren nach Anspruch 11, wobei die Hefe aus der Gruppe ausgewählt ist, die aus Pichia, Candida, Hansenula und Saccharomyces besteht.

## Revendications

1. Procédé de récupération de butanol à partir d'un milieu de fermentation, le procédé comprenant les étapes consistant à:
a) se procurer un milieu de fermentation comprenant du butanol, de l'eau, et un micro-organisme génétiquement modifié qui produit du butanol à partir d'un milieu de fermentation comprenant au moins une source de carbone fermentescible;
b) faire passer un gaz à travers le milieu de fermentation pour former une phase gazeuse contenant du butanol;
c) mettre en contact le milieu de fermentation avec i) au moins un premier agent d'extraction organique non miscible à l'eau choisi dans le groupe constitué par les alcools gras en C₁₂ à C₂₂, les acides gras en C₁₂ à C₂₂, les esters d'acides gras en C₁₂ à C₂₂, les aldéhydes gras en C₁₂ à C₂₂ , et leurs mélanges, et optionnellement (ii) un second agent d'extraction organique non miscible à l'eau choisi dans le groupe constitué par les alcools gras en C₁₂ à C₂₂, les acides gras en C₁₂ à C₂₂, les esters d'acides gras en C₁₂ à C₂₂, les aldéhydes gras en C₁₂ à C₂₂ , et leurs mélanges, pour former un mélange à deux phases comprenant une phase aqueuse et une phase organique contenant du butanol;
d) séparer la phase organique contenant du butanol de la phase aqueuse; et
e) récupérer le butanol à partir de la phase organique contenant du butanol et de la phase gazeuse contenant du butanol pour produire du butanol récupéré.

2. Procédé de production de butanol comprenant les étapes consistant à:
a) se procurer un micro-organisme génétiquement modifié qui produit du butanol à partir d'un milieu de fermentation comprenant au moins une source de carbone fermentescible;
b) faire passer un gaz à travers le milieu de fermentation pour former une phase gazeuse contenant du butanol;
c) cultiver le micro-organisme dans un milieu de fermentation à deux phases comprenant une phase aqueuse et un agent d'extraction organique non miscible à l'eau choisi dans le groupe constitué par les alcools gras en C₁₂ à C₂₂, les acides gras en C₁₂ à C₂₂, les esters d'acides gras en C₁₂ à C₂₂, les aldéhydes gras en C₁₂ à C₂₂ , et leurs mélanges, où ledit milieu de fermentation à deux phases comprend environ 3% à environ 60% en volume dudit agent d'extraction organique non miscible à l'eau, pendant une durée suffisante pour permettre l'extraction du butanol dans l'agent d'extraction organique pour former une phase organique contenant du butanol;
d) séparer la phase organique contenant du butanol de la phase aqueuse; et
e) récupérer le butanol à partir de la phase organique contenant du butanol et de la phase gazeuse contenant du butanol pour produire du butanol récupéré.

3. Procédé de production de butanol comprenant les étapes consistant à:
a) se procurer un micro-organisme génétiquement modifié qui produit du butanol à partir d'un milieu de fermentation comprenant au moins une source de carbone fermentescible;
b) cultiver le micro-organisme dans un milieu de fermentation où le micro-organisme produit ledit butanol dans le milieu de fermentation pour produire un milieu de fermentation contenant du butanol;
c) faire passer un gaz à travers le milieu de fermentation pour former une phase gazeuse contenant du butanol;
d) mettre en contact le milieu de fermentation contenant du butanol avec i) au moins un premier agent d'extraction organique non miscible à l'eau choisi dans le groupe constitué par les alcools gras en C₁₂ à C₂₂, les acides gras en C₁₂ à C₂₂, les esters d'acides gras en C₁₂ à C₂₂, les aldéhydes gras en C₁₂ à C₂₂ , et leurs mélanges, et optionnellement (ii) un second agent d'extraction organique non miscible à l'eau choisi dans le groupe constitué par les alcools gras en C₁₂ à C₂₂, les acides gras en C₁₂ à C₂₂, les esters d'acides gras en C₁₂ à C₂₂, les aldéhydes gras en C₁₂ à C₂₂ , et leurs mélanges, pour former un mélange à deux phases comprenant une phase aqueuse et une phase organique contenant du butanol;
e) séparer la phase organique contenant du butanol de la phase aqueuse; et
f) récupérer le butanol à partir de la phase organique contenant du butanol et de la phase gazeuse contenant du butanol.

4. Procédé de production de butanol comprenant les étapes consistant à:
a) se procurer un micro-organisme génétiquement modifié qui produit du butanol à partir d'un milieu de fermentation comprenant au moins une source de carbone fermentescible;
b) cultiver le micro-organisme dans un milieu de fermentation dans des conditions aérobies pendant une durée suffisante pour parvenir à un niveau de croissance prédéfini;
c) basculer en conditions microaérobies ou anaérobies pour stimuler la production de butanol dans le milieu de fermentation pour former un milieu de fermentation contenant du butanol;
d) faire passer un gaz à travers le milieu de fermentation pour former une phase gazeuse contenant du butanol;
e) mettre en contact le milieu de fermentation contenant du butanol avec i) au moins un premier agent d'extraction organique non miscible à l'eau choisi dans le groupe constitué par les alcools gras en C₁₂ à C₂₂, les acides gras en C₁₂ à C₂₂, les esters d'acides gras en C₁₂ à C₂₂, les aldéhydes gras en C₁₂ à C₂₂, et leurs mélanges, et optionnellement (ii) un second agent d'extraction organique non miscible à l'eau choisi dans le groupe constitué par les alcools gras en C₁₂ à C₂₂, les acides gras en C₁₂ à C₂₂, les esters d'acides gras en C₁₂ à C₂₂, les aldéhydes gras en C₁₂ à C₂₂ , et leurs mélanges, pour former un mélange à deux phases comprenant une phase aqueuse et une phase organique contenant du butanol;
f) séparer la phase organique contenant du butanol de la phase aqueuse; et
g) récupérer le butanol à partir de la phase organique contenant du butanol et de la phase gazeuse contenant du butanol.

5. Procédé de production de butanol comprenant les étapes consistant à:
a) se procurer un milieu de fermentation comprenant du butanol, de l'eau, et un micro-organisme génétiquement modifié qui produit du butanol à partir d'un milieu de fermentation comprenant au moins une source de carbone fermentescible;
b) faire passer un gaz à travers le milieu de fermentation pour former une phase gazeuse contenant du butanol;
c) mettre en contact le milieu de fermentation via un écoulement d'agent d'extraction cocourant ou à contre-courant avec i) au moins un premier agent d'extraction organique non miscible à l'eau choisi dans le groupe constitué par les alcools gras en C₁₂ à C₂₂, les acides gras en C₁₂ à C₂₂, les esters d'acides gras en C₁₂ à C₂₂, les aldéhydes gras en C₁₂ à C₂₂ , et leurs mélanges, et optionnellement (ii) un second agent d'extraction organique non miscible à l'eau choisi dans le groupe constitué par les alcools gras en C₁₂ à C₂₂, les acides gras en C₁₂ à C₂₂, les esters d'acides gras en C₁₂ à C₂₂, les aldéhydes gras en C₁₂ à C₂₂ , et leurs mélanges, pour former un mélange à deux phases comprenant une phase aqueuse et une phase organique contenant du butanol;
d) séparer la phase organique contenant du butanol de la phase aqueuse; et
e) récupérer le butanol à partir de la phase organique contenant du butanol et de la phase gazeuse contenant du butanol pour produire du butanol récupéré.

6. Procédé selon l'une quelconque des revendications 1 à 5 dans lequel l'agent d'extraction organique est choisi dans le groupe constitué par l'alcool oléylique, l'alcool béhénylique, l'alcool cétylique, l'alcool laurylique, l'alcool myristylique, l'alcool stéarylique, le 1-undécanol, l'acide oléique, l'acide laurique, l'acide myristique, l'acide stéarique, le myristate de méthyle, l'oléate de méthyle, l'undécanal, l'aldéhyde laurique, le 2-méthylundécanal, et leurs mélanges.

7. Procédé selon l'une quelconque des revendications 1 à 6 dans lequel l'agent d'extraction organique comprend de l'alcool oléylique.

8. Procédé selon l'une quelconque des revendications 1 à 7 dans lequel le butanol récupéré a un titre effectif d'au moins environ 37 g par litre de la phase aqueuse.

9. Procédé selon l'une quelconque des revendications 1 à 8 dans lequel le milieu de fermentation comprend en outre de l'éthanol, et la phase organique contenant du butanol contient de l'éthanol.

10. Procédé selon l'une quelconque des revendications 1 à 9 dans lequel le butanol est de l'isobutanol.

11. Procédé selon l'une quelconque des revendications 1 à 10 dans lequel le micro-organisme génétiquement modifié est choisi dans le groupe constitué par les bactéries, les cyanobactéries, les champignons filamenteux et les levures.

12. Procédé selon la revendication 11 dans lequel les bactéries sont choisies dans le groupe constitué par Zymomonas, Escherichia, Salmonella, Rhodococcus, Pseudomonas, Bacillus, Lactobacillus, Enterococcus, Pediococcus, Alcaligenes, Klebsiella, Paenibacillus, Arthrobacter, Corynebacterium, et Brevibacterium.

13. Procédé selon la revendication 11 dans lequel la levure est choisie dans le groupe constitué par Pichia, Candida, Hansenula et Saccharomyces.
